# EUROPEAN PATENT SPECIFICATION

(11) **EP 2 585 477 B1**
(45) Date of publication and mention of the grant of the patent: **30.05.2018**
(21) Application number: 11737889.3
(22) Date of filing: 24.06.2011
(51) Int. Cl.: C07K 14/005, C12Q 1/70

(54) **REARRANGED TT VIRUS MOLECULES FOR USE IN DIAGNOSIS, PREVENTION AND TREATMENT OF CANCER AND AUTOIMMUNITY**
NEUE ANGEORDNETE TT-VIRUS-MOLEKÜLE ZUR VERWENDUNG BEI DER DIAGNOSE, VORBEUGUNG UND BEHANDLUNG VON KREBS UND AUTOIMMUNITÄT
MOLÉCULES VIRALES TT RÉARRANGÉES POUVANT ÊTRE UTILISÉES POUR DIAGNOSTIQUER, PRÉVENIR ET TRAITER LE CANCER ET L'AUTO-IMMUNITÉ

(30) Priority: 23.11.2010 US 952300; 23.06.2010 US 821634; 23.11.2010 EP 10014907; 23.06.2010 EP 10006541
(43) Date of publication of application: 01.05.2013
(73) Proprietor: Deutsches Krebsforschungszentrum, 69120 Heidelberg (DE)
(72) Inventor: DE VILLIERS, Ethel-Michele, 69483 Waldmichelbach (DE); ZUR HAUSEN, Harald, 69483 Waldmichelbach (DE)
(74) Representative: Schüssler, Andrea
(86) International application number: PCT/EP2011/003119
(87) International publication number: WO 2011/160848

(56) References cited:
- WO-A2-00/28039
- WO-A2-00/46407
- WO-A2-01/42299
- WO-A2-03/023027
- WO-A2-2007/130519
- WO-A2-2008/138619
- JELCIC I ET AL: "Isolation of multiple TT virus genotypes from spleen biopsy tissue from a Hodgkin's disease patient: Genome reorganization and diversity in the hypervariable region", JOURNAL OF VIROLOGY, THE AMERICAN SOCIETY FOR MICROBIOLOGY, US, vol. 78, no. 14, 1 January 2004 (2004-01-01), pages 7498-7507, XP002463335, ISSN: 0022-538X, DOI: DOI:10.1128/JVI.78.14.7498-7507.2004 -& DATABASE EMBL [Online] 3 February 2009 (2009-02-03), "Torque teno virus, isolate tth25, complete genome", XP002610817, retrieved from EBI accession no. AJ620222 Database accession no. AJ620222
- MIREIA SOSPEDRA ET AL: "Recognition of Conserved Amino Acid Motifs of Common Viruses and Its Role in Autoimmunity", PLOS PATHOGENS, vol. 1, no. 4, 1 January 2005 (2005-01-01) , page E41, XP55011250, ISSN: 1553-7366, DOI: 10.1371/journal.ppat.0010041 cited in the application
- LAURA KAKKOLA ET AL: "Construction and biological activity of a full-length molecular clone of human Torque teno virus (TTV) genotype 6", FEBS JOURNAL, vol. 274, no. 18, 1 September 2007 (2007-09-01), pages 4719-4730, XP55011217, ISSN: 1742-464X, DOI: 10.1111/j.1742-4658.2007.06020.x -& DATABASE EMBL [Online] 27 August 2004 (2004-08-27), "Torque teno virus 3 strain HEL32, complete genome.", XP002662777, retrieved from EBI accession no. EMBL:AY666122 Database accession no. AY666122
- PENG Y H ET AL: "Analysis of the entire genomes of thirteen TT virus variants classifiable into the fourth and fifth genetic groups, isolated from viremic infants", ARCHIVES OF VIROLOGY, vol. 147, no. 1, January 2002 (2002-01), pages 21-41, XP002610938, ISSN: 0304-8608
- DE VILLIERS E M ET AL: "Intragenomic rearrangement in TT viruses: a possible role in the pathogenesis of disease.", CURRENT TOPICS IN MICROBIOLOGY AND IMMUNOLOGY 2009 LNKD- PUBMED:19230559, vol. 331, 2009, pages 91-107, XP009153717, ISSN: 0070-217X

## Description

The present invention relates to rearranged molecules of (a) a specific TT virus sequence and (b) a nucleotide sequence encoding a polypeptide showing homology to mammalian proteins associated with cancer or an autoimmune disease that are capable of replicating autonomously.

The family *Anelloviridae* includes Torque teno viruses (TTV), TT-midiviruses (TTMDV) and TT-miniviruses (TTMV), the majority originating from samples of human origin (Nishizawa et al., 1997; Takahashi et al., 2000; Ninomiya et al., 2007; Okamoto, 2009; Biagini and de Micco, 2010). The plurality of this family of ssDNA viruses is reflected not only in DNA sequence, but also in genome size and organization.

Multiple attempts have been made to find a suitable in vitro system for the replication and propagation of TT viruses. Replicative forms of its DNA have been demonstrated in bone marrow cells and in the liver (Kanda et al., 1999; Okamoto et al., 2000a, c, d). Peripheral blood acts as reservoir for TT viruses (Okamoto et al., 2000b) and replication in vivo seems to occur preferably in activated mononuclear cells (Maggi et al., 2001b; Mariscal et al., 2002; Maggi et al., 2010). Although in vitro transcription has been investigated in a variety of cell lines (Kamahora et al., 2000; Kamada et al., 2004; Kakkola et al., 2007; 2009; Qiu et al., 2005; Müller et al., 2008), long term replication leading to virus production has been difficult to achieve (Leppik et al., 2007).

The presence of a variety of intragenomic rearranged TT subviral molecules in sera samples and the in vitro transcription of a subviral molecule constituting only 10% of the complete genome, initiated the discussion whether TT viruses may share similarities to the plantvirus family *Geminiviridae* (Leppik et al., 2007; de Villiers et al., 2009).

Both mono- and bipartite Geminiviruses associate with single-stranded DNA satellites to form disease-inducing complexes (Saunders et al., 2000; Stanley, 2004; Nawaz-ul-Rehman and Fauquet, 2009; Jeske 2009; Paprotka et al., 2010; Patil et al., 2010).

Infections occur within the first days of life with close to 100% of infants being infected at one year of age. The primary route of infection however still remains unclear (Kazi et al., 2000; Peng et al., 2002; Ninomiya et al., 2008). The ubiquitous nature of TTV infections has hampered efforts to associate it with the pathogenesis of disease (Jelcic et al., 2004; Leppik et al., 2007; de Villiers et al., 2009; Okamoto, 2009). A possible etiological association with diseases of the liver (reviewed in Okamoto, 2009), respiratory tract (Biagini et al., 2003; Maggi et al., 2003a,b; Pifferi et al., 2005), hematopoietic malignancies (Jelcic et al., 2004; Leppik et al., 2007; de Villiers et al., 2002; 2009; Shiramizu et al., 2002; Garbuglia et al., 2003; zur Hausen and de Villiers, 2005) and auto-immune diseases (Sospedra et al., 2005; Maggi et al., 2001a; 2007; de Villiers et al., 2009) have been reported. During the past years, additional data has been compiled indicative of an association of TT virus infection with human malignant tumors. A high rate of TT virus load has been noted in a spleen biopsy of a patient with Hodgkin's lymphoma (24 individual TTV genotypes). Similarly, other reports describe a higher rate of TTV prevalence in colorectal and esophageal cancer and in hematopoietic malignancies in comparison to non-tumorous tissue from the same or other patients. Yet, the ubiquity of these infections rendered an interpretation of these results rather difficult and did not permit a linkage of these observations with tumor development.

WO 2008/138619 discloses rearranged and autonomously replicating, subviral TT virus molecules which are associated with cancer.
Jelcic et al. (2004) discloses a rearranged TT virus polynucleic acid comprising the sequence of tth25.

Sospedra (2005) discloses a complete genome of a rearranged TT virus polynucleic acid and suggests that complete genome of TT virus is associated with autoimmune disease.
WO 00/46407 discloses a TTV based vector, wherein a gene of interest is inserted within the complete genome of a TT virus.

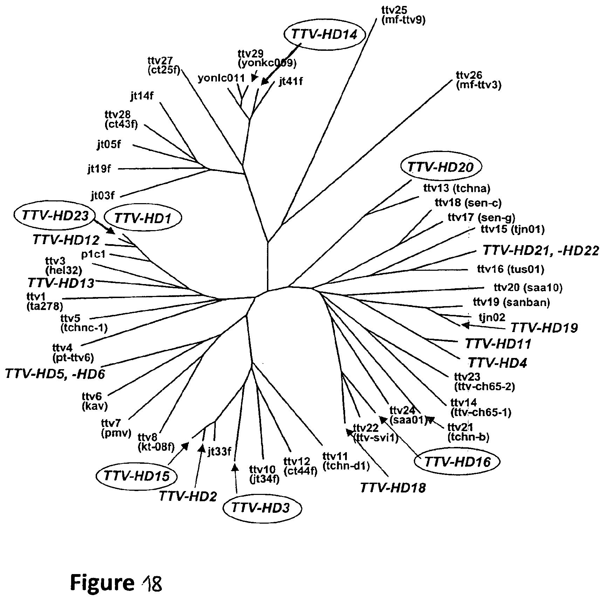

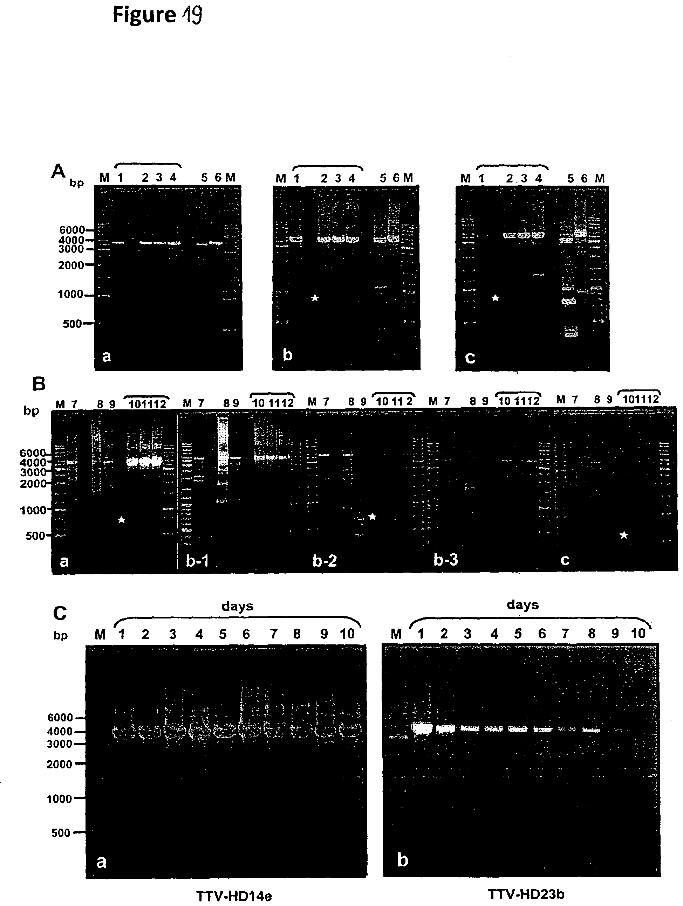

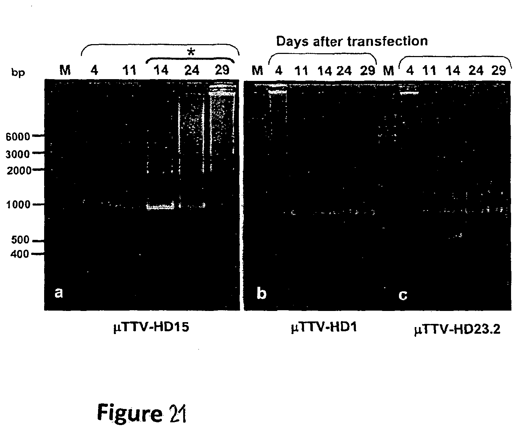

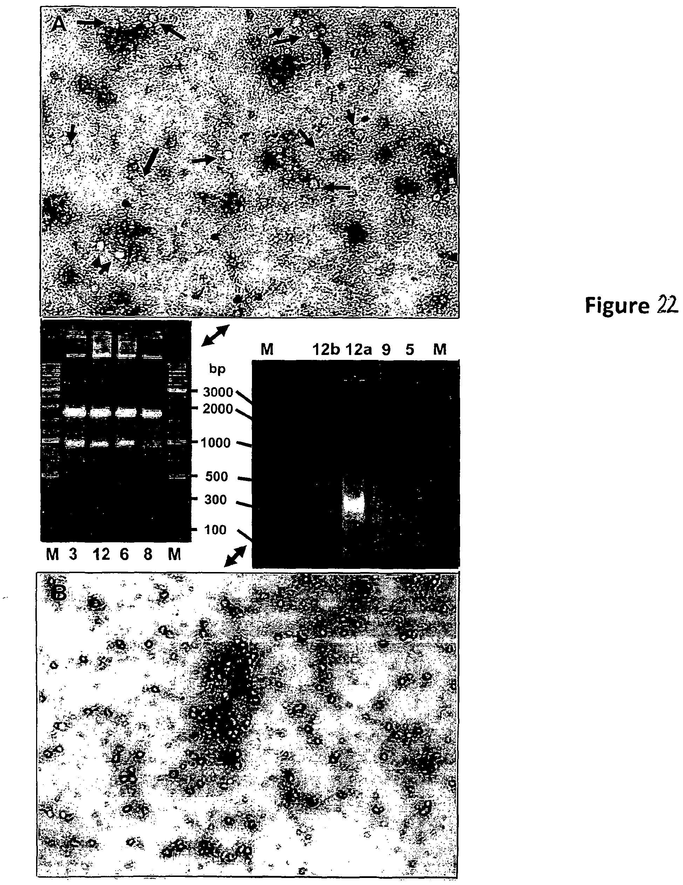

During the experiments resulting in the present invention more than 200 genomes of TT virus have been isolated. The isolates grouping in the genus Alphatorquevirus (ca 3,8kb in size) share very low DNA sequence homology and differ in their genome organization. A short stretch (71 bp) of the intergenic region is highly conserved among all human TTV isolates (Peng et al., 2002) and is widely used to demonstrate TT virus infection. Samples from a broad spectrum of diseases were analysed for the presence of torque teno virus DNA by applying PCR-amplification of this conserved region (Jelcic et al., 2004; Leppik et al., 2007; de Villiers et al., 2009; Sospedra et al., 2005; de Villiers and Gunst, unpublished results). Identification of individual TT virus types however requires the amplification of full-length genomes. Thus far 93 full-length genomes of TTVs (ca 3,8kb) were isolated from human samples (Jelcic et al., 2004; Leppik et al., 2007; de Villiers et al., 2009; present experiments). These included samples obtained from healthy individuals, patients with leukaemia and lymphoma, rheumatoid arthritis, multiple sclerosis and kidney disease. The present disclosure describes the in vitro replication and transcription of 12 isolates after initial transfection of the genomic DNA and followed by virus propagation using frozen infected cells or purified particles. Intragenomic rearranged subviral molecules µTTY (microTTV) appearing in early passages were cloned and characterized. These also propagated independently in cell culture resulting in novel particle-like structures which are able to infect virus-free 293TT cells.
The ubiquity of torque teno viruses, together with the absence of suitable in vitro culture systems, has hampered progress in investigating this group of viruses. The multitude and heterogeneity of types (Biagini and de Micco, 2010; Okamoto, 2009), as well as their ubiquitous presence in hematopoietic cells (Takahashi et al., 2002; Kanda et al., 1999; Zhong et al., 2002), have added to the delay in gaining information on whether these viruses are involved in the pathogenesis of any disease. A spectrum of TTV types was isolated (Jelcic et al., 2004; Leppik et al., 2007; de Villiers et al., 2009; present disclosure). Full-length genomes of a number of TTV types were often isolated from an individual sample depending on the composition of primers used for long-distance PCR amplification. The scattered distribution of the new isolates of the present disclosure on a phylogenetic tree of genus Alphatorquevirus (Figure 18) indicates their heterogeneity, irrespective of origin. The variation in genome organization resulting from minor differences in sequence identity across the genome was often observed between isolates of the same type and has prompted questions as to the functionality of these modified genes.
In the past attempts were made to propagate TTV genomes in a number of cell lines and in peripheral blood monocytes under varying in vitro culturing conditions. Moderate success with single isolates was achieved in Hodgkin's lymphoma cell lines and in 293T cells. Replication was however slow and occurred at low levels (Leppik et al., 2007; Leppik and de Villiers, unpublished data). For the studies of the present disclosure the human embryonic kidney cell line 293TT was engineered to express high-levels of SV-40 large-T antigen (Buck et al., 2005). Transfecting TTV genomes into these cells resulted in virus DNA replication and production of virus-like particles of ca. 30 nm in size (Figure 22). The structures of these virus-like particles differ from those previously published as TTV particles (Itoh et al., 2000). This is possibly a consequence of the isolation of the latter from faeces.
The differences in the level of DNA replication observed between TTV-isolates cannot presently be explained. Phylogenetic information does not provide an answer. Noticeable is that 6 isolates (TTV-HD14, TTV-HD15 and TTV-HD16) which originated from brain biopsies of patients with multiple sclerosis all replicated much less in the system of the present disclosure Virus production (Figure 22) or virus propagation (Figures 19 and 21) did not seem to be influenced despite the varying levels of DNA replication or modifications in the genome organization which included modified ORF1s. Transcription levels however, seemed to be influenced and fewer of the common transcripts described for other TTV-types were detected in the four TTV-14 isolates than in TTV-HD15a and TTV-HD16a cultures. Previously reported transcripts (Leppik et al., 2007; Kakkola et al., 2009) were isolated from all infected cultures. Interestingly, no transcript was identified which would code for full-length ORF1 protein (suspected to play a major role in coding for the viral capsid, but not yet proven) of any of the TTV-HD types studied, despite the isolation of full-length genome-carrying virus-like particles from all infected cultures. A number of putative protein sequences were identified which may have resulted from fusion products of any two or three genes. Translation strategies known to be used by viruses, such as leaky scanning, re-initiation and ribosomal shunting (Ryabova et al., 2006) might be involved here. Dual coding in alternative reading frames is an additional mechanism which may be involved (Kovacs et al., 2010). Interestingly, transcripts of the control region were also isolated. Here two groups of transcripts were identified. One group involved transcripts spanning at least part of the intergenic region and extending into the rest of the genome covering the known genes. The second group consisted of transcripts varying in length and without recognizable coding capacity. It has been proposed that the nature of the TTV intergenic region with its high GC content may play a role in transcription-dependent replication blockage (Belotserkovskii et al., 2010).

A very prominent observation in the present study is the formation of subviral molecules already early during the replication cycle of the majority of the isolates obtained. Two groups of subviral molecules were distinguished. The formation of multiple subviral DNA molecules ranging in size occurred frequently and extensively in TTV-HD20a-, TTV-HD3a- and TTV-HD1a-infected cultures. Previously similar rearranged subviral molecules were demonstrated in serum samples (Leppik et al., 2007). Transfection into L428 cells (Hodgkin's lymphoma cell line) of a small number of the subviral genomes originating from sera resulted in limited replication and transcription for a few days (de Villiers et al., 2009). Data shown in the present disclosure indicate a role as defective interfering particles during in vitro replication of the full-length genome. Replication of the full-length genome is reduced during simultaneously increasing levels of subviral molecules (Figure 19b). Similar subviral molecules were occasionally and inconsistently demonstrated in cultures of the other 9 isolates, but did not influence the replication of the full-length genome. This difference also underlines not only the diversity between TTV types, but also that this phenomenon does not result from PCR artifacts. Similar defective interfering molecules have also been reported in Geminiviruses where they accumulate during improper replication (Jeske, 2009).
The second group of subviral molecules µTTV evolved during replication of TTV isolates TTV-HD14b, TTV-HD14c, TTV-HD14a and TTV-HD14e, TTV-HD15a, TTV-HD16a, TTV-HD1a, TTV-HD23b, TTV-HD23d and TTV-HD23a and remained constant in size and composition during propagation, as evidenced after cloning and sequencing. Their production in the case of the latter 4 isolates seemed to be influenced by culturing conditions. Interestingly, the subviral molecule µTTV-HD1 in the TTV-HD1a infected culture was detectable in the cell culture even after loss of detectable parental full-length genome (Figure 19c). Two molecules µTTV-HD23.1 (409 bases) and µTTV-HD23.2 (642 bases) were isolated from all 3 TTV-HD23 infected cultures. µTTV-HD23.2 is composed of the µTTV-HD23.1 molecule plus a duplication of 306 nt of the smaller molecule. Subviral molecules (µTTV-HD14) which were isolated from the 4 TTV-HD14 cultures were all identical in sequence and appeared very early after the initial transfection of the parental genome. The production of these smaller molecules did not seem to be influenced by the variation in genome structure between isolates of the same TTV type. All subviral molecules were composed of parts of the parental TTV type, although the genome regions involved, differed. They were all amplified by long-distance PCR using the same back-to-back primers as for amplification of the parental genome. The episomal replication of a TTV subviral molecule isolated from a serum sample over a period of 23 days had previously been observed (de Villiers et al., 2009). Multimeric subviral RNA was demonstrated during this process. The subviral molecules reported in the present disclosure are able to replicate autonomously, can be propagated in vitro (Figure 21) and appear to be related to small protein structures observed in these cultures by electronmicroscope (Figure 22). It is not known whether they are transmitted as part of an infectious TT virus or whether they are induced only after infection by the parent virus and then transmitted by autonomously infecting other cells. Similar subviral DNAs have been associated with the geminivirus disease complex (Stanley, 2004). β-satellites enhance symptom phenotypes in plants. They share a network of protein interactions with geminiviruses and are dependent on them for trans-replication, encapsidation and vector transmission. The only sequence shared between β-satellites and geminiviruses lies in the short origin of replication (Nawaz-ul-Rehman and Fauquet, 2009; Patil and Fauquet, 2010; Paprotka et al., 2010). This is in contrast to the TTV subviral molecules (µTTV) which share almost identical sequences with the parental genome. The cytopathic effect observed during in vitro propagation of the TTV subviral molecules of the present invention points to their possible role as the disease-inducing component of some torque teno viruses. Signature motifs of proteins involved in autoimmune disease have been identified by in silico analyses of putative proteins expressed by these subviral molecules, as well as from virus transcripts isolated from the TTV-infected cultures. The observation of a DNA encoding a protein containing a signature motif of a mammalian protein associated with cancer or an autoimmune disease linked to the 71 bp highly conserved TT virus region (HCR) is the basis for the following conclusion: The rearranged open reading frames of TTV and µTTV code for antigenic epitopes which mimic cellular protein sequences which are attacked in cancer or autoimmune diseases. Their shared, but not identical sequence should provoke an immune response against these epitopes present also in normal tissue.

### A novel role for TT viruses in human cancer and autoimmunity

The surprising observation of host cell DNA linked to an apparently single-stranded form to TT virus HCR is the basis for the following conclusion: TT viral sequences have not yet been demonstrated as integrated into double-stranded cellular DNA, persisting within host cell chromosomes. Thus, the opposite finding of host cell DNA, linked in a single-stranded state to the TTV HCR should have biological significance. The present data indicate their long-time persistence as episomes in human cancer cell lines, pointing to a role of this persistence in cell proliferation. Two aspects seem to require specific consideration: a possible role of those recombinants in cancer and in autoimmunity.

One possibility is the random integration of host cell sequences into TTV episomes. This may happen after strand displacement in the course of aberrant DNA replication or after reverse transcription of cellular RNA. In case of random integration a larger number of recombinants should be innocuous and harmless for cells carrying these recombinants. A growth-promoting property of transcripts of the TTV HCR, as well as integration and transcription of growth-stimulating host cell genes, their modification in the process of integration or their dysregulation by the TTV HCR however, will result in proliferative consequences. These episomes should acquire immortalizing and under certain conditions transforming properties. In combination with additional modifications of the host cell genome they may direct malignant growth. This mode of action reveals a distant resemblance to the insertion of cellular oncogenes into retroviral genomes.

### The TTV-Oncogene concept

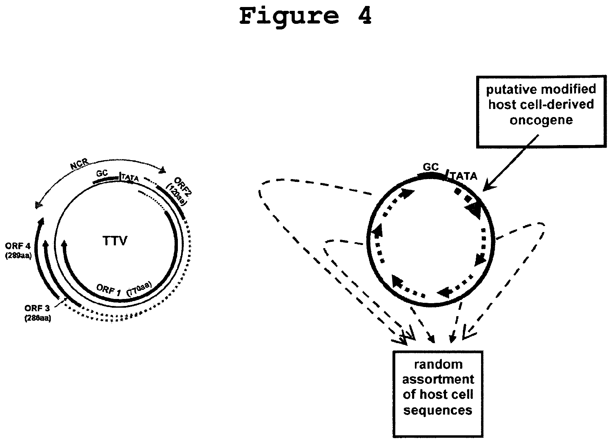

The previous considerations are summarized in Fig 4. Obviously, the recombination between the TTV regulatory region and cellular nucleic acids must be a relatively frequent process, since such recombinants are found in the majority of cell lines thus far analyzed. It also should contribute to cell proliferation, otherwise the regular persistence of such molecules, in part over decades of continuous proliferation, would be difficult to explain. It is assumed that this type of recombination is a random process, involving different types of cellular genes. The coding function of the TTV HCR and/or the uptake of genes steering cell proliferation, or blocking the function of proliferation antagonists, or inhibiting cell differentiation should lead to an accumulation of cells containing these types of recombinants. It is envisaged that this, in combination with additional mutational or recombinational events of the cells harbouring such TTV-host cell nucleic acid recombinants, provides a selective advantage for cells carrying such episomes. The presence of the latter would represent a prime risk factor for malignant conversion. In this sense those recombinations should be of general importance for different types of human cancers, although a certain degree of specificity for a limited set of genes would be expected for individual cancer types.

The implications of this model are profound. They reach from cancer prevention, early detection into cancer therapy. The important role of TTV infections and of the persistence of TTV HCR is stressed by the available information. Prevention of these infections should reduce the risk for the development of the described recombinants. The diagnosis of specific recombinants would probably contribute to cancer risk assessment. Profound implications would be expected for cancer therapy: the TTV HCR emerges as the prime determinant for the persistence and maintenance of the single-stranded episomes. Since this region appears to be part of an open reading frame, it should be vulnerable to small interfering RNAs or DNAs. Thus, it offers a suitable target for future therapeutic deliberations.

Two other aspects deserve discussion: certain parallels which seem to exist to retroviral carcinogenesis in rodents and chicken and the use of autonomously replicating TTV-based vector systems for gene therapy. Insertional mutagenesis, the uptake and modification of cellular growth-stimulating genes, rendering them into oncogenes has frequently been analyzed in animal systems. This has thus far not been reported for human cancers. Do TT viruses replace this niche in human and other primate cells? Do TTV compete successfully with retrovirus infections in taking over their role in specific species? The episomal persistence of single-stranded DNA, however, emerges as a remarkable difference to retrovirus-induced carcinogenesis.

Autonomously replicating subviral DNA molecules of approximately 400 bases of TTV origin have been described before. It is tempting to speculate that they or specific TTV-host cell recombinants may represent optimal vector systems for future approaches in gene therapy and for the construction of artificial chromosomes.

### The recombinant TTV-host cell DNA autoimmunity concept

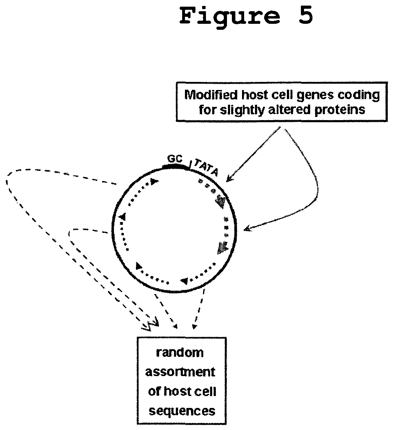

The existence of TTV host cell nucleic acid recombinants also permits a novel view on aspects of autoimmune diseases and other chronic diseases (potentially even conditions like arteriosclerosis and Alzheimer's disease). Modification or dys-regulation of cellular proteins may originate from insertional events of cellular genes into single-stranded DNA or to the different HCRs exerted by TTV elements (Fig. 5). They could provide a convenient explanation for autoimmune reactions, even for local ones, like in multiple sclerosis (MS) or Crohn's disease. In the latter two cases in particular, the reactivation of other local infections (potentially herpes-type viruses) would provide a stimulus for the local amplification and gene activity of the respective TTV-host cell nucleic acid recombinants. In MS, this could explain recurrent episodes of disease progression. A model of the autoimmunity concept is depicted in Figure 5.

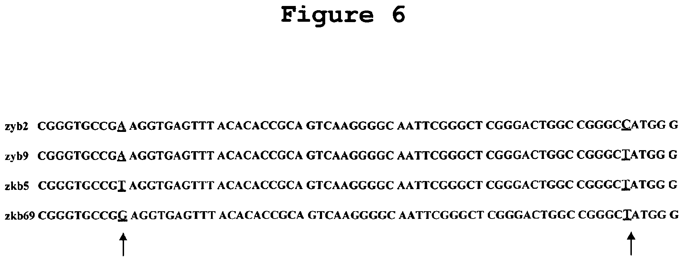

Similarly, rearranged TT virus molecules of 719, 642, and 621 bases have been identified which replicate autonomously upon transfection of specific cell lines. Their DNA composition and derivation from specific complete TTV genotypes is shown in Figure 6. Here the rearrangement results in novel open reading frames in part with epitopes related to those of juvenile diabetes and rheumatoid arthritis.

### Conclusion

The models of the present invention for a role of TTV-host cell nucleic acid recombinants is based on the demonstration of the single-stranded chimeric molecules between the TTV HCR and host cell DNA and rearranged autonomously replicating TTV molecules of substantially reduced molecular weights. Both, the TTV oncogene concept and the TTV autoimmunity concept will clearly provide novel approaches to prevention, diagnosis, and in particular to therapy of these conditions and will improve the prognosis of the respective patients.

### Definitions

Unless defined otherwise, all technical and scientific terms used herein have the same meaning as commonly understood by those of ordinary skill in the art to which the invention belongs. Although any methods and materials similar or equivalent to those described herein can be used in the practice or testing of the present invention, preferred methods and materials are described. For the purposes of the present invention, the following terms are defined below.

By "signature motif of a mammalian protein being associated with an autoimmune disease" is meant an amino acid sequence showing striking identity to a motif that can be found in any of the proteins listed in Table 1. Preferably, the length of the signature motif is at least 5 aa, preferably at least 10 aa, more preferably at least 20 aa, and most preferably at least 30 aa and/or the degree of identity of this signature motif to a corresponding motif in a mammalian protein is at least 50%, 60%, 70%, 80%, 90% or 95%.

By *"antibody"* is meant a protein of the immunoglobulin family that is capable of combining, interacting or otherwise associating with an antigen. The term *"antigen"* is used herein in its broadest sense to refer to a substance that is capable of reacting in and/or inducing an immune response. Typically, but not necessarily, antigens are foreign to the host animal in which they produce immune reactions.

By *"epitope" is* meant that part of an antigenic molecule against which a particular immune response is directed. Typically, in an animal, antigens present several or even many antigenic determinants simultaneously. Thus, the terms "epitope" and "antigenic determinant" mean an amino acid sequence that is immunoreactive. Generally an epitope consists of 4, and more usually 5,6,7,8 or 9 contiguous amino acids. However, it should also be clear that an epitope need not be composed of a contiguous amino acid sequence. The immunoreactive sequence may be separated by a linker, which is not a functional part of the epitope. The linker does not need to be an amino acid sequence, but can be any molecule that allows the formation of the desired epitope.

The term *"biological sample"* as used herein refers to a sample that may be extracted, untreated, treated, diluted or concentrated from an animal. Biological sample refers to any biological sample (tissue or fluid) containing a TTV polynucleic acid of the invention and refers more particularly to blood serum samples, plasma samples, biopsy samples, cerebrospinal fluid samples etc..

By *"carrier" is* meant any substance of typically high molecular weight to which a non- or poorly immunogenic substance (e.g., a hapten) is naturally or artificially linked to enhance its immunogenicity.

The term *"diagnosis"* is used herein in its broadest sense to include detection of an antigen reactive to a sub-immunoglobulin antigen-binding molecule. Also included within its scope, is the analysis of disorder mechanisms. Accordingly, the term "diagnosis" includes the use of monoclonal antibodies for research purposes as tools to detect and understand mechanisms associated with a disease or condition of interest. It also includes the diagnostic use of TTV polynucleic acid of the invention for the detection of homologous or complementary RNA transcribed from such molecules.
The term *"immunogenicity"* is used herein in its broadest sense to include the property of evoking an immune response within an organism. Inmunogenicity typically depends partly upon the size of the substance in question, and partly upon how unlike host molecules it is. It is generally considered that highly conserved proteins tend to have rather low immunogenicity.
The term *"patient"* refers to patients of human or other mammal origin and includes any individual it is desired to examine or treat using the methods disclosed herein. However, it will be understood that *"patient"* does not imply that symptoms are present. Suitable mammals that fall within the scope of the invention include, but are not restricted to, primates, livestock animals (e.g., sheep, cows, horses, donkeys, pigs), laboratory test animals (e.g., rabbits, mice, rats, guinea pigs, hamsters), companion animals (e.g., cats, dogs) and captive wild animals (e.g., foxes, deer, dingoes).
By *"pharmaceutically acceptable carrier"* is meant a solid or liquid filler, diluent or encapsulating substance that may be safely used in any kind of administration.
The term *"related disease or condition"* is used herein to refer to a disease or condition that is related anatomically, physiologically, pathologically and/or symptomatically to a reference disease or condition. For example, diseases or conditions may be related to one another by affecting similar anatomical locations (e.g., affecting the same organ or body part), affecting different organs or body parts with similar physiological function (e.g., the oesophagus, duodenum and colon which rely an peristalsis to move food from one end of the alimentary canal to the other), by having similar or overlapping pathologies (e.g., tissue damage or rupture, apoptosis, necrosis) or by having similar or overlapping symptoms (i.e., allergic response, inflammation, lymphocytosis). Thus, for example, an antigen associated with ulcerated colitis may also be associated with perforation of the colon because these disease affects the same organ (i.e., colon).

The term *"treating"* is used herein in its broadest sense to include both therapeutic and prophylactic (i.e., preventative) treatment designed to ameliorate the disease or condition.

The term *"episome"* is used herein to refer to a portion of genetic material that can exist independent of the main body of genetic material (chromosome) at some times or continuously and replicate autonomously, while at other times is able to integrate into the chromosome. Examples of episomes include insertion sequences, transposons and the TTV of the invention.

### Figure legends

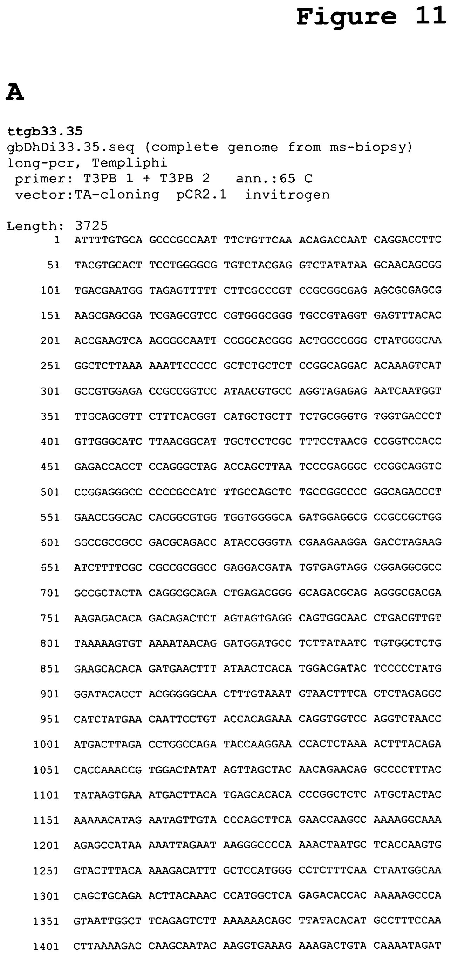

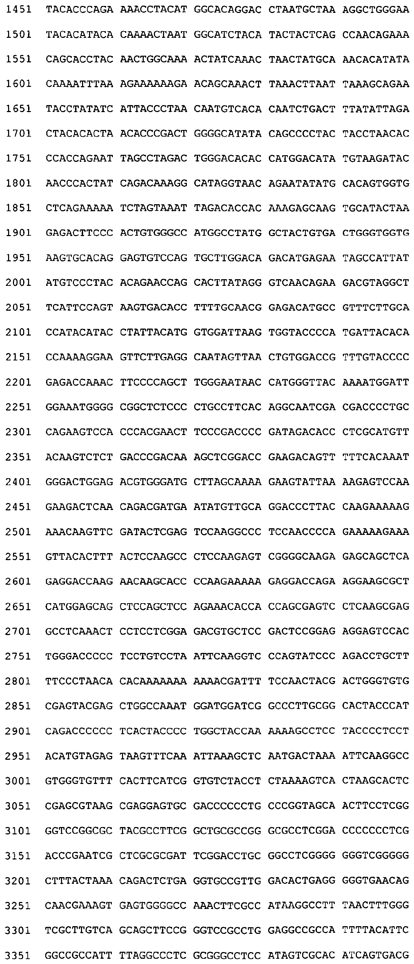

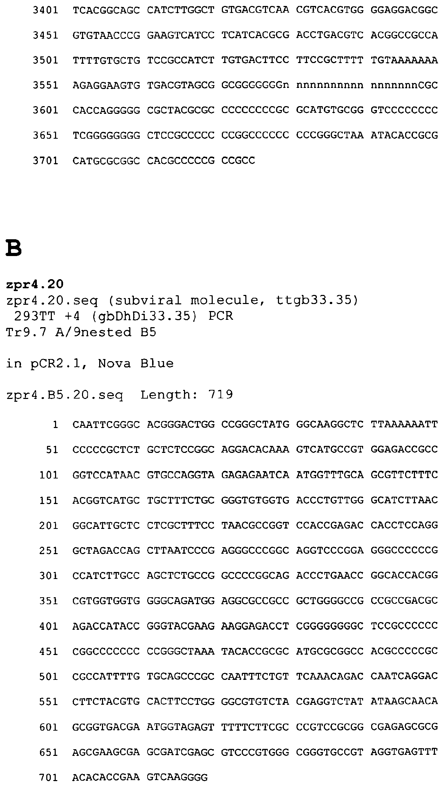

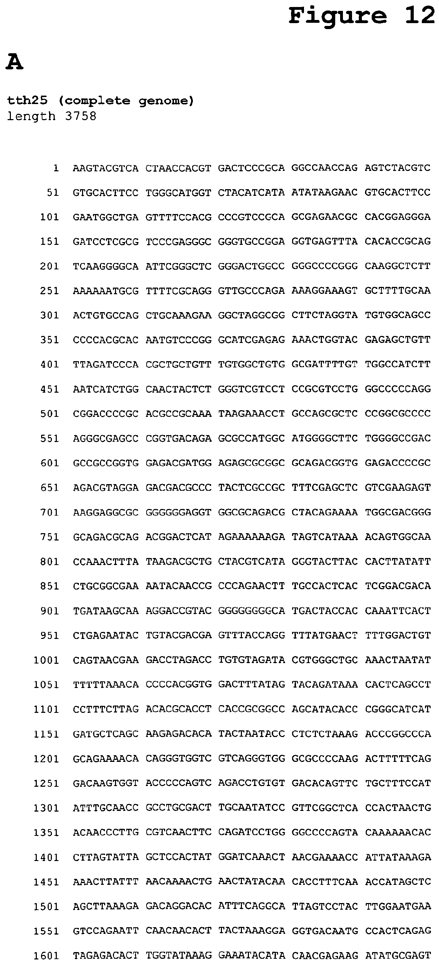

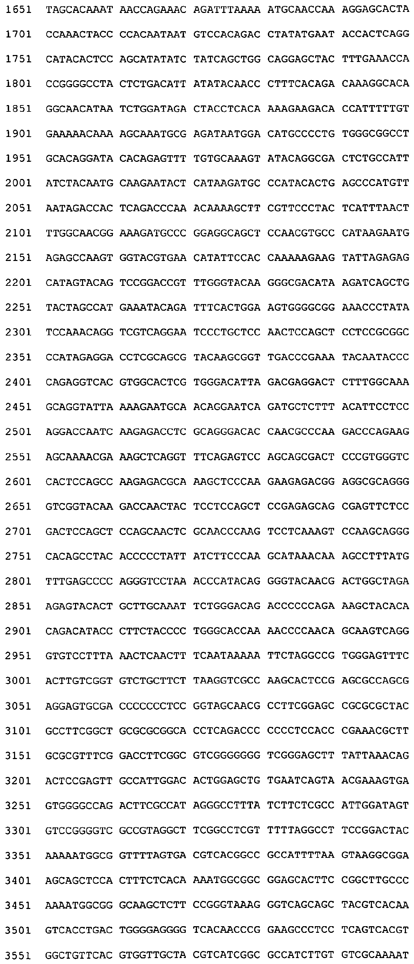

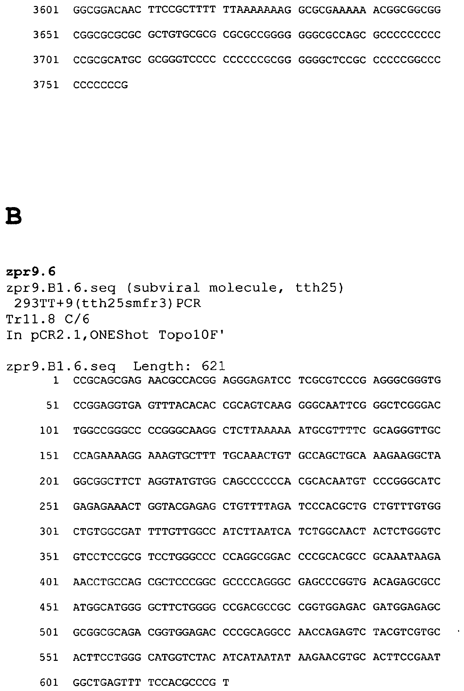

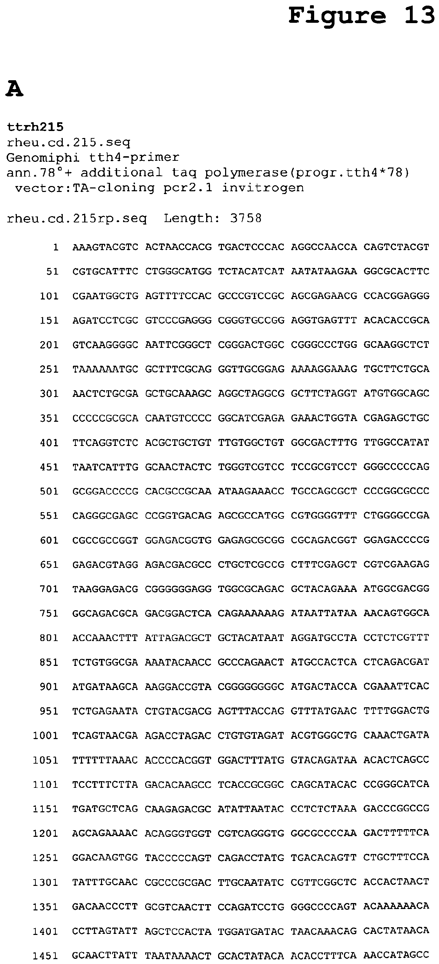

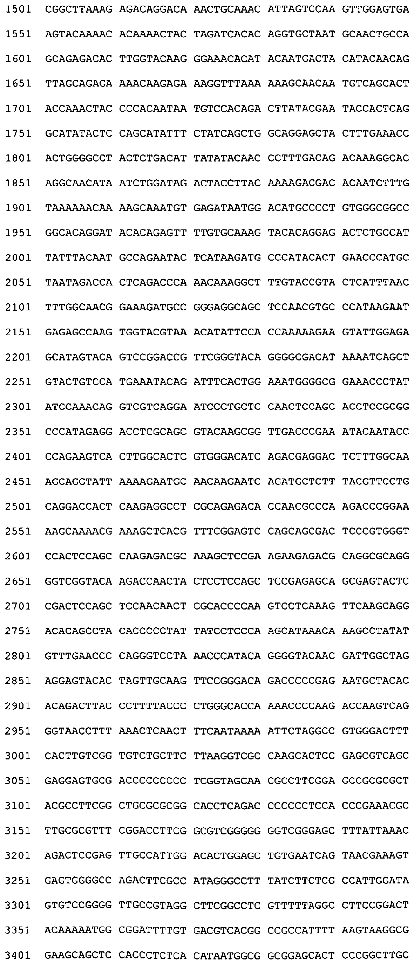

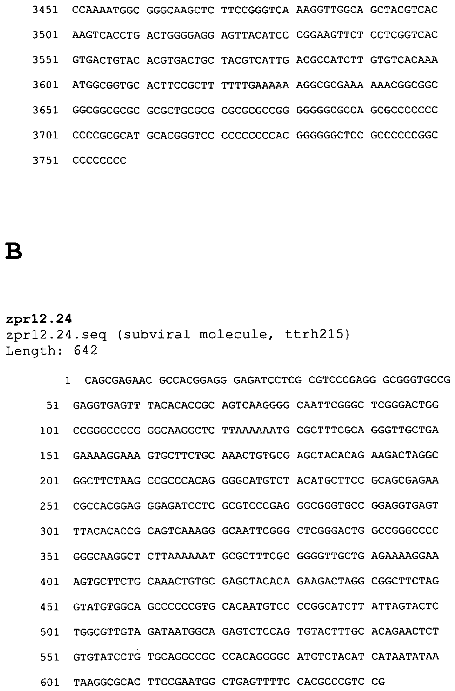

Figure 1: PCR amplification of a 71 base fragment containing the highly conserved TTV region (HCR) in 4 different cell lines, *L1236* (EBV-negative Hodgkin's lymphoma line), *HSB-2* (acute lymphoblastic leukemia line), KR and *IGL* (melanoma cell lines) and placenta DNA
Figure 2: Spooled DNA remaining in the supernatant of L1236 cells after precipitation and removal of high molecular weight DNA and RNase digestion
   Two bands are visible in the region between 4.3 and 6.6 base bands.
Figure 3: Outwards-directed long-PCR, using primers of the 71 base TTV HCR region in HSB-2 DNA
   Two bands are visible in regions corresponding to 4.5 to 7 kb. In addition, bands emerge in the region corresponding to 0.4 to 0.7 kb.
Figure 4: Schematic outline of the TTV oncogene concept
   The left part represents the genomic organization of wild-type TTV genomes. The right part envisages the integration of host cell DNA into the single-stranded plasmids.
Figure 5: Schematic outline of the TTV host cell DNA autoimmunity concept
   The modified host cell genes should code for immuno-reactive antigenic epitopes.
Figure 6: PCR amplification of the 71 base highly conserved region (HCR) from the DNA of 4 different cell lines
   The arrows point to the two sites with variations in the nucleotide sequences.
Figure 7:
   **(A)** The autonomously replicating 719 base TTV DNA (right) and the complete TTV sequence from which it is derived. The nucleotide composition of both molecules is found in Figure 11A+B.
   **(B)** The autonomously replicating 621 base TTV DNA (right) and the complete DNA sequence from which it is derived. The nucleotide composition of both molecules is found in Figure 12A+B.
   **(C)** The autonomously replicating 642 base TTV DNA (right) and the complete DNA sequence from which it is derived. The nucleotide composition of both molecules is found in Figure 13A+B.

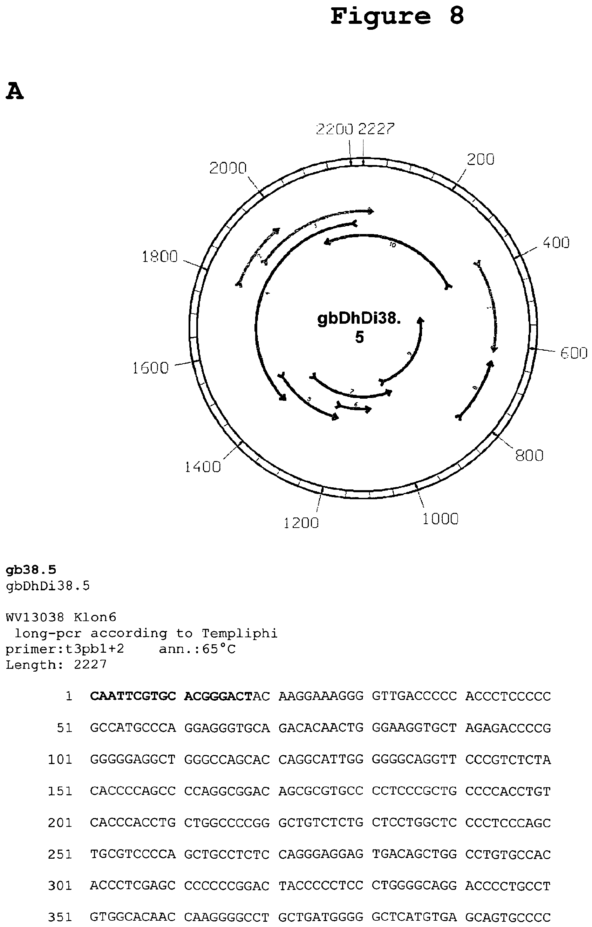

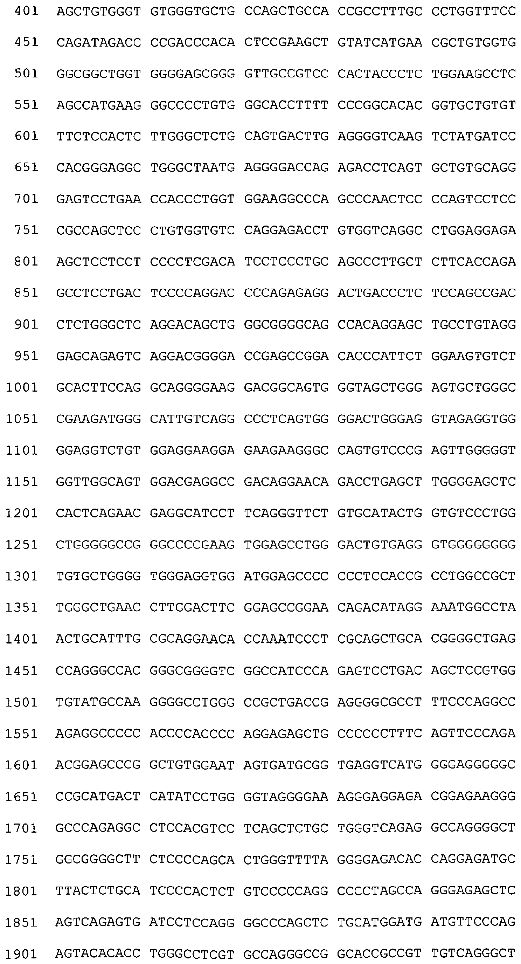

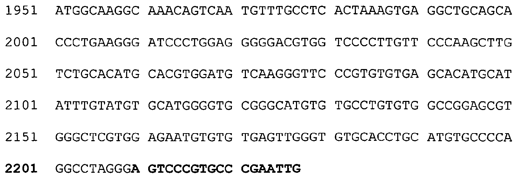

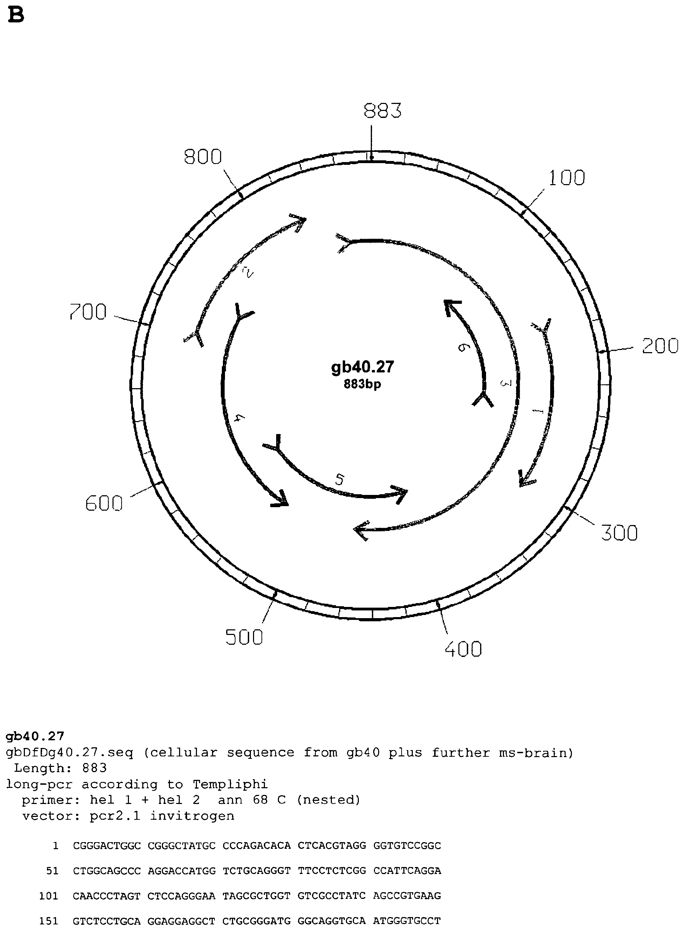

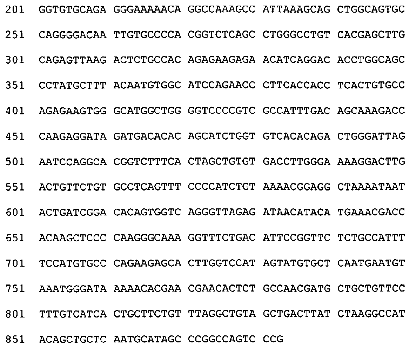

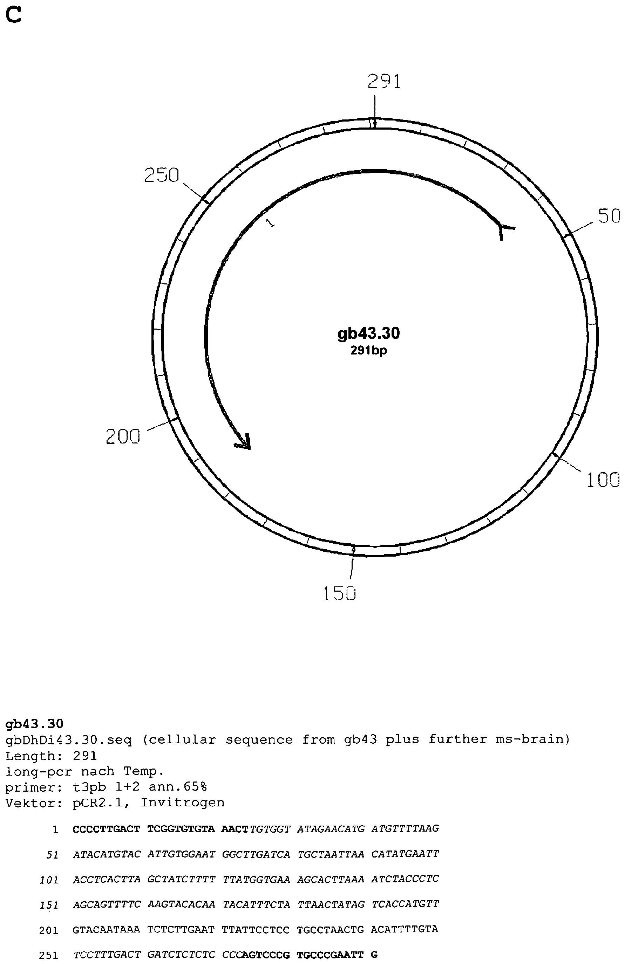

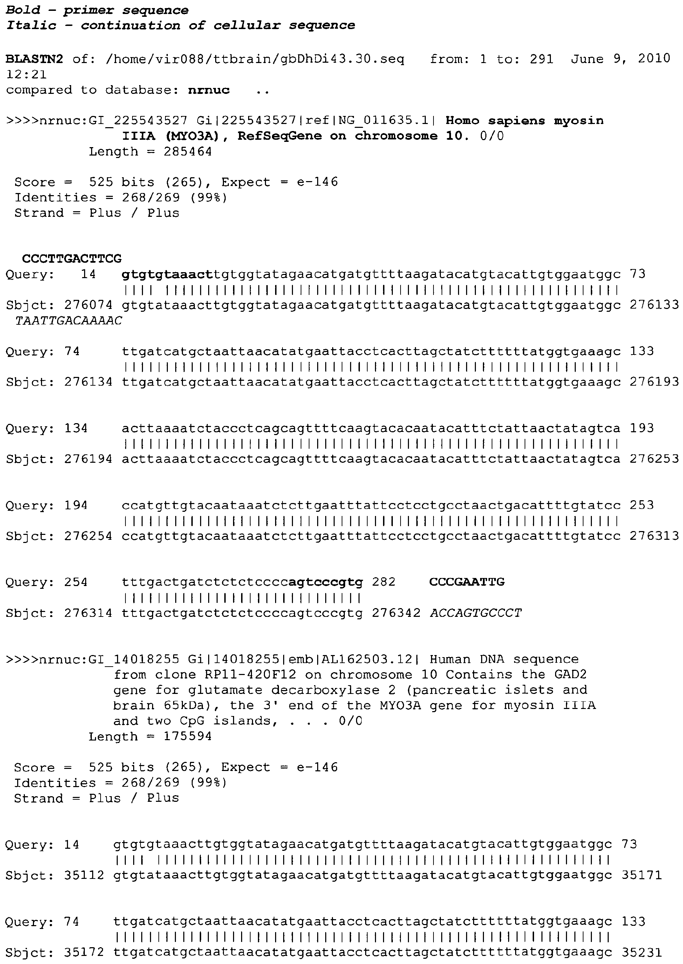

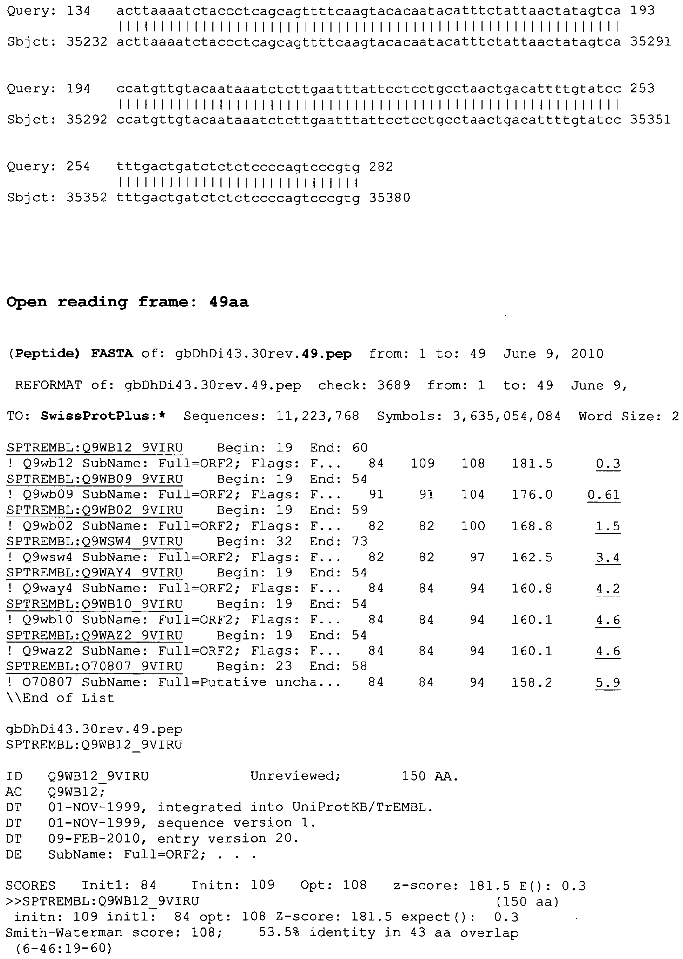

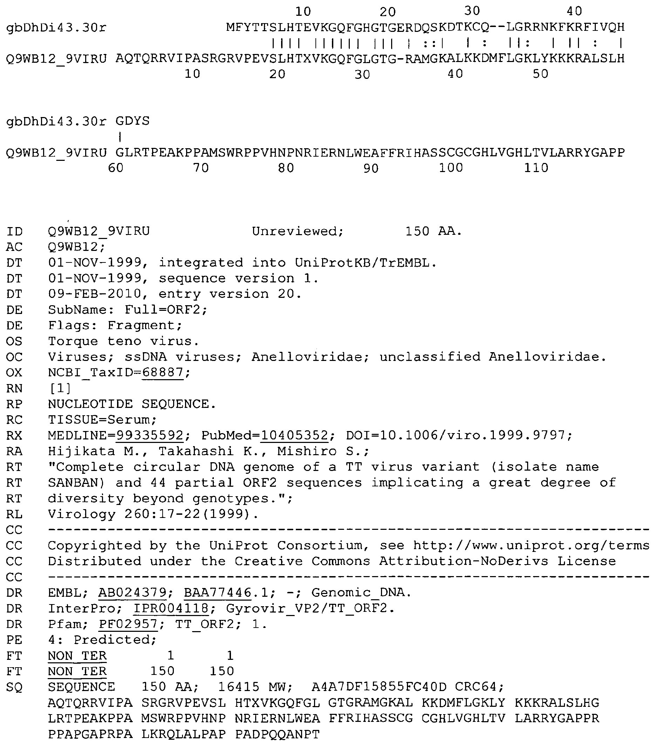

Figure 8: Three exemplary chimeric TTV / truncated host cell DNA sequences from brain biopsies of patients with multiple sclerosis
   **(A)** Chimeric cellular sequences derived from chromosome 1 with some homologies to prion and Wilms tumor sequences and the 3' end of myeloid lymphoid leukemia 3 (MLL3) pseudogene. Human DNA sequence from clone RP11-14N7 on chromosome 1. Contains 3'end of a myeloid/lymphoid or mixed lineage leukemia 3 (MLL3) pseudogene, a seven transmembrane helix receptor pseudogene, the 5'-end of a novel gene.
   **(B)** Chimeric cellular sequences derived from chromosome 16. Homologies to transcription factor 3 (TF 3C), protein signatures for chemokine receptors and leukotriene B4 receptor.
   **(C)** Chimeric cellular sequences derived from chromosome 10, truncated sequence of myosin, reactivity reported for multiple sclerosis patients and those with rheumatoid arthritis (sequence contains both full primers front and back).

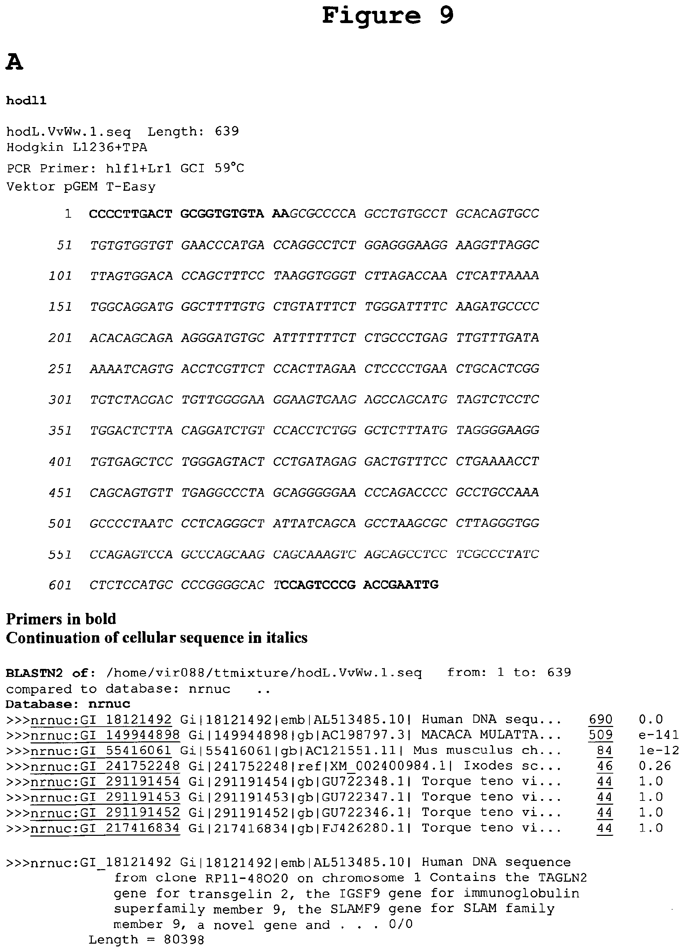

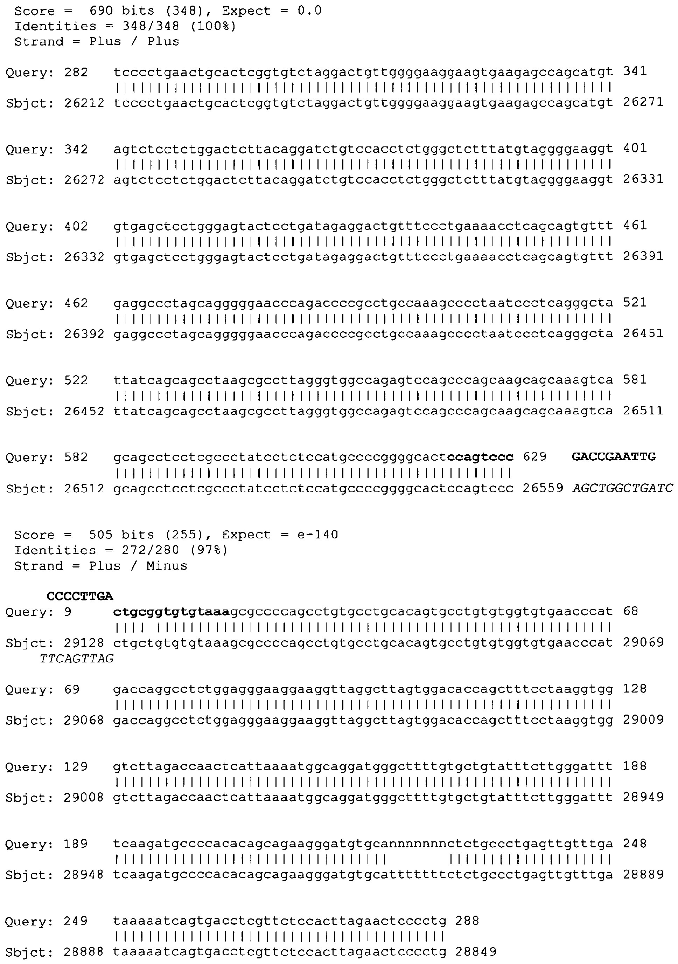

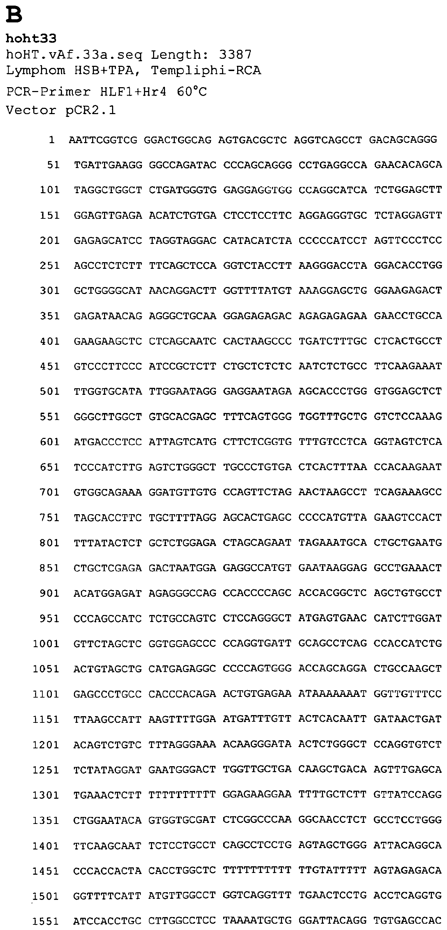

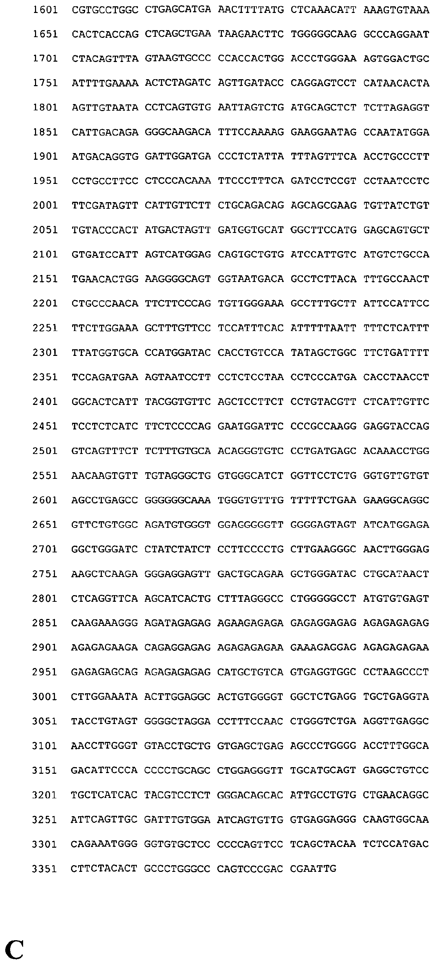

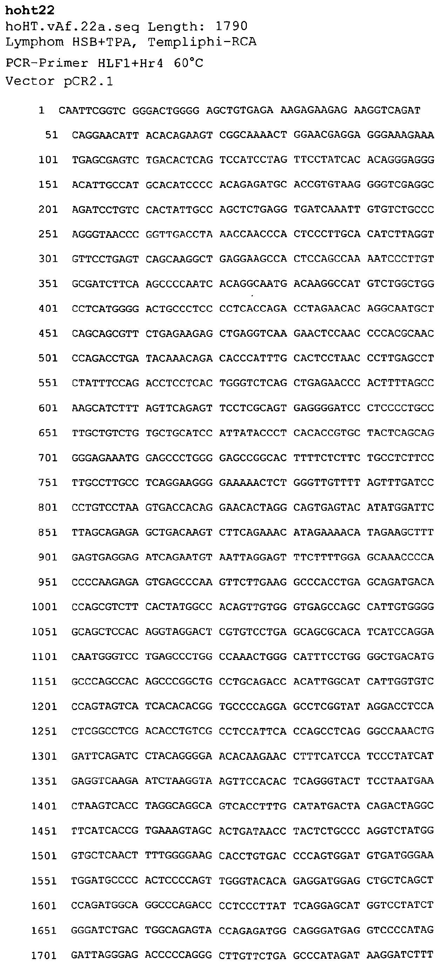

Figure 9: Three exemplary chimeric TTV/truncated host cell DNA sequences from cell lines derived from patients with Hodgkin's disease or leukemia
   **(A)** Chromosome 1 sequences with part of transgelin 2, the IGSF9 gene for immunoglobulin superfamily member 9, the SLAM9 gene.
   **(B)** Translated protein sequences with substantial homology to the oncogenes v-myb (avian myeloblastosis viral oncogene), but also to c-myb. This sequence was amplified with the forward primer at both ends.
   **(C)** Derived from chromosome 10. High homology with "Deleted in malignant 1 Protein" (DMBT), an identified tumor suppressor gene. This sequence was amplified with the forward primer at both ends.

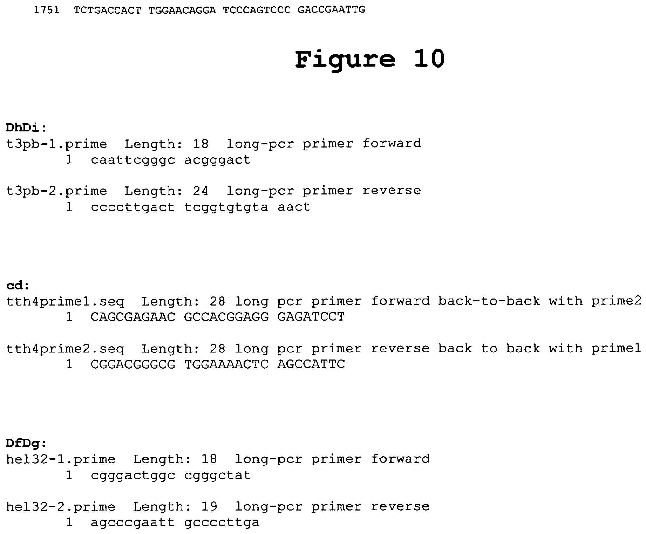

Figure 10: Primer sequences used in the reactions described in the Examples, derived from the 71 base HCR.
Figure 11:
   **(A)** Complete TTV sequence from which autonomously replicating 719 base DNA has been obtained.
   **(B)** Complete sequence of the autonomously replicating 719 base TTV DNA.
Figure 12:
   **(A)** Complete TTV sequence (tth25) from which autonomously replicating 621 base DNA has been obtained.
   **(B)** Complete sequence of the autonomously replicating 621 base TTV DNA.
Figure 13:
   **(A)** Complete TTV sequence (ttrh215) from which autonomously replicating 642 base DNA has been obtained.
   **(B)** Complete sequence of the autonomously replicating 642 base TTV DNA.

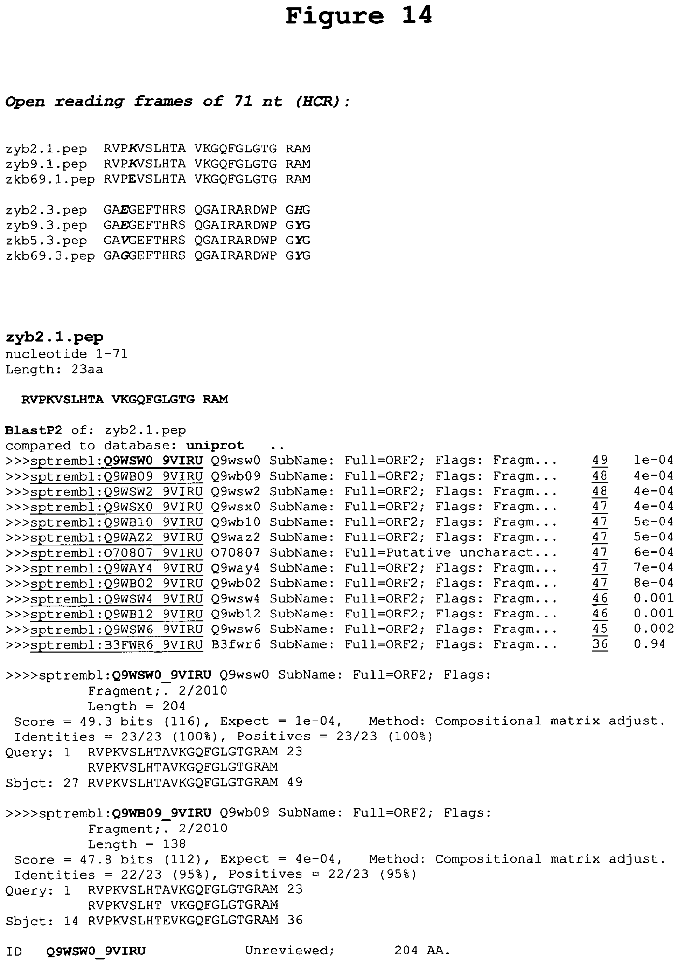

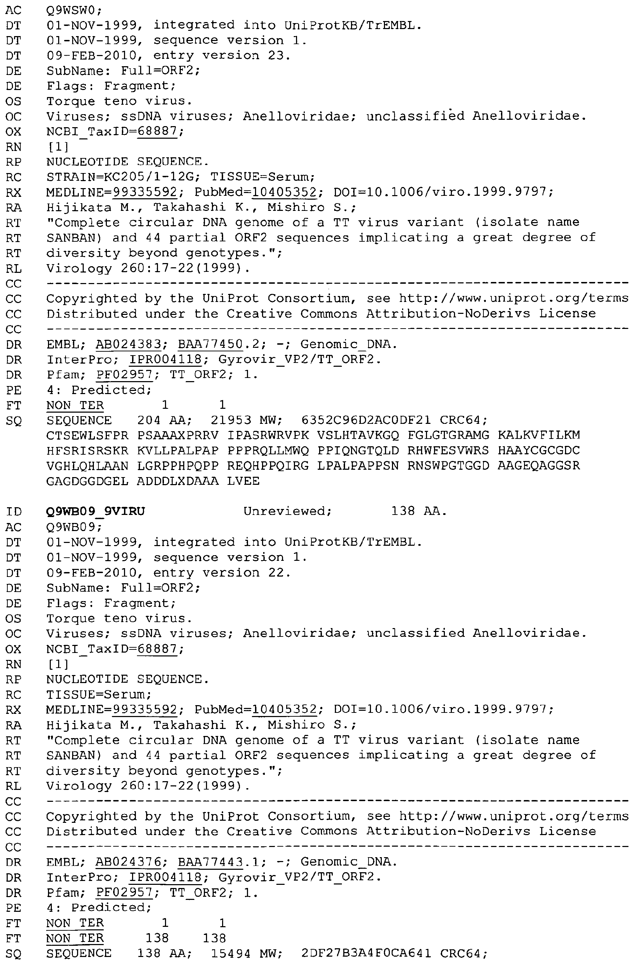

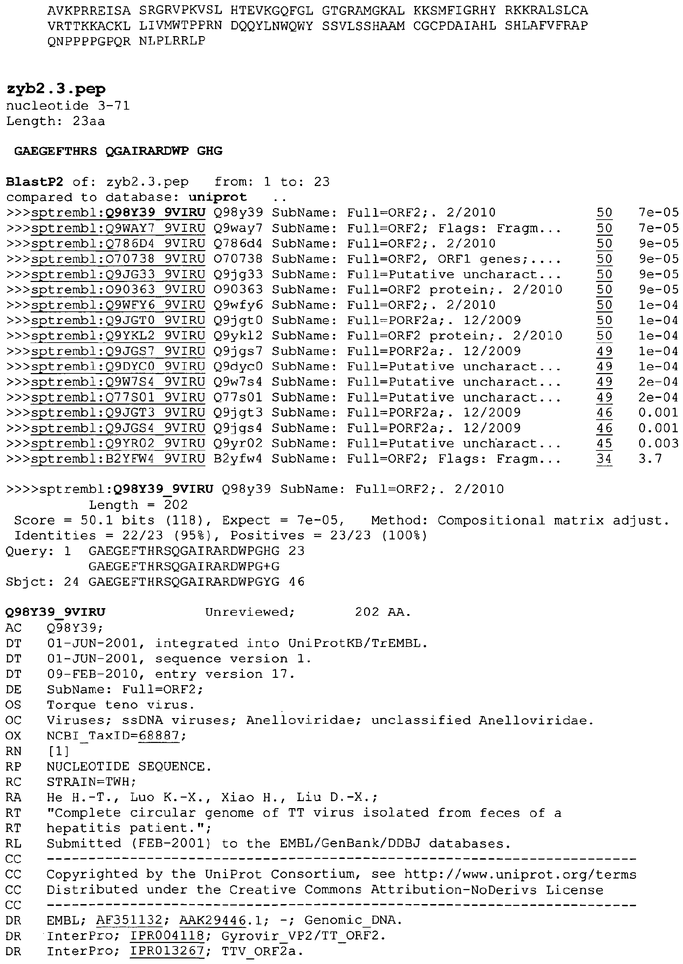

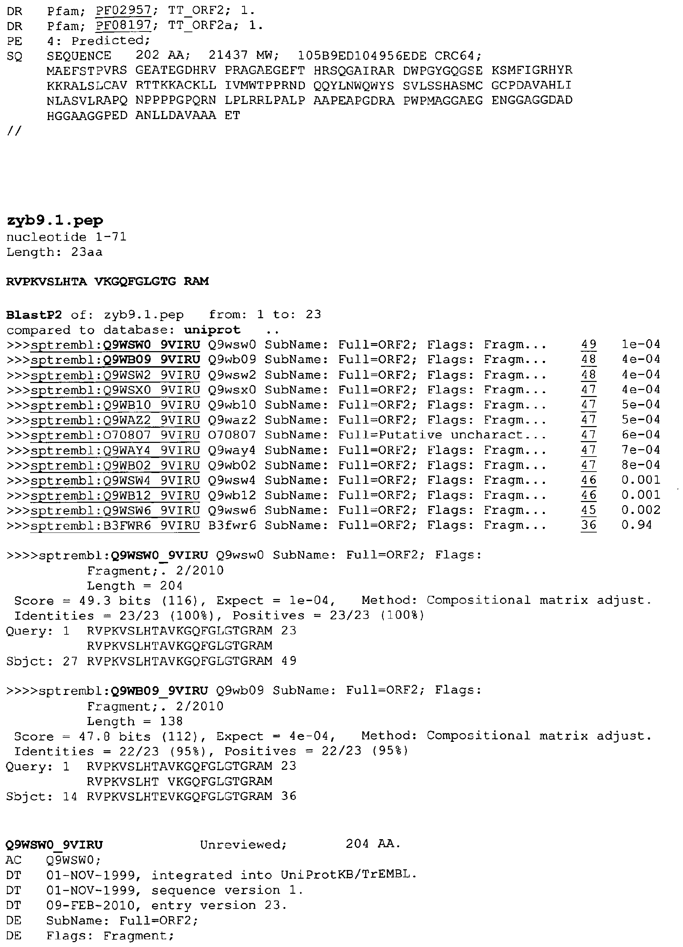

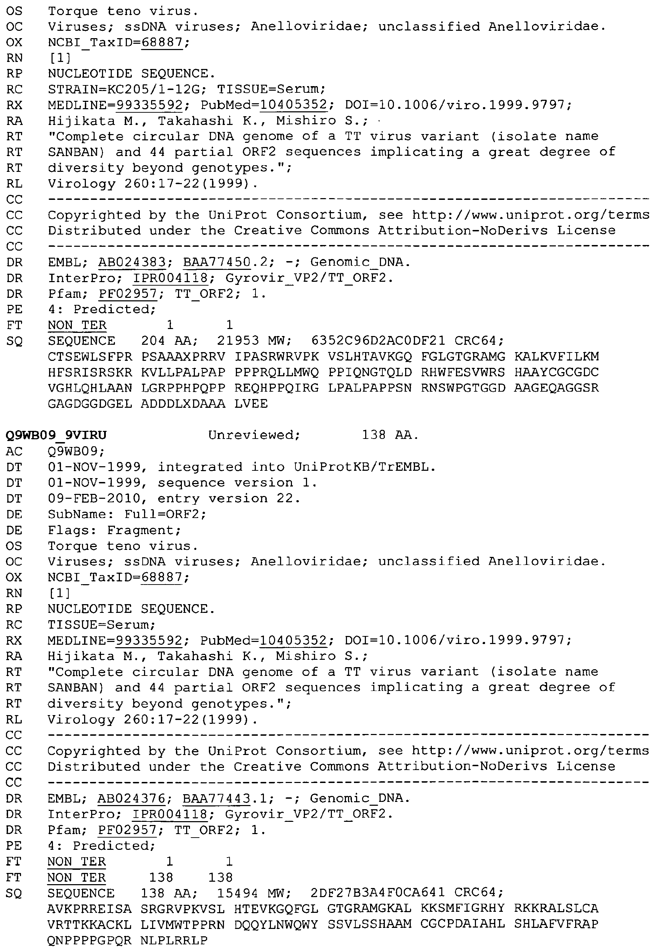

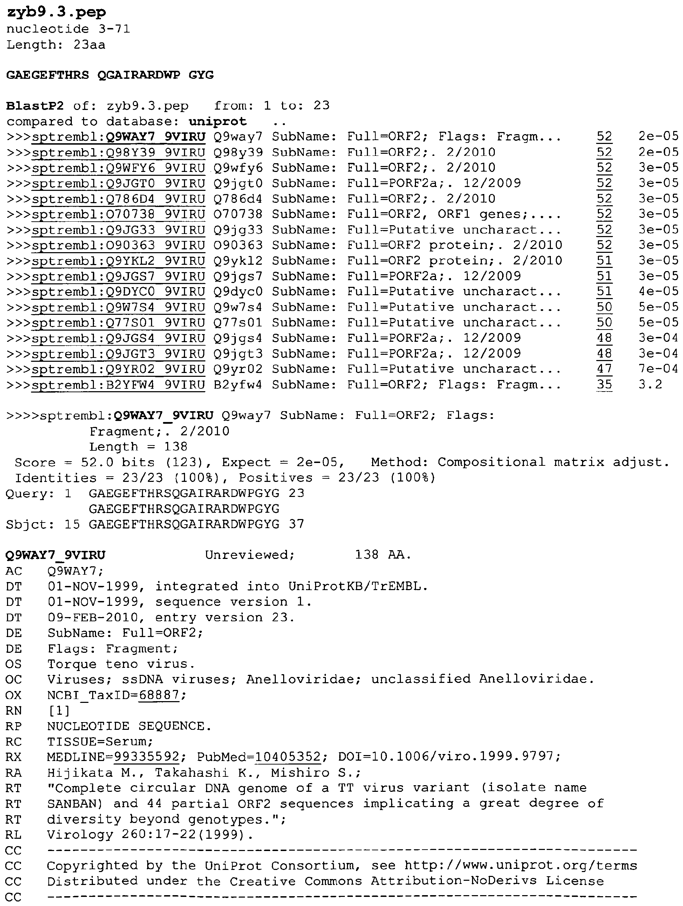

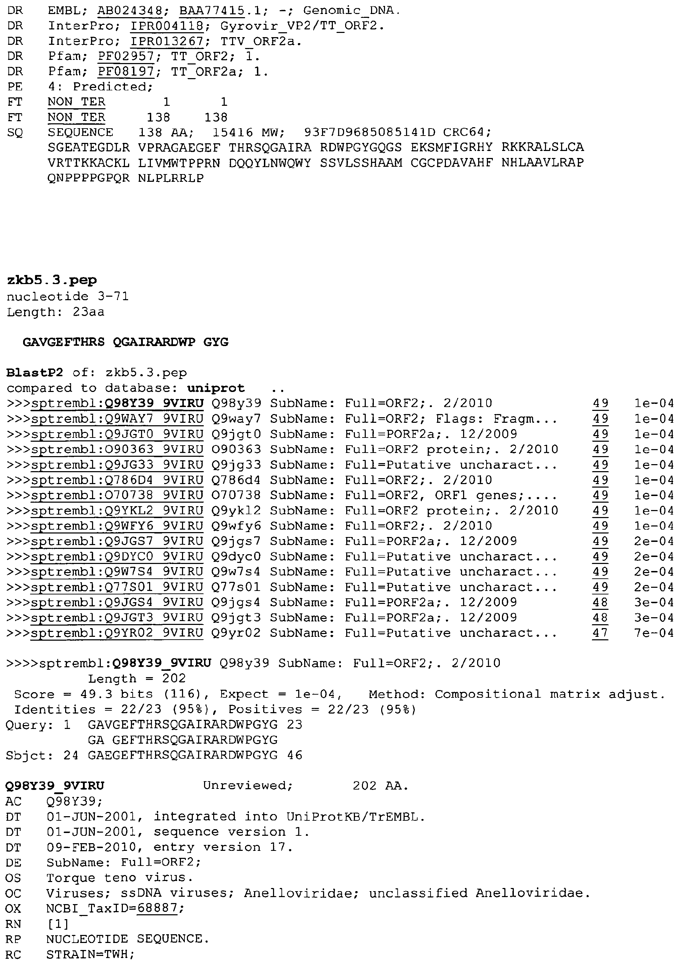

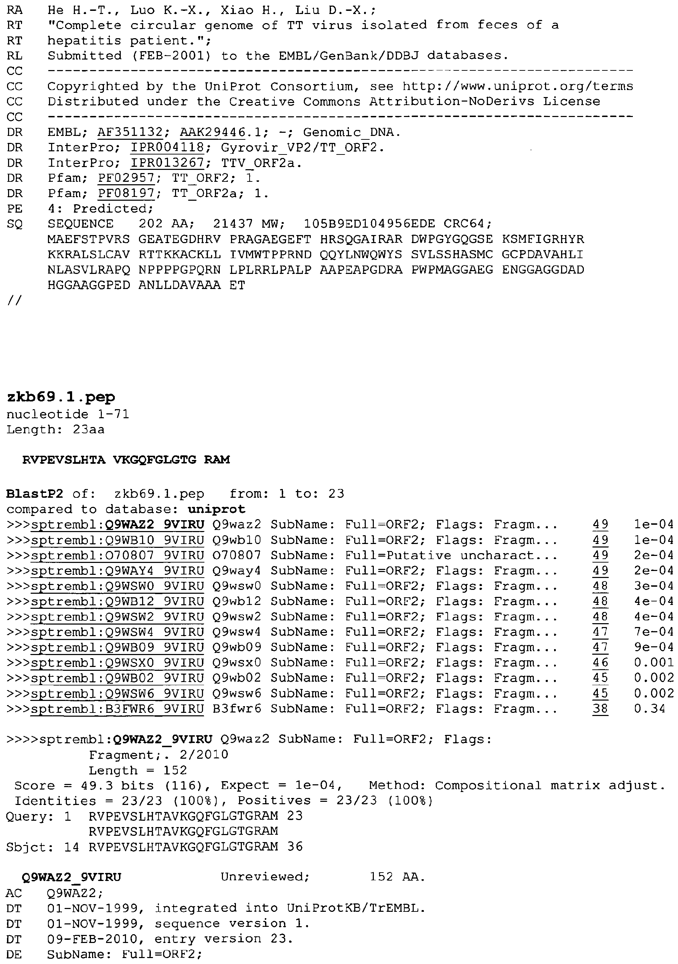

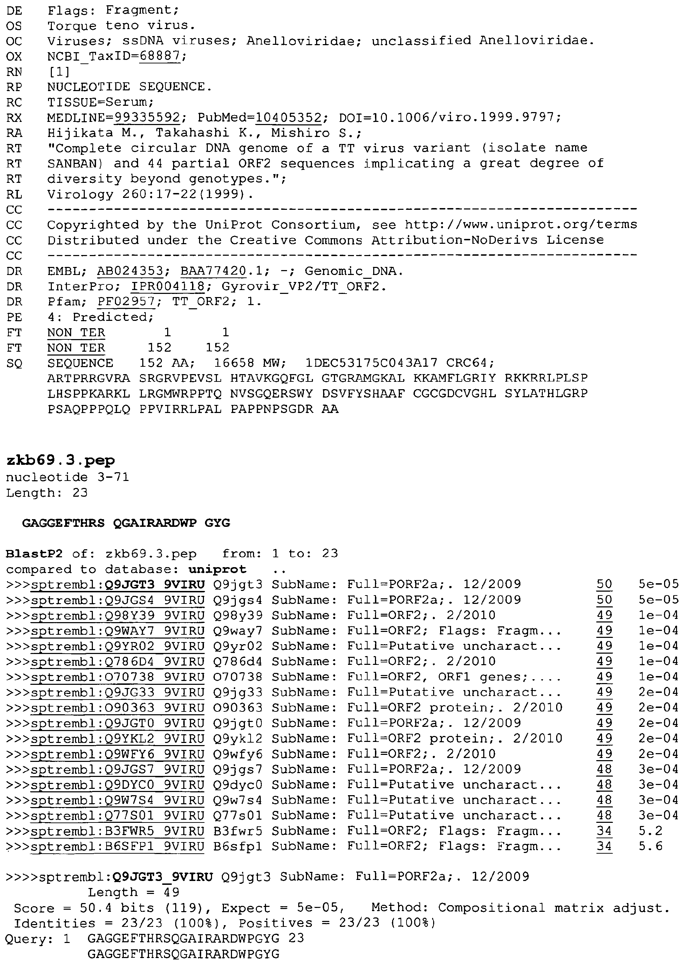

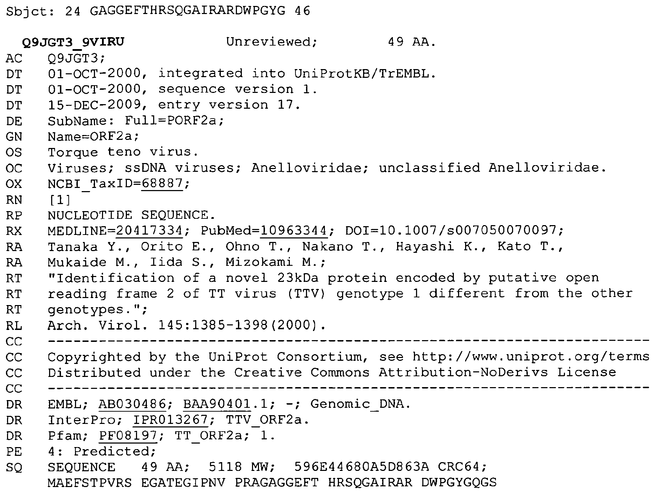

Figure 14: Open reading frames (ORFs) found within the nucleotide sequence of 71 nt
   zyb2.1.pep, zyb9.1.pep, and zkb69.1.pep are starting at the first triplet, zyb2.3.pep, zyb9.3.pep, zkb5.3.pep, and zkb69.3.pep are starting from the third triplet. This region is actively transcribed.

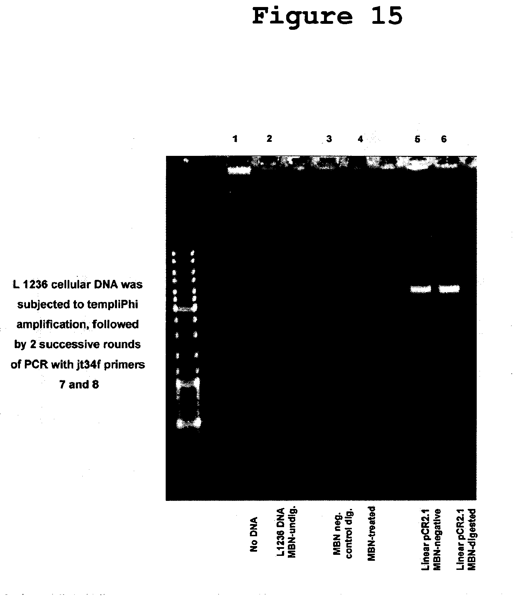

Figure 15: Digestion of single-stranded DNA by mung-bean nuclease (MBN) Lanes 2 and 3 show that the amplified DNA can be digested by pretreatment with MBN. Lanes 5 and 6 demonstrate that plasmid-DNA pretreated in the same way is not digested by MBN.

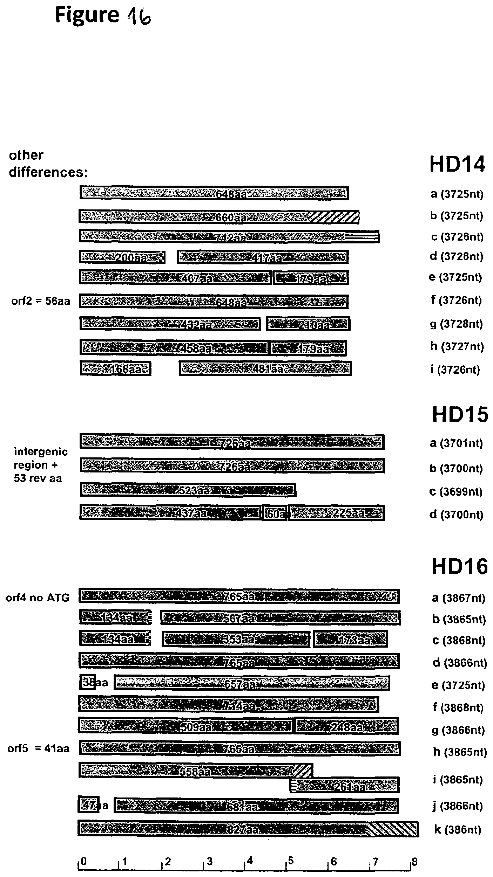

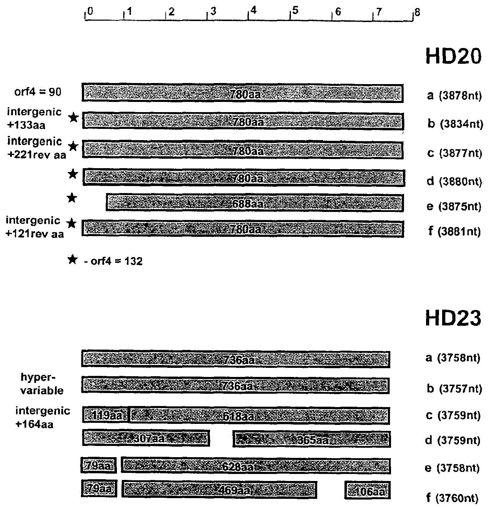

Figure 16: Schematic presentation of the ORF1 of a number of TTV-HD isolates
   ORF1 was either divided into one to several smaller ORFs or fused to other ORFs.

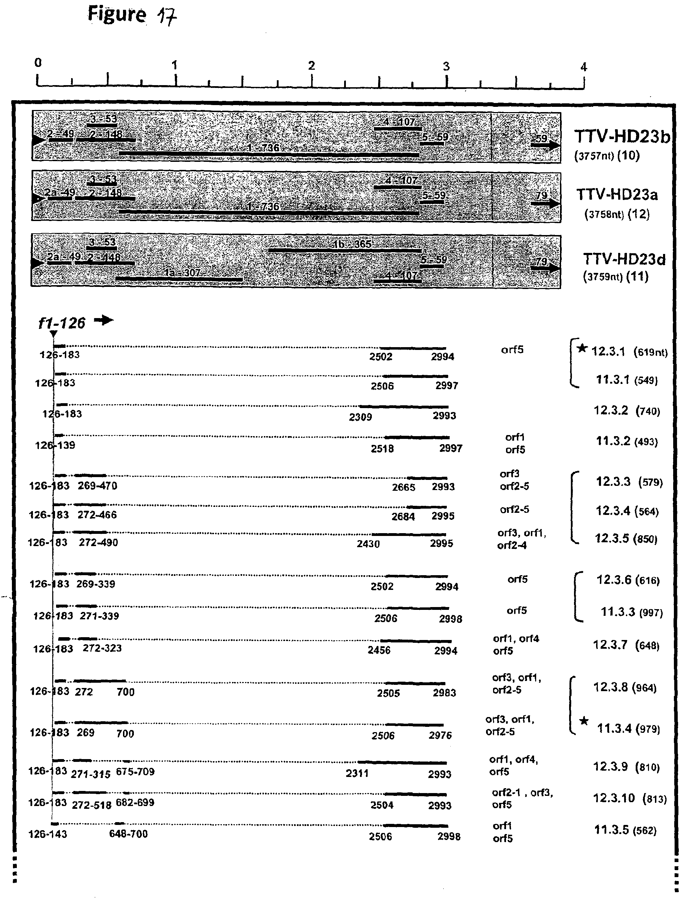

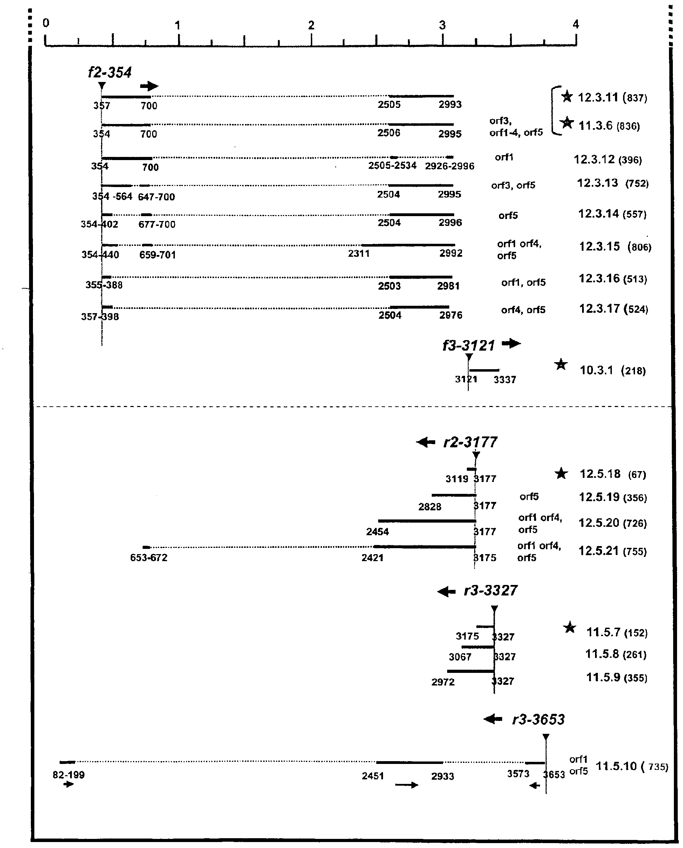

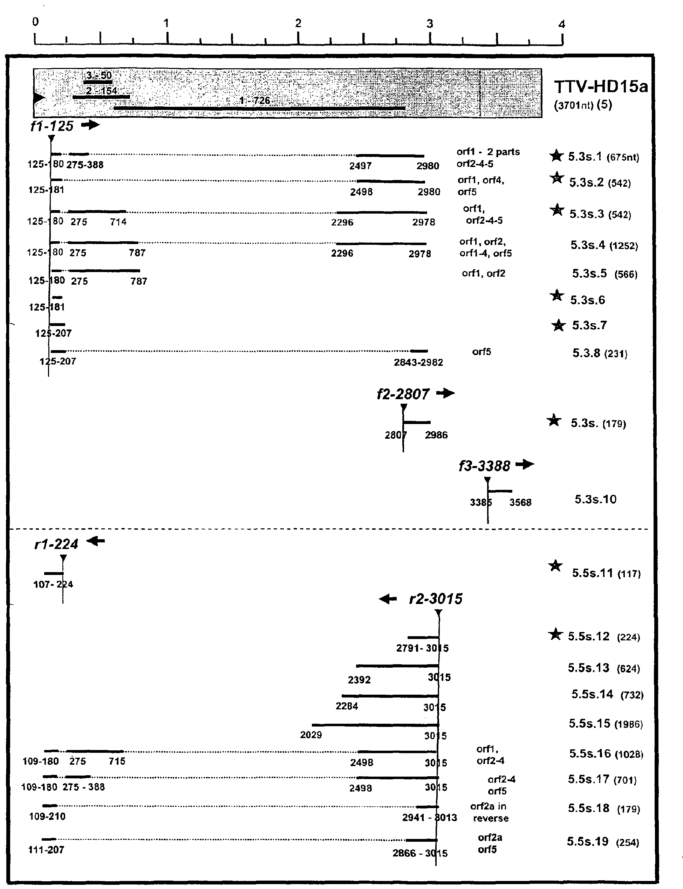

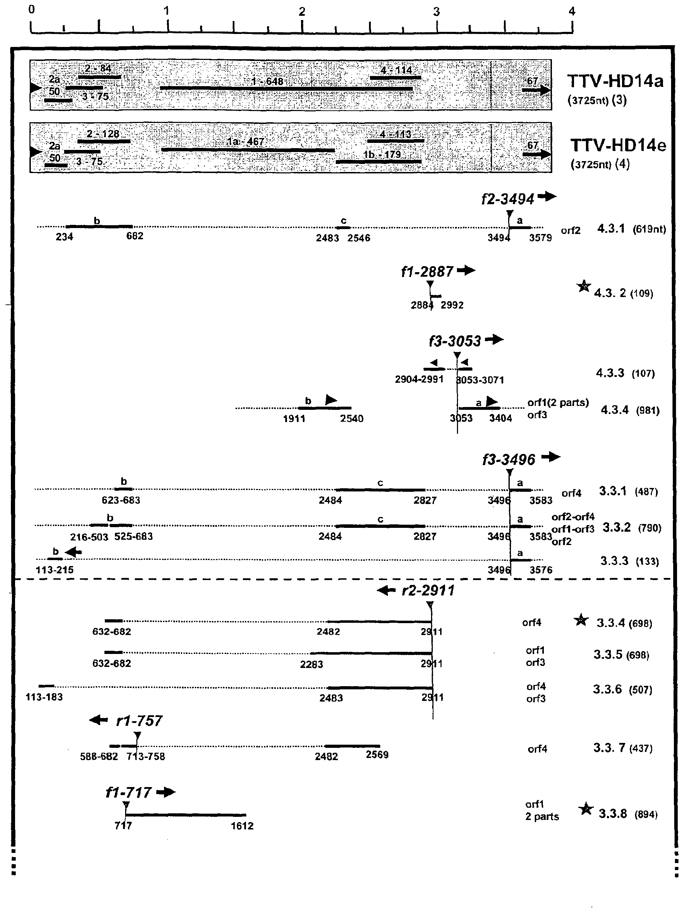

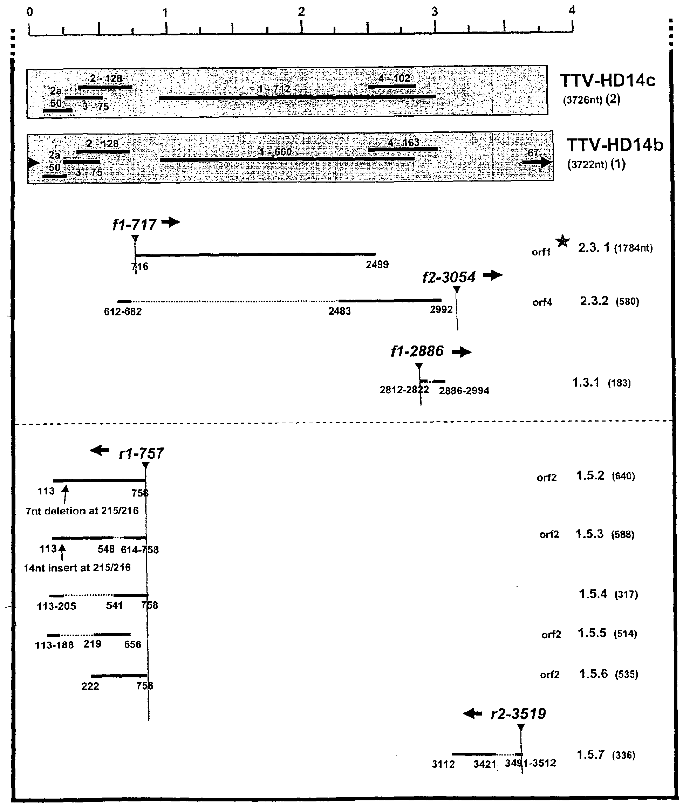

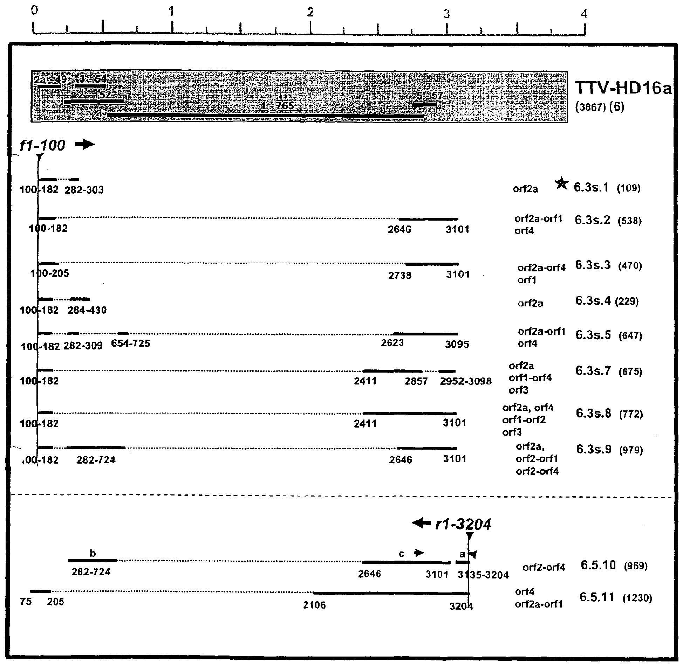

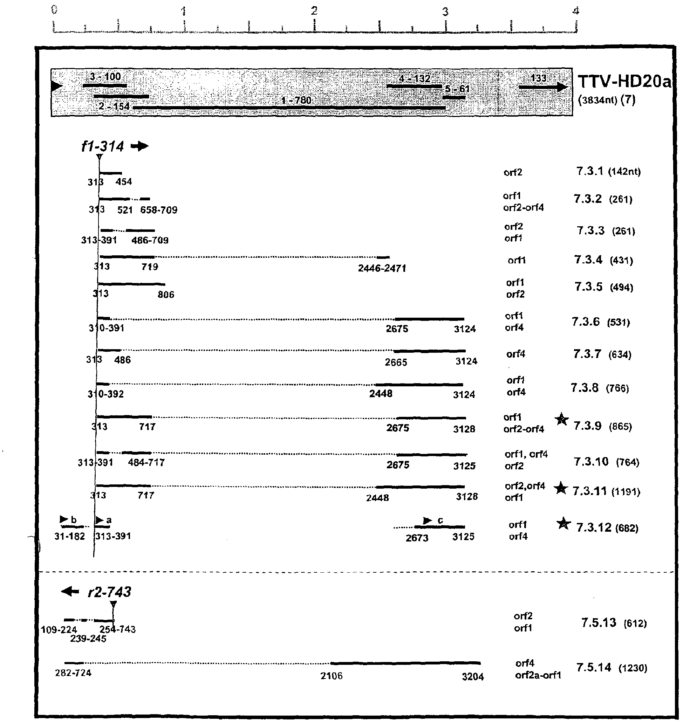

Figure 17: Transcripts isolated during in vitro replication of TTV-HD isolates
   Labelling of individual transcripts indicates "isolate.5'- or 3'-race (s - single strand).no". TTV-isolate numbers (1-12) indicated with respective schematic genome and TTV-HD number. * - transcripts which were more often isolated.
Figure 18: Phylogenetic tree showing TTV species and isolates of genus Alphatorquevirus, as well as all TTV-HD types TTV-HD types propagated in in vitro cell cultures are encircled.
Figure 19: Propagation of full-length TTV-HD genomes in 293TT cells Examples of propagation of
   **(A)** TTV-HD14b, TTV-HD14c, TTV-HD14a, and TTV-HD14e (lanes 1-4), TTV-HD15a (lane 5) and TTV-HD16a (lane 16) after nested PCR amplification;
   **(B)** TTV-HD20a (lane 7), TTV-HD3a (lane 8), TTV-HD1a (lane 9), TTV-HD23b, TTV-HD23d, and TTV-HD23a (lanes 10-12) after single PCR amplification. a, b and c - examples of propagations, approximately 7 days after infection. b-1, b-2, and b-3 indicate variability observed when propagating same passage.
   **(C)** Daily sampling of TTV-HD14e (nested PCR) and TTV-HD23b cultures. M - DNA size marker; * - indicate subviral molecules of different cultures.

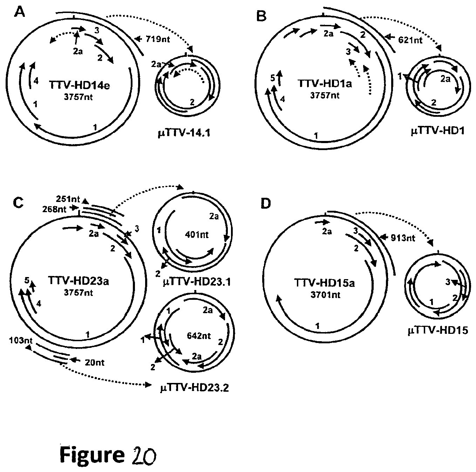

Figure 20: Schematic presentation of full-length TTV-HD with their respective µTTV-HD molecules Numbers indicate ORFs in the DNA genome.
Figure 21: Independent propagation of µTTV-HD µTTV-HD15 replicated stronger after initial transfection, but decreased over time (*-indicate nested PCR amplification). µTTV-HD1 and µTTV-HD23.2 replicated increasingly after additional propagation steps. µTTV-HD23.2 molecules formed during replication of µTTV-HD23.1.
Figure 22:
   **(A)** Partially purified virus-like particles Particles were lysed and content separated on agarose gel.
   **(B)** Partially purified mTTV particles Particles were lysed and DNA content separated on agarose gel.
      3 - TTV-HD14a, 5 - µTTV-14, 6 - TTV-HD16a, 8 - TTV-HD3a, 9 - µTTV-HD1, 12 - TTV-HD23a, 12a - µTTV-HD12.1, 12b - µTTV-HD12.2
   Disclosed herein is a rearranged TT virus polynucleic acid comprising (or consisting of)
   (a) a nucleotide sequence shown in Figure 6;
   (b) a nucleotide sequence which shows at least 70%, 80%, 90%, 95% or at least 98% identity to a nucleotide sequence of (a) and is capable of replicating autonomously;
   (c) a nucleotide sequence which is the complement of the nucleotide sequence of (a) or (b); or
   (d) a nucleotide sequence encoding an amino acid sequence selected from the amino acid sequences zyb2.1.pep, zyb9.1.pep, zkb69.1.pep, zyb2.3.pep, zyb9.3.pep, zkb5.3.pep and zkb69.3.pep shown in Figure 14 and said rearranged TT virus polynucleic acid is capable of replicating autonomously upon transfection in 293TT cells,
   wherein said nucleotide sequence of (a), (b), (c) or (d) is linked to a polynucleic acid encoding a protein containing a signature motif of a protein being associated with cancer or an autoimmune disease via a phosphodiester bond.

The protein is a mammalian protein. Particularly preferably the mammalian protein is a human protein.. Also disclosed herein are fragments of the nucleotide sequences described above that are capable of replicating autonomously. The skilled person can derive at fragments still having the biological activity of the full length molecule without undue experimentation.

The person skilled in the art can easily determine which nucleic acid sequences are related to the nucleotide sequence of Figure 6 or which fragments are still capable of replicating autonomously by using standard assays or the assays described in the examples, below.

The term "polynucleic acid" refers to a single-stranded or double-stranded nucleic acid sequence. A polynucleic acid may consist of deoxyribonucleotides or ribonucleotides, nucleotide analogues or modified nucleotides, or may have been adapted for therapeutic purposes. Preferably, the rearranged TT virus polynucleic acid is a single-stranded DNA.

Preferably, the rearranged TT virus polynucleic acid of the invention is present as an extrachromosomal episome.

The mammalian protein associated with cancer or an autoimmune disease or allergen associated with an autoimmune disease is a protein as shown in Table 1.

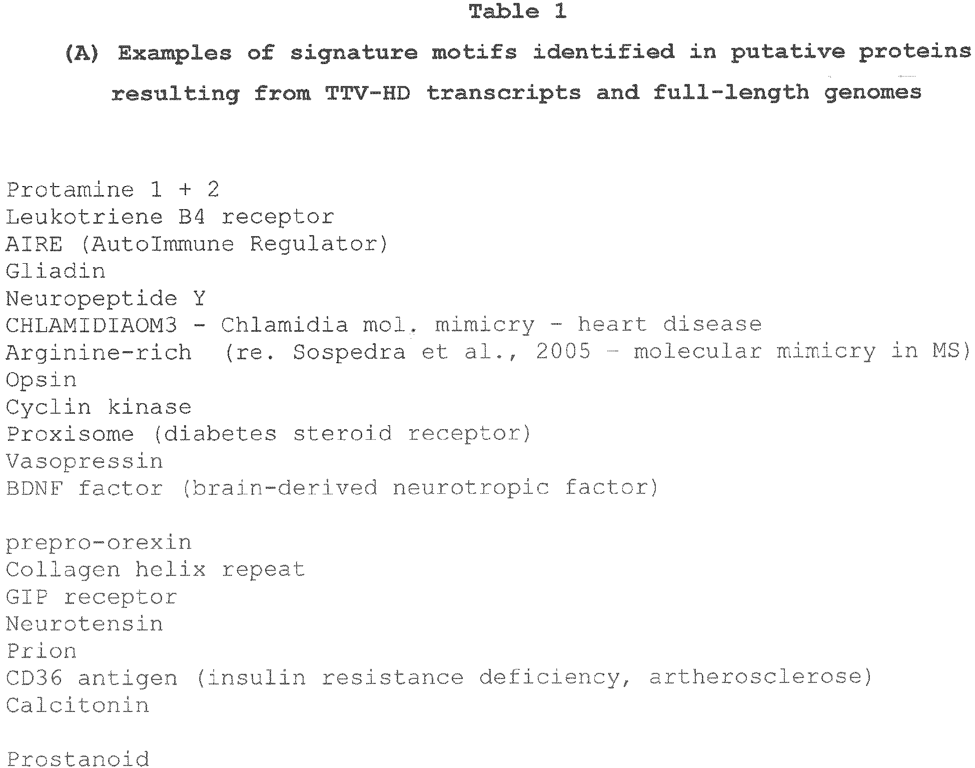

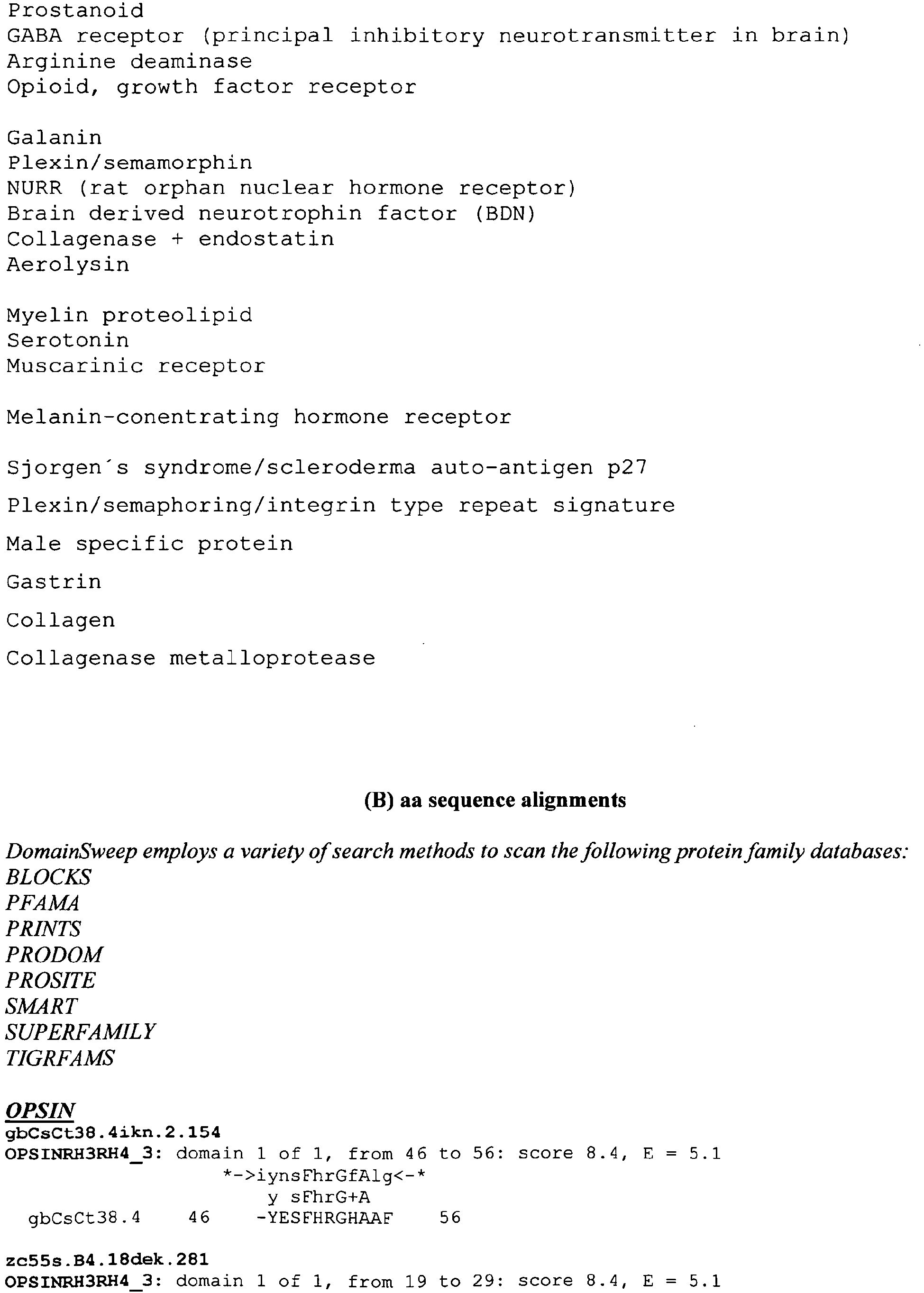

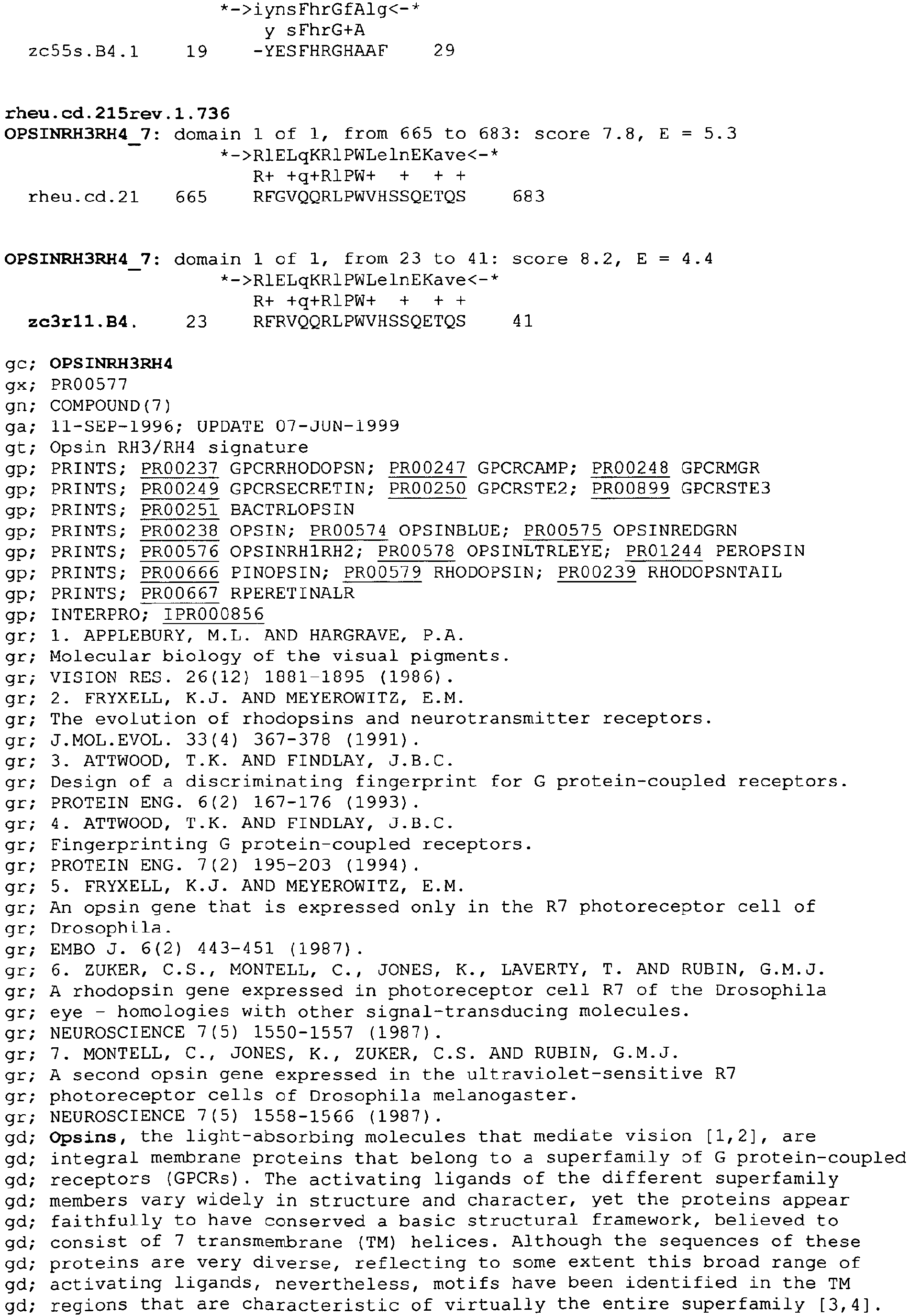

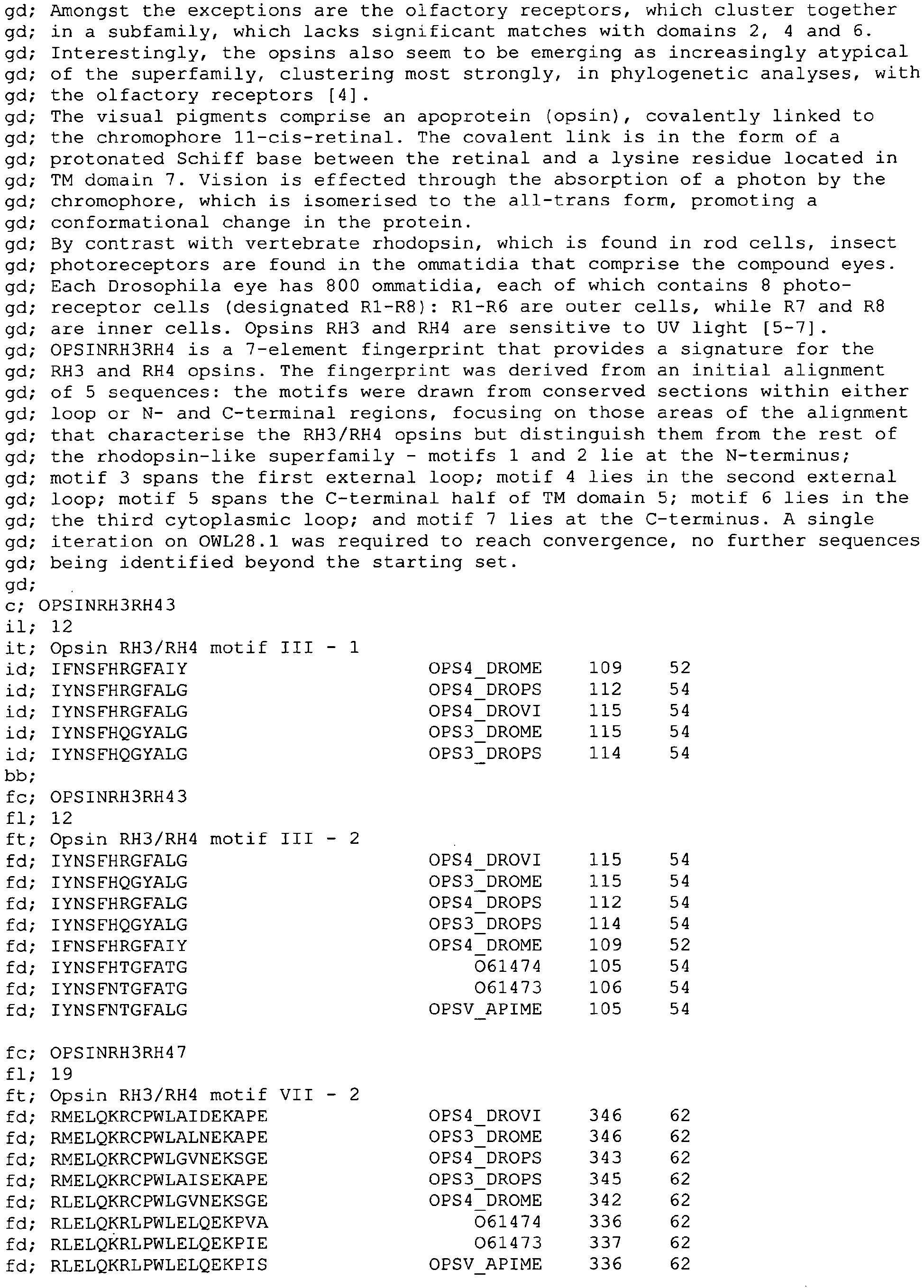

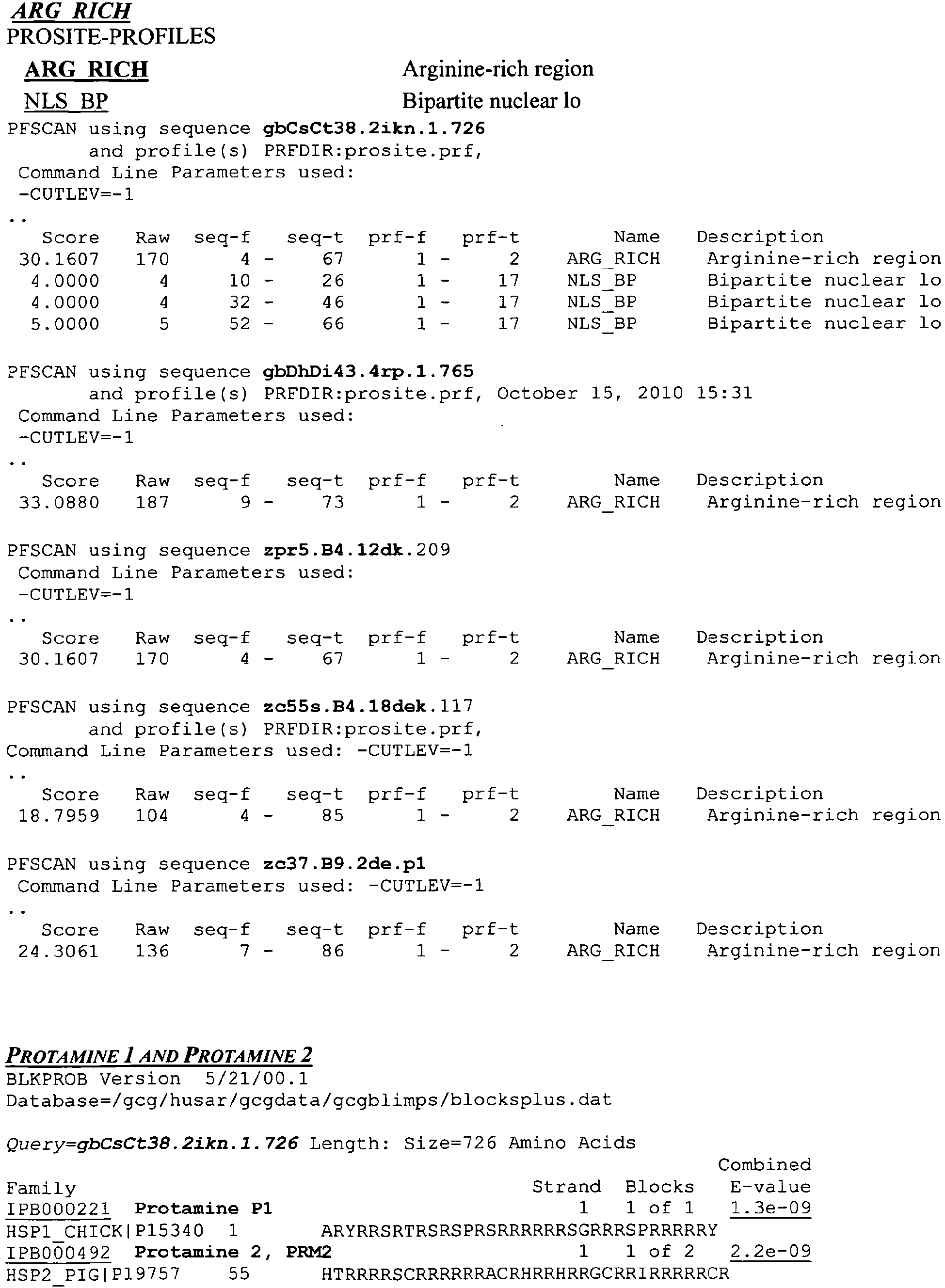

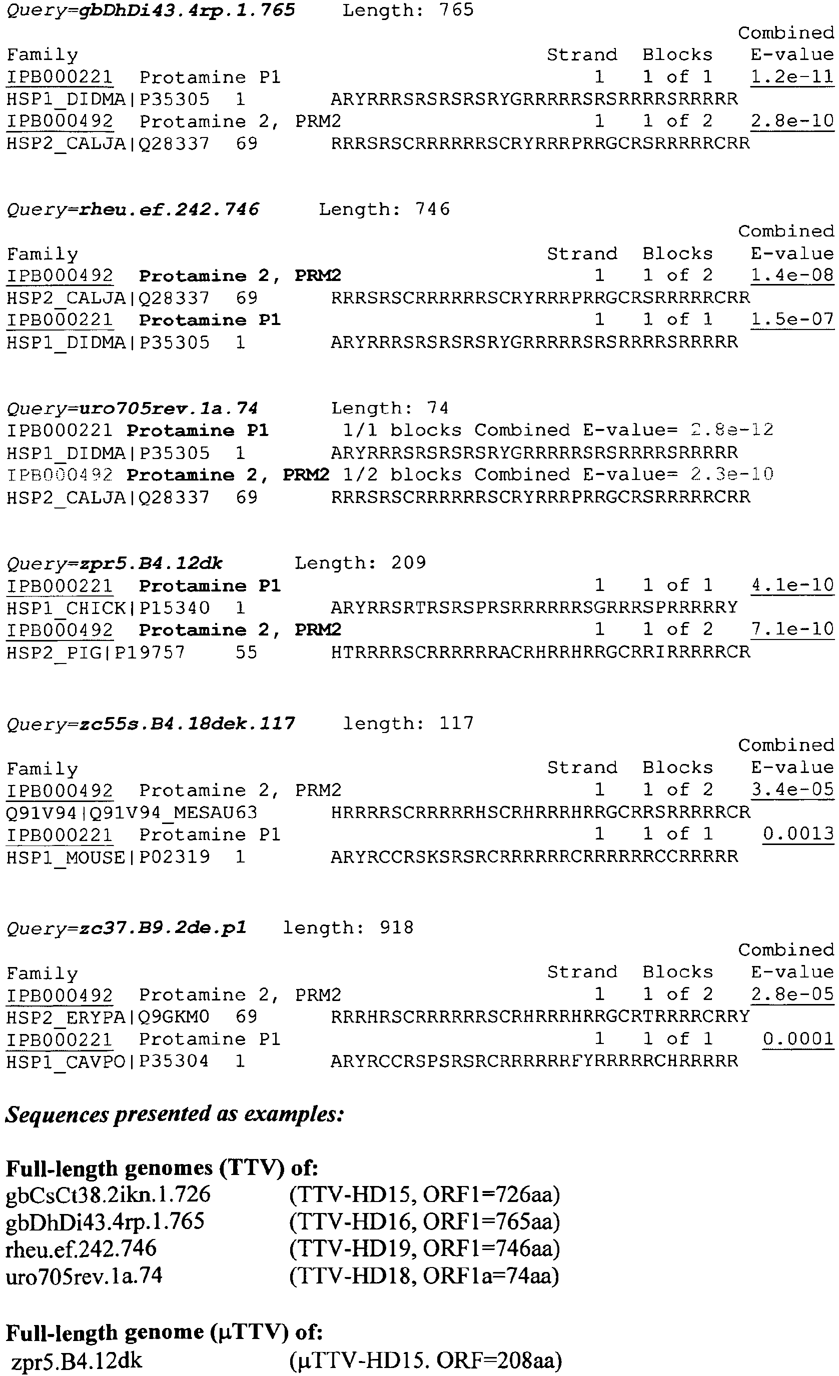

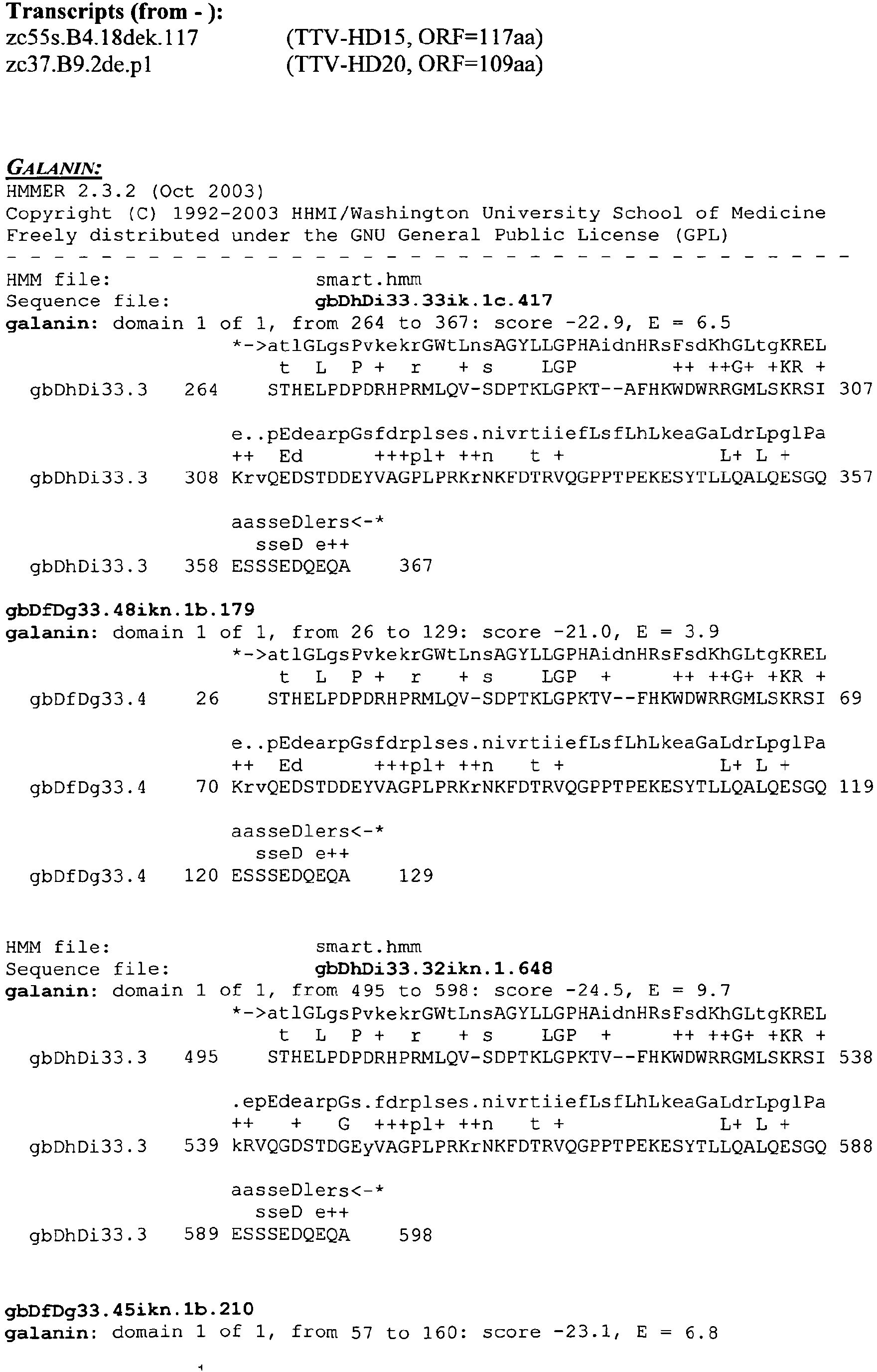

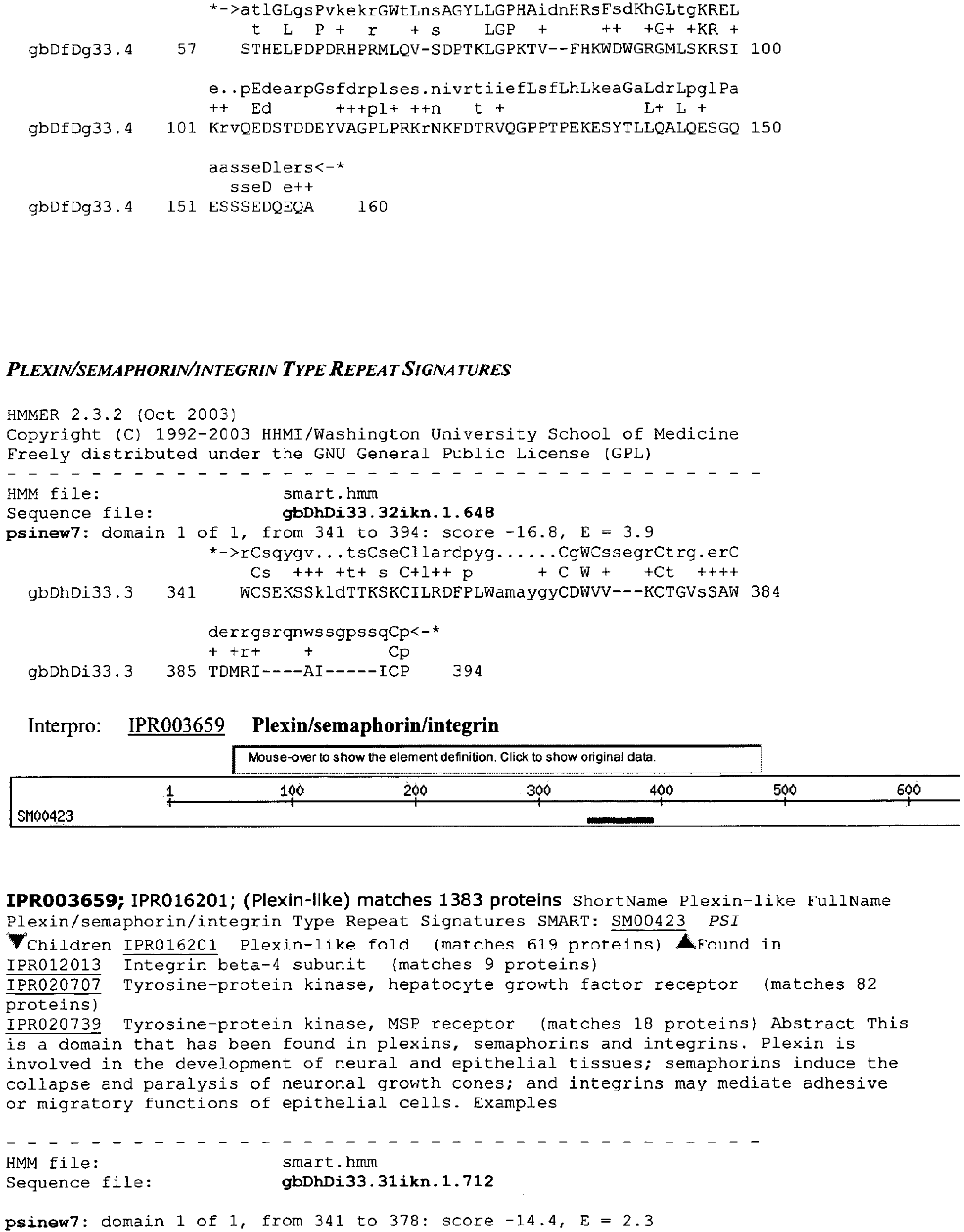

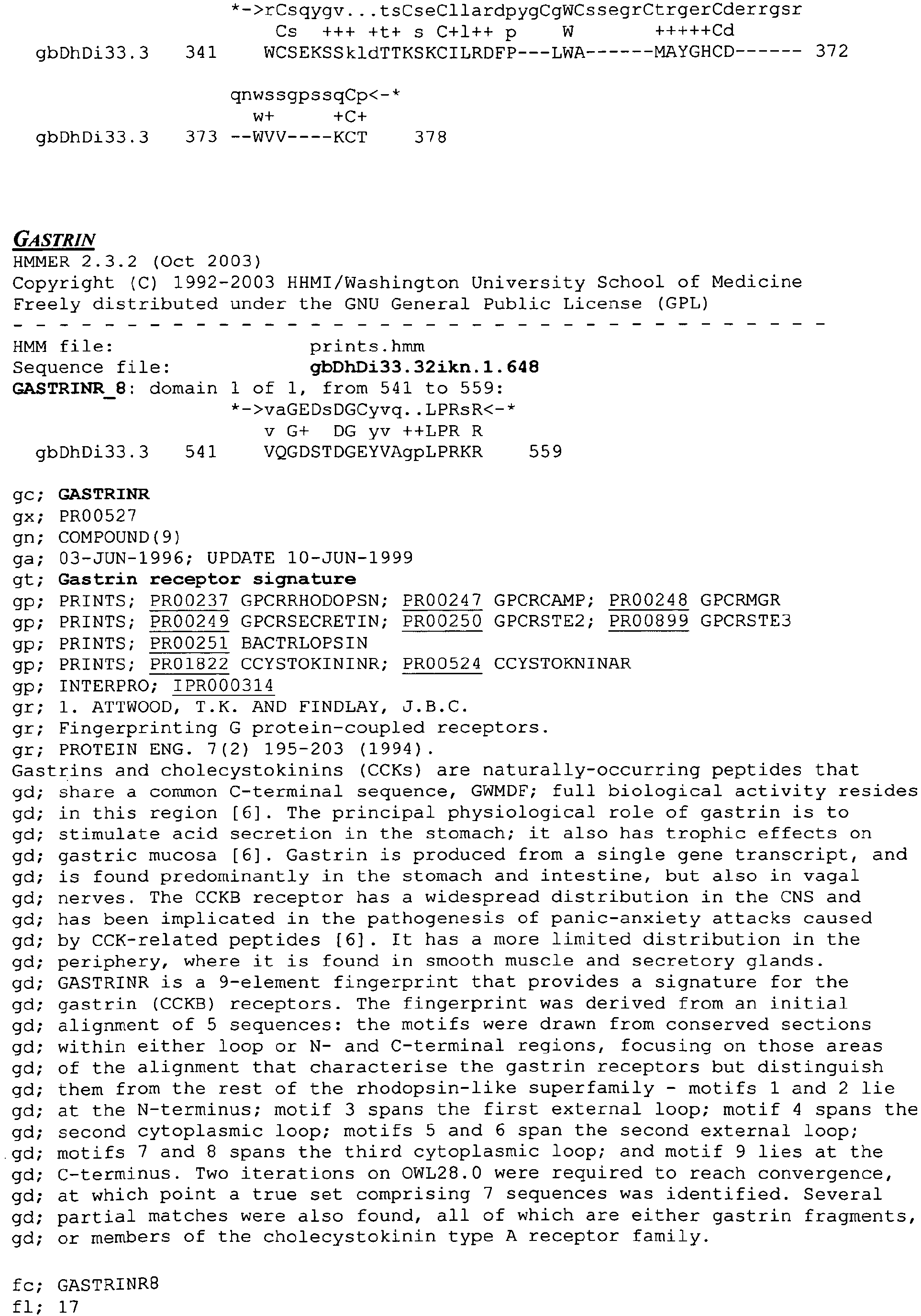

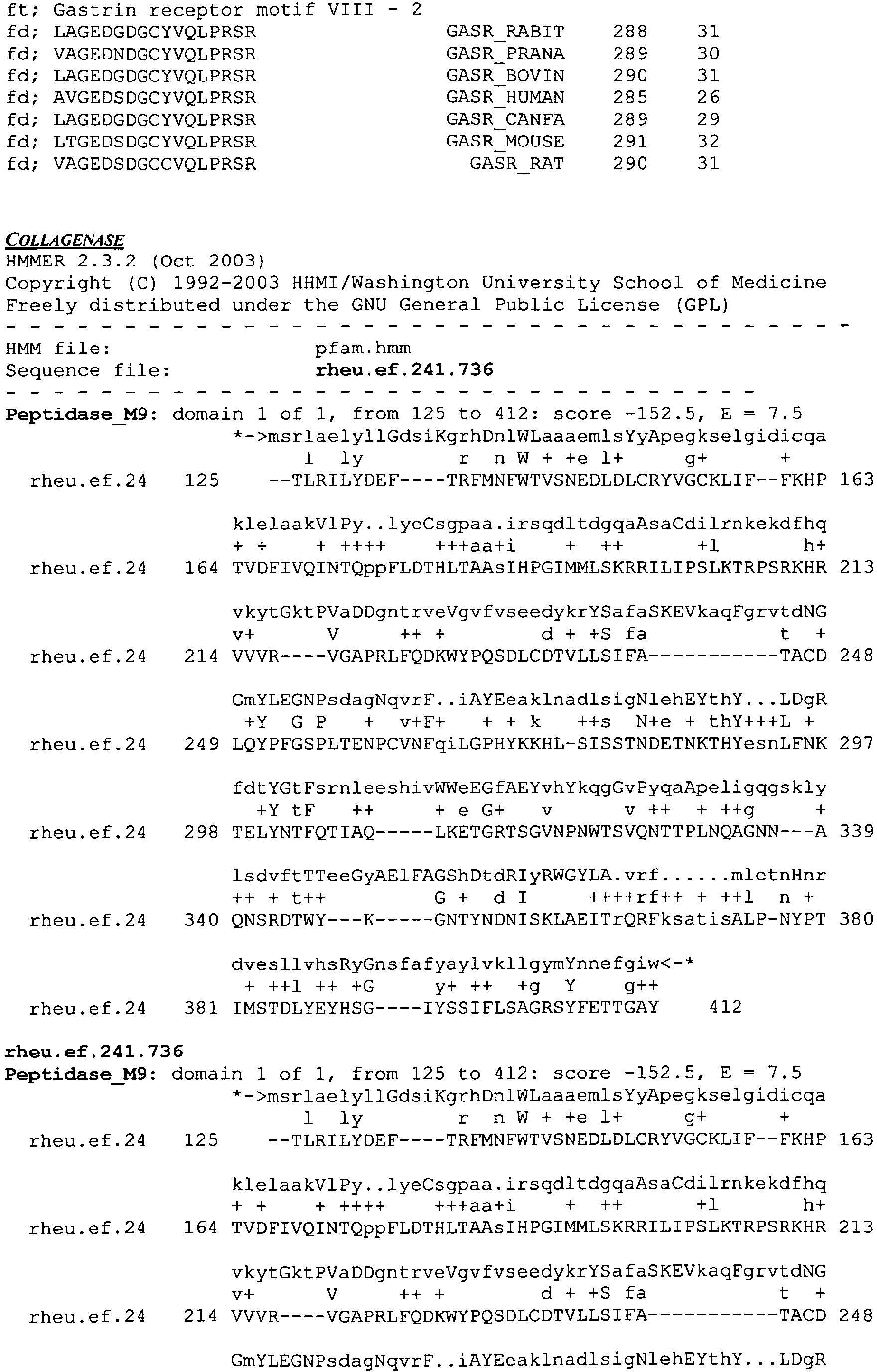

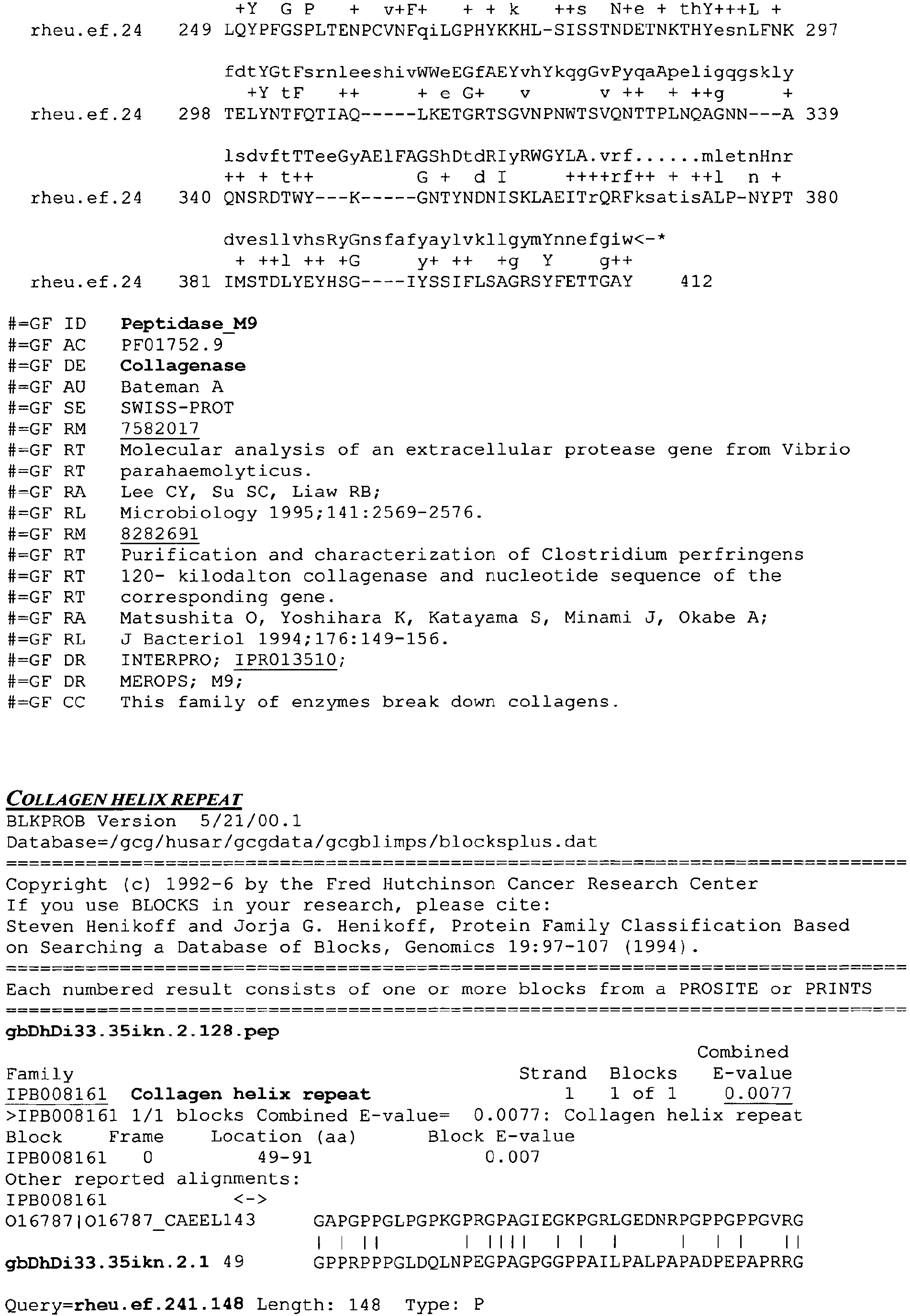

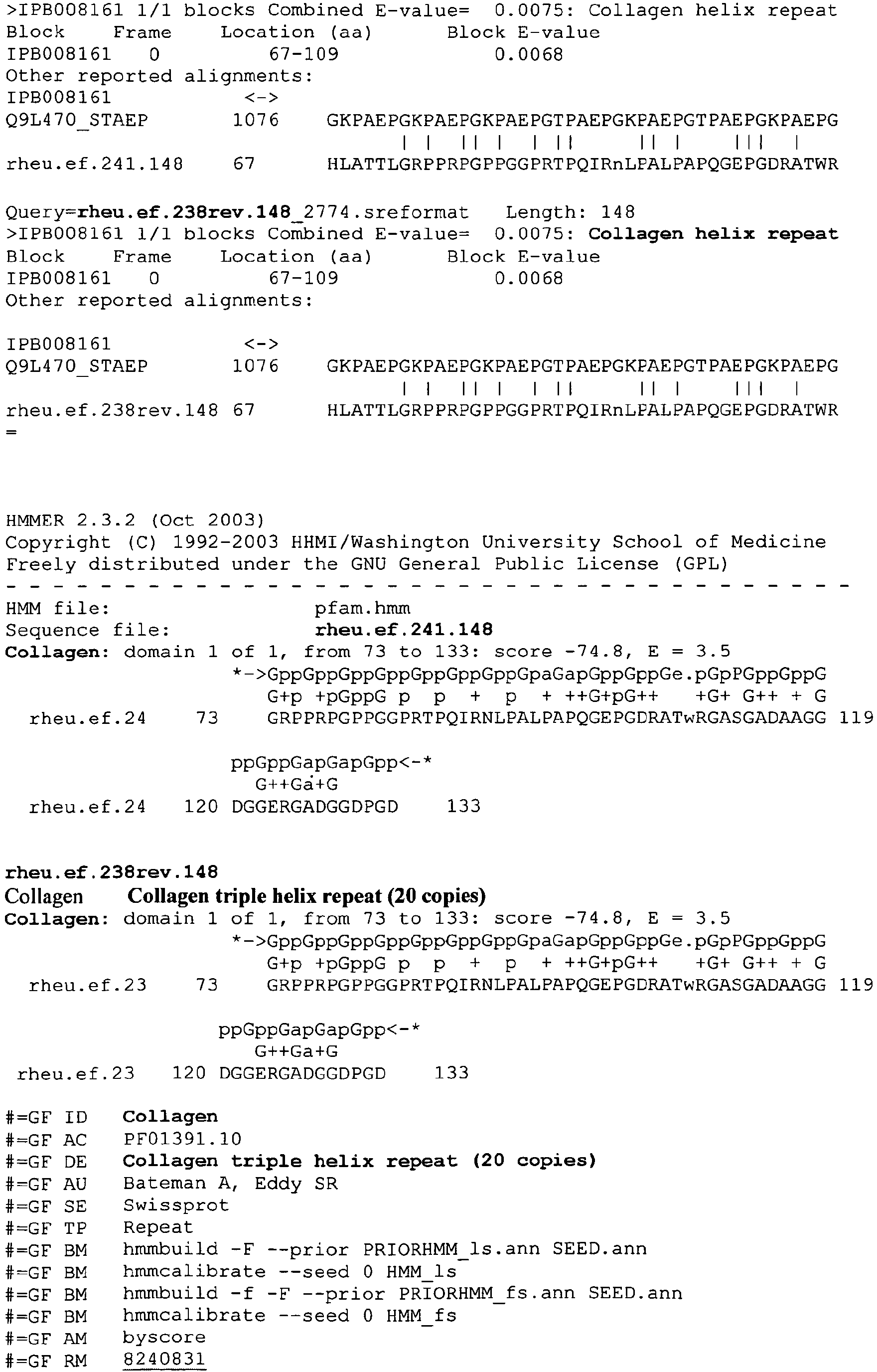

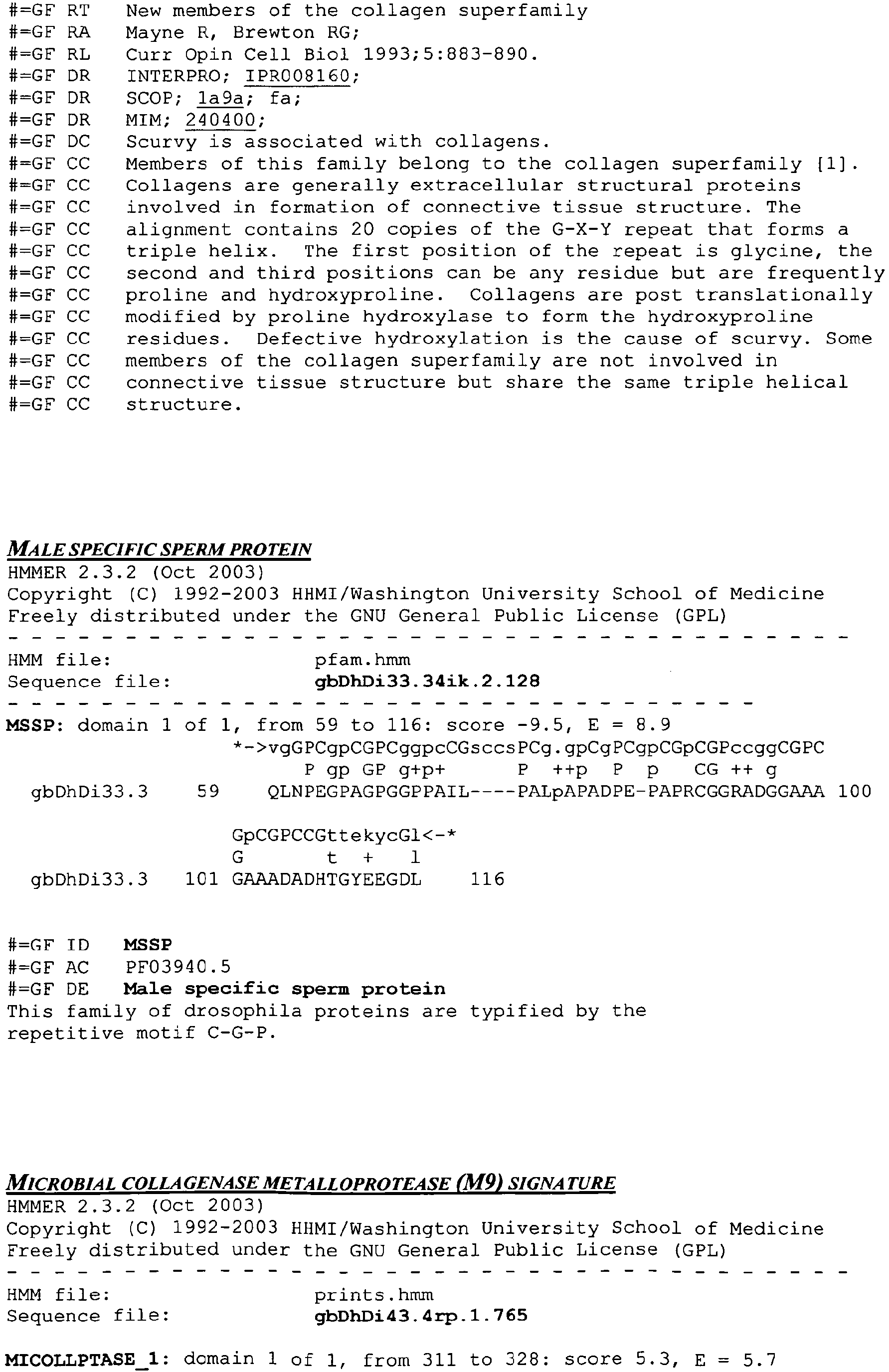

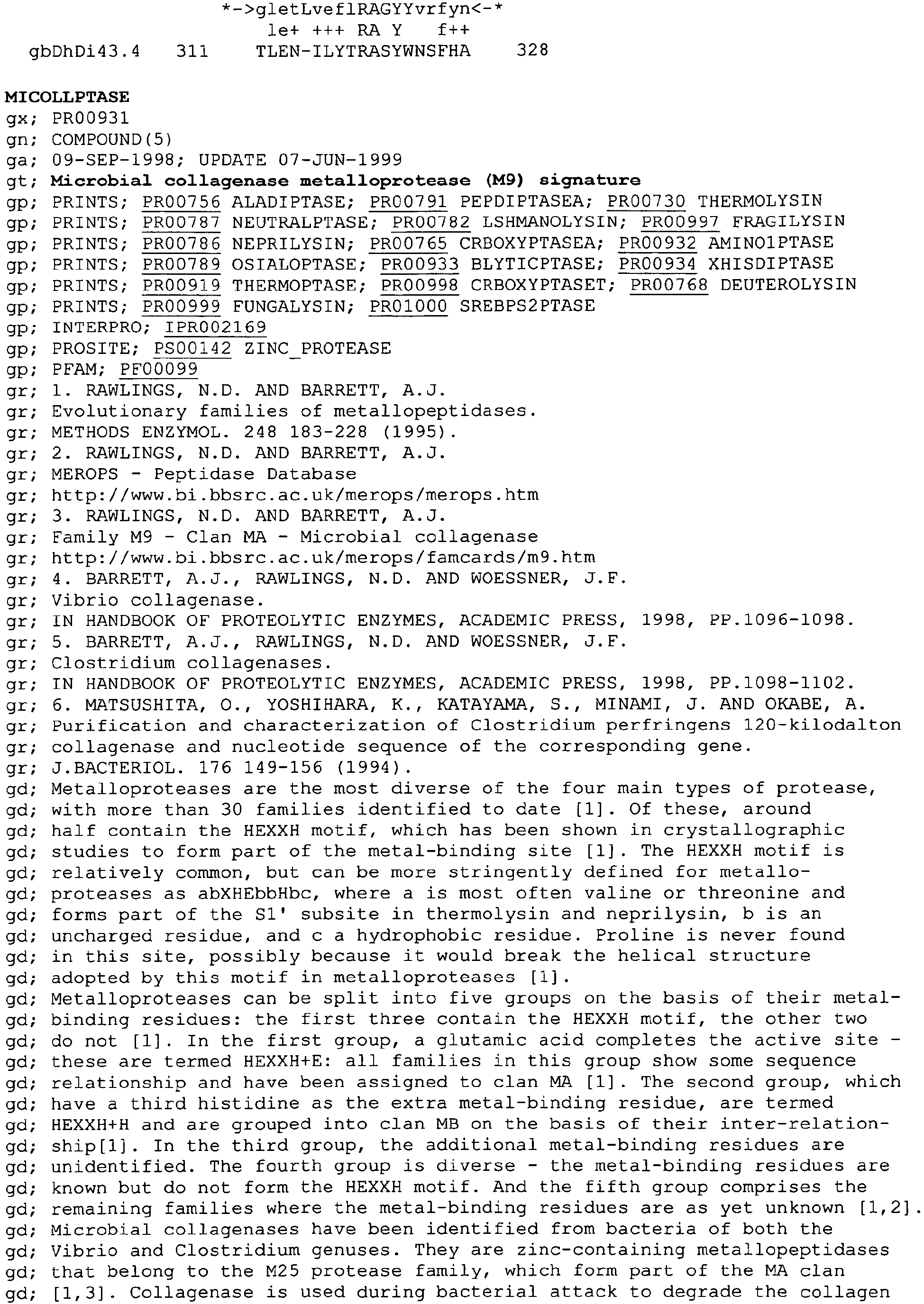

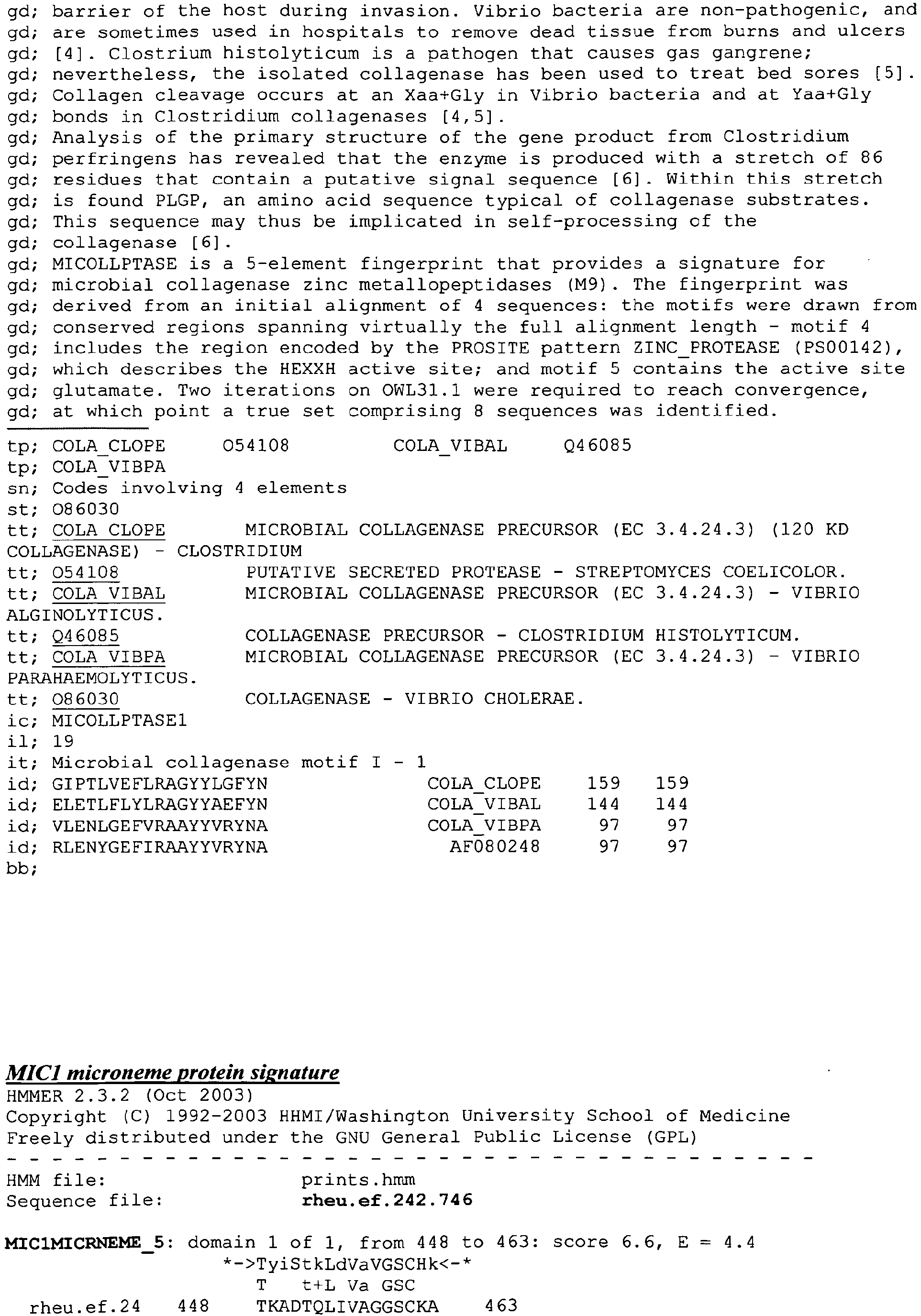

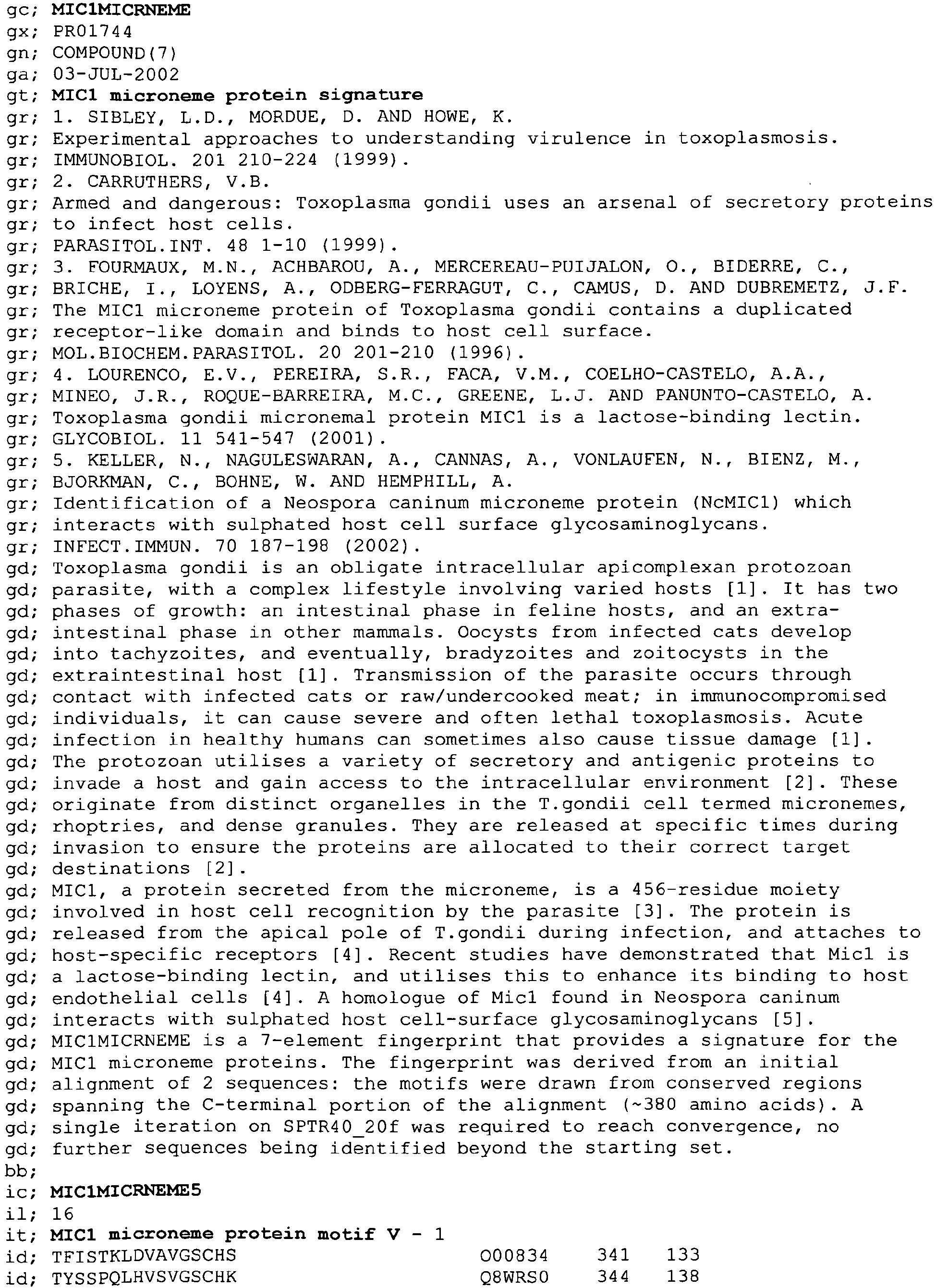

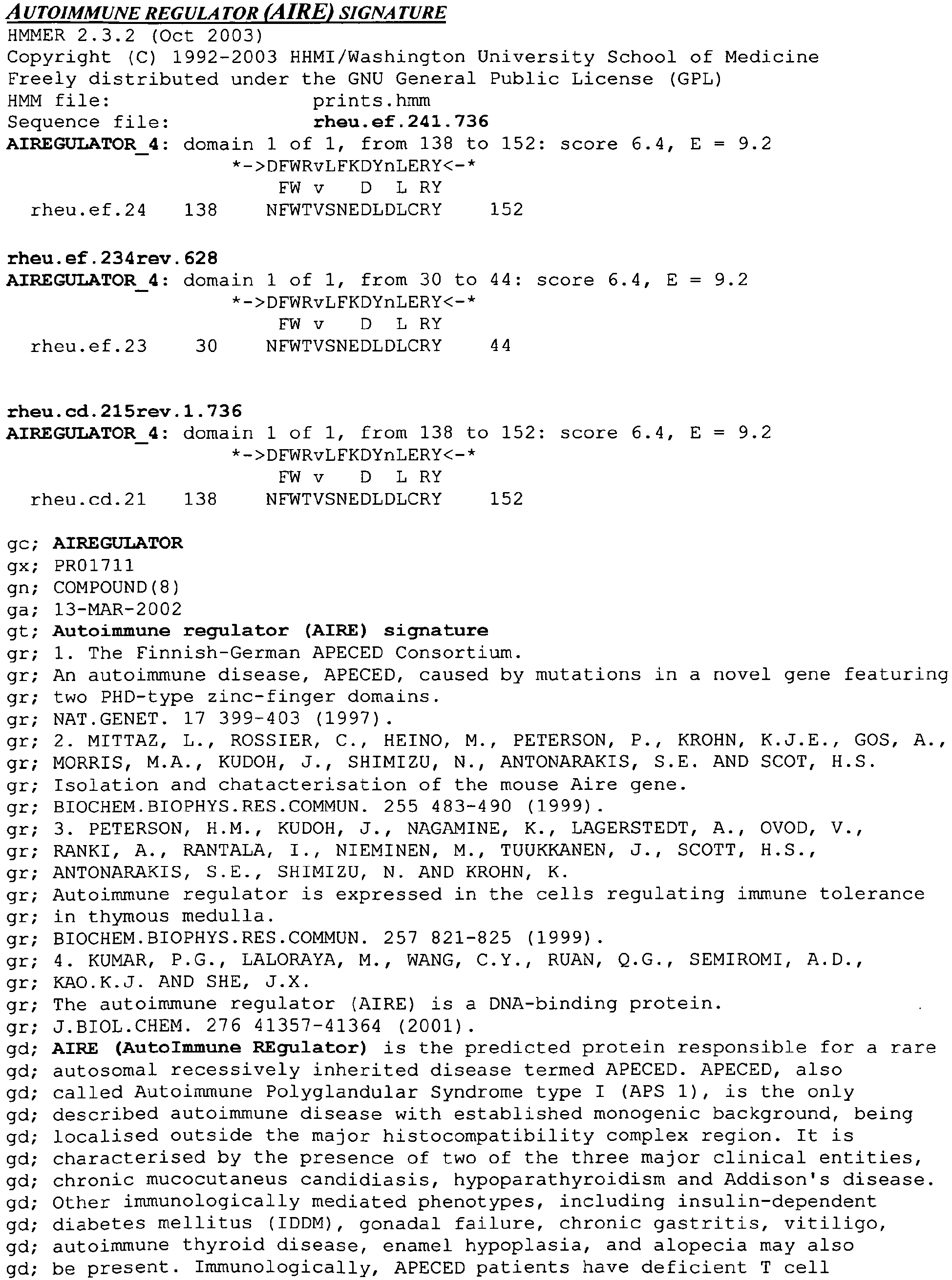

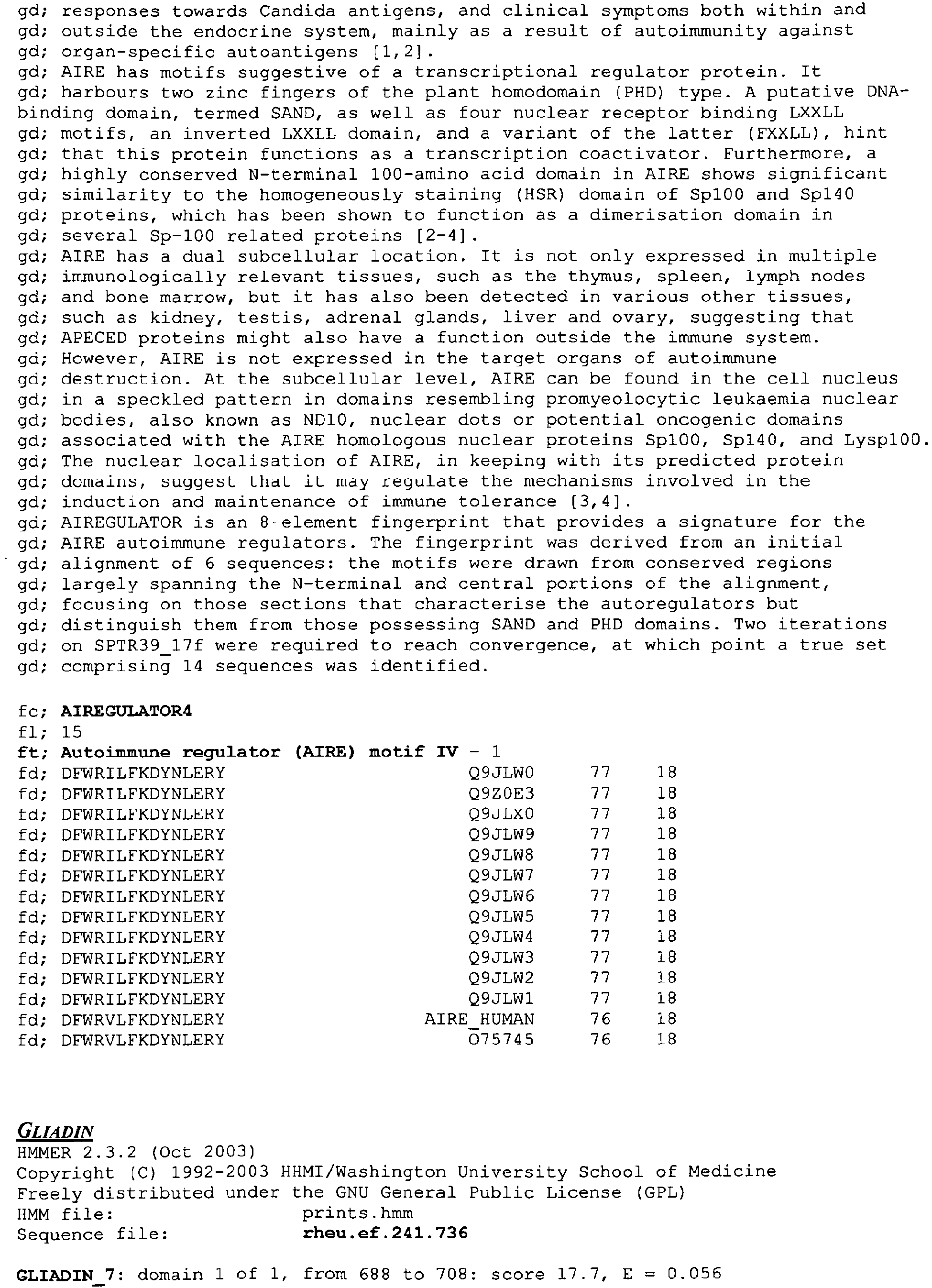

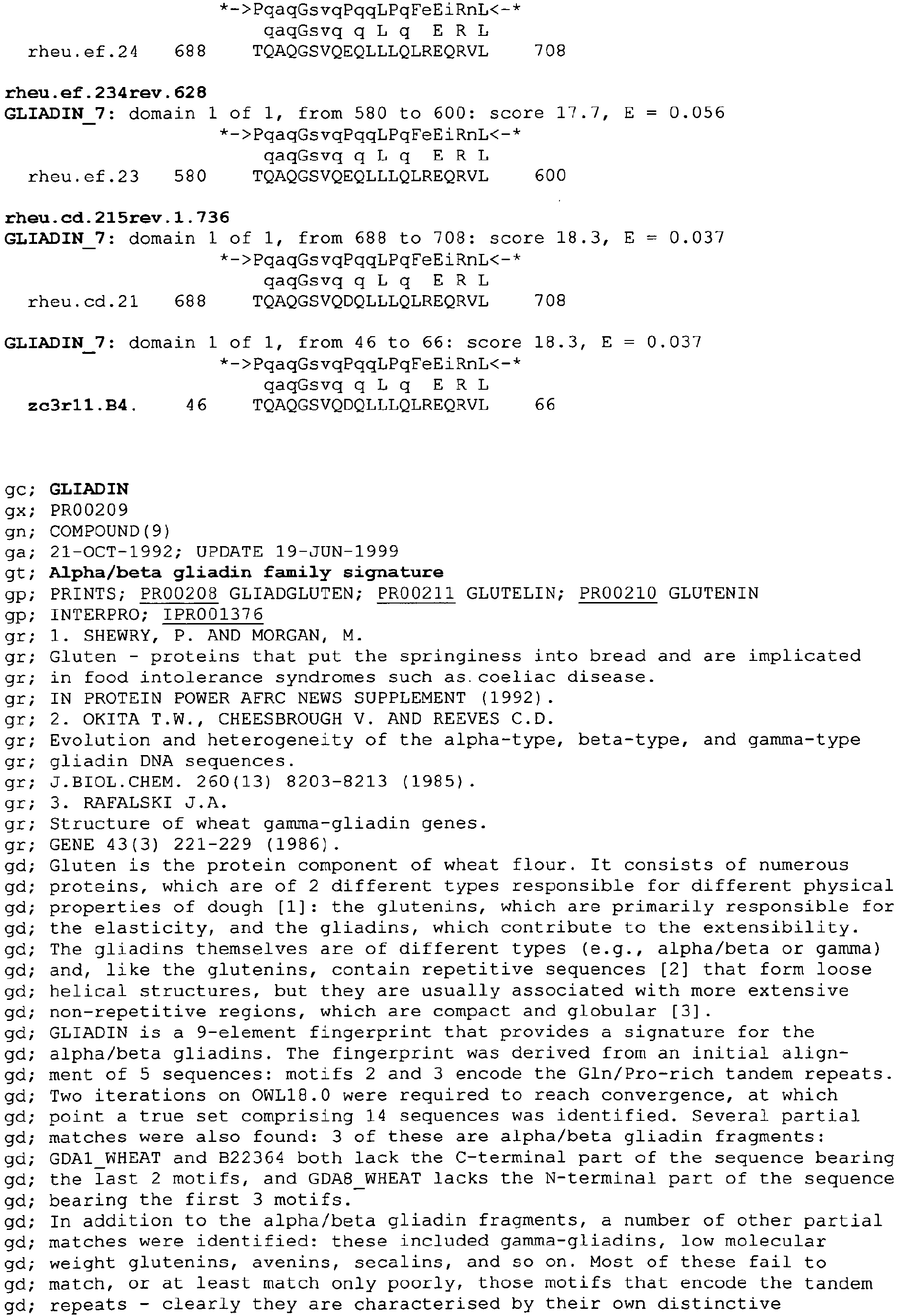

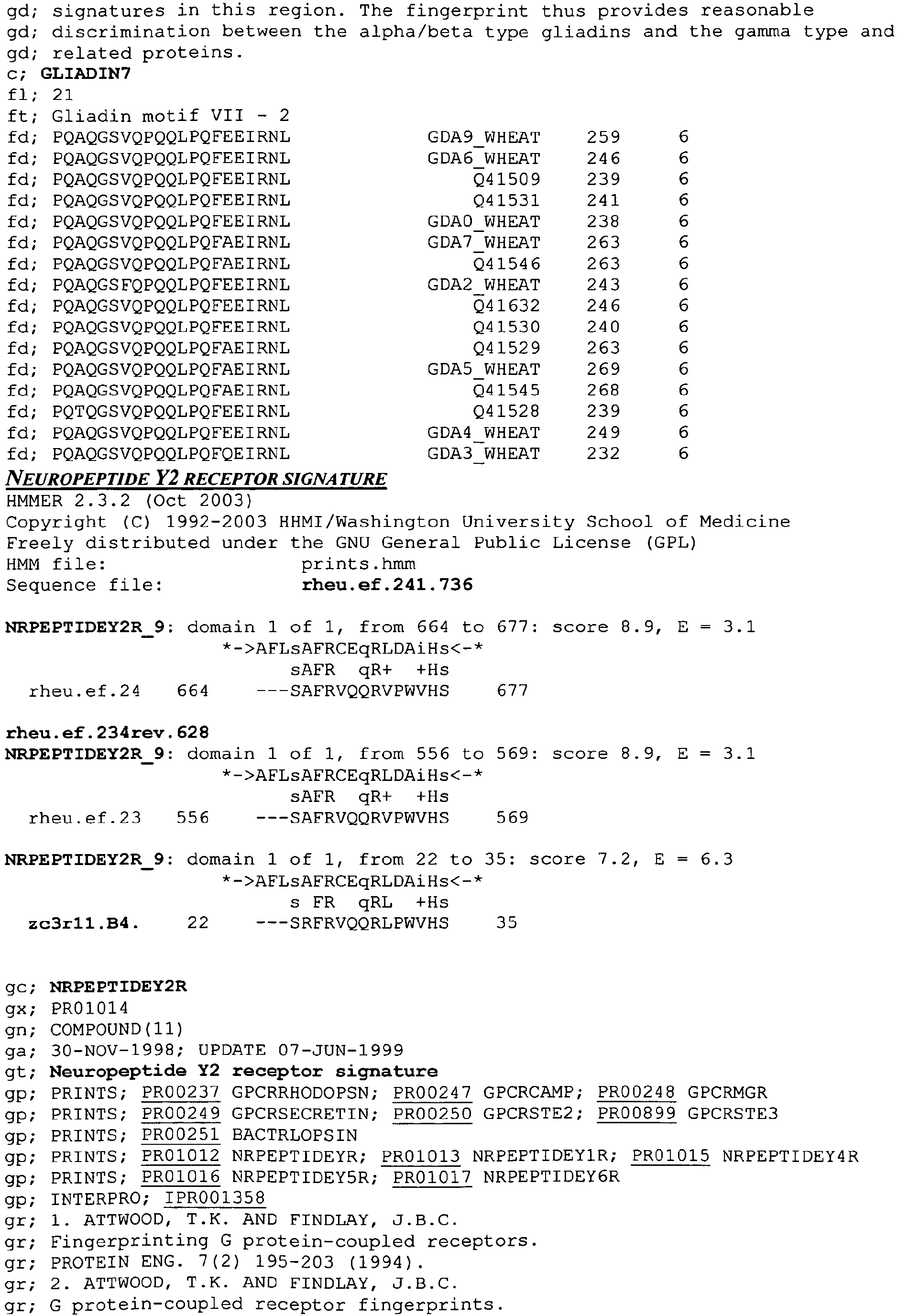

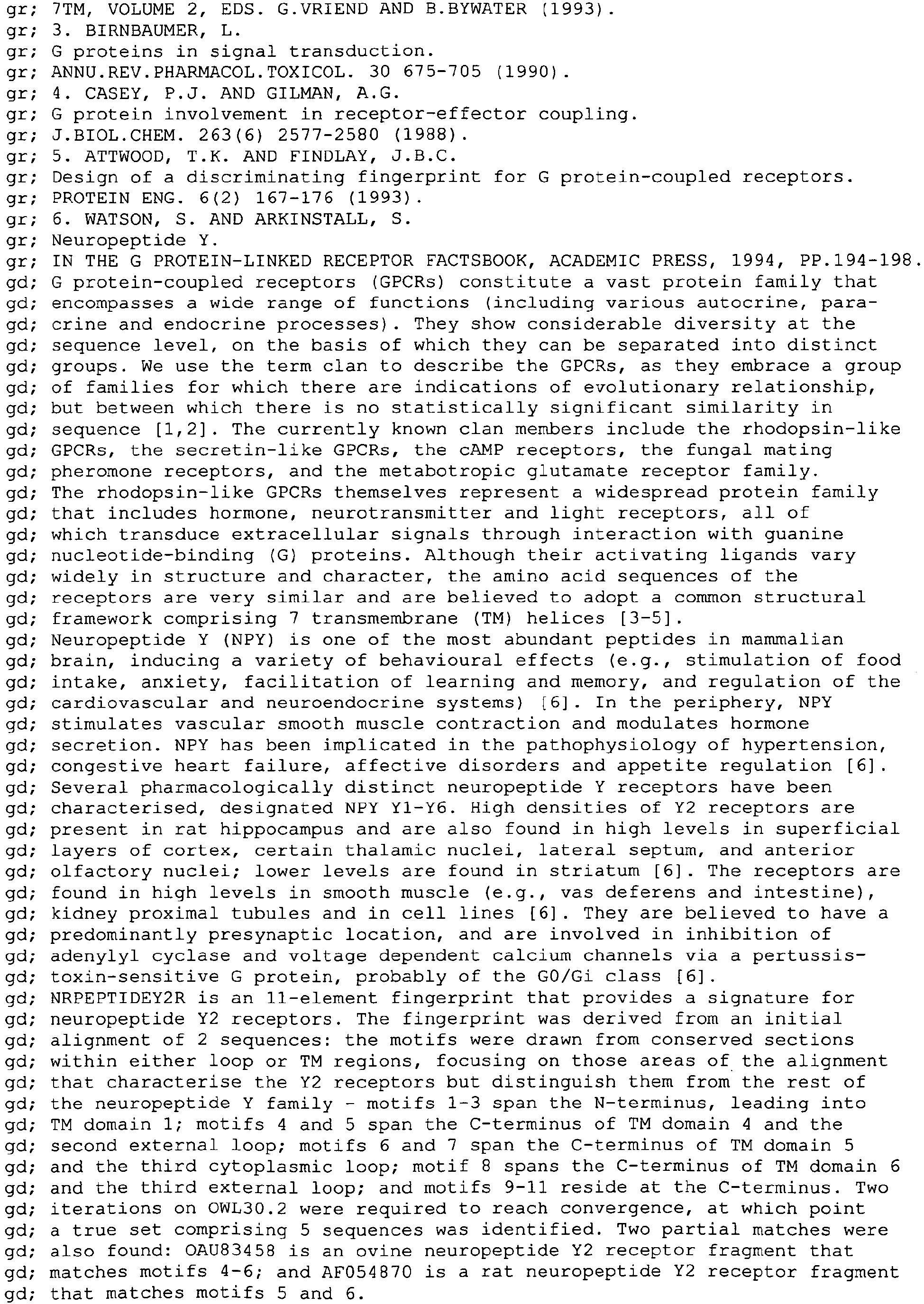

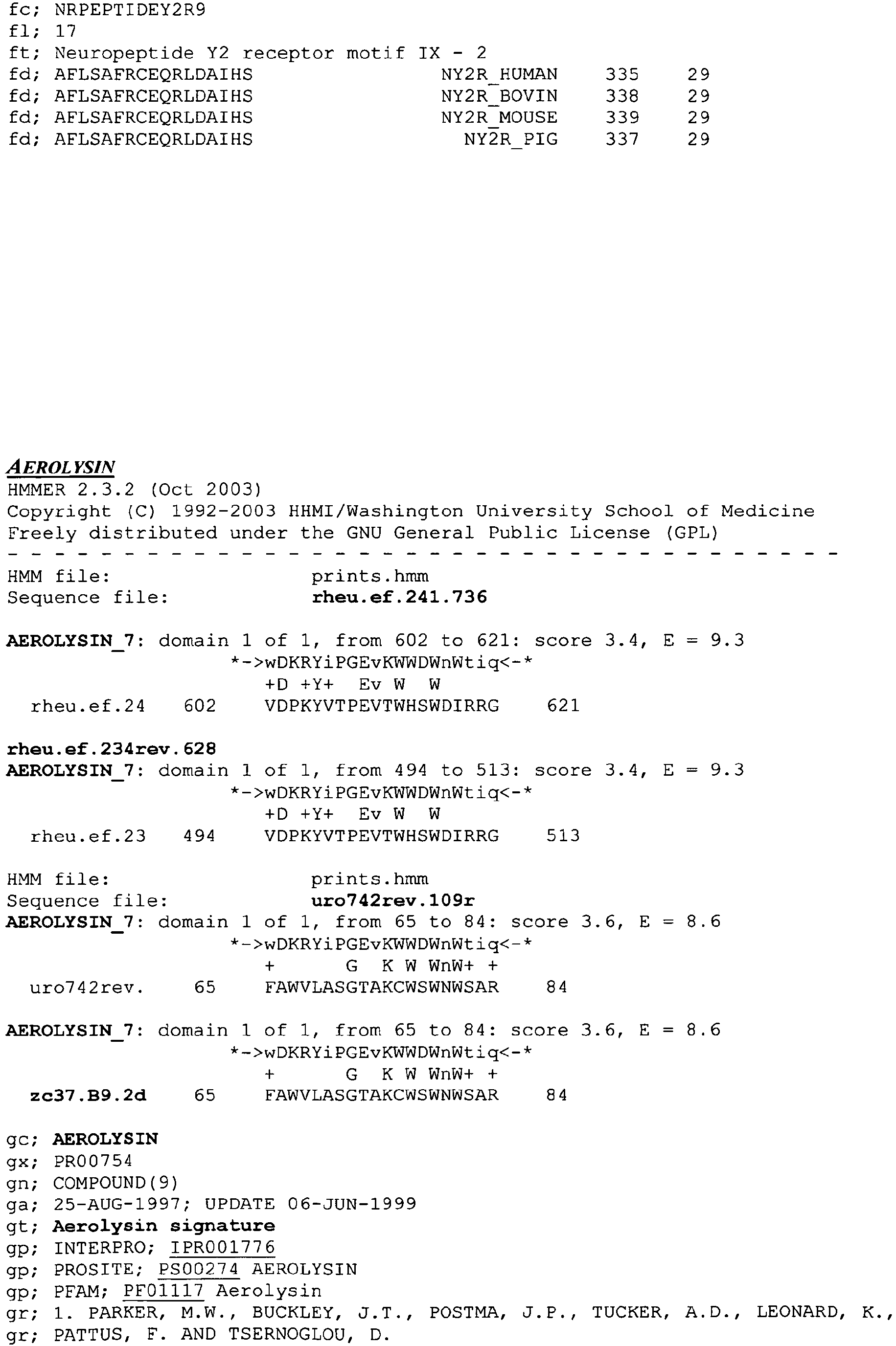

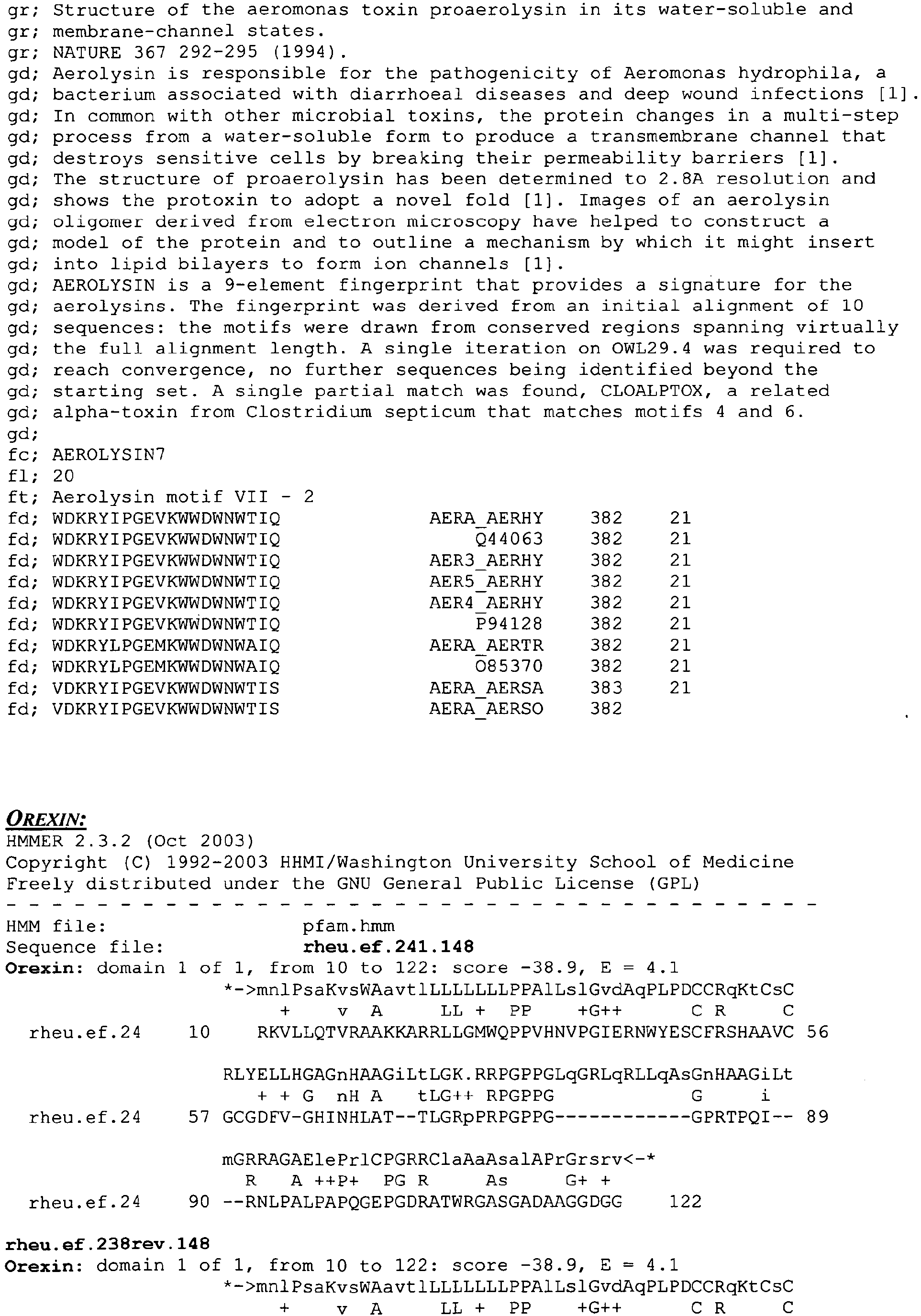

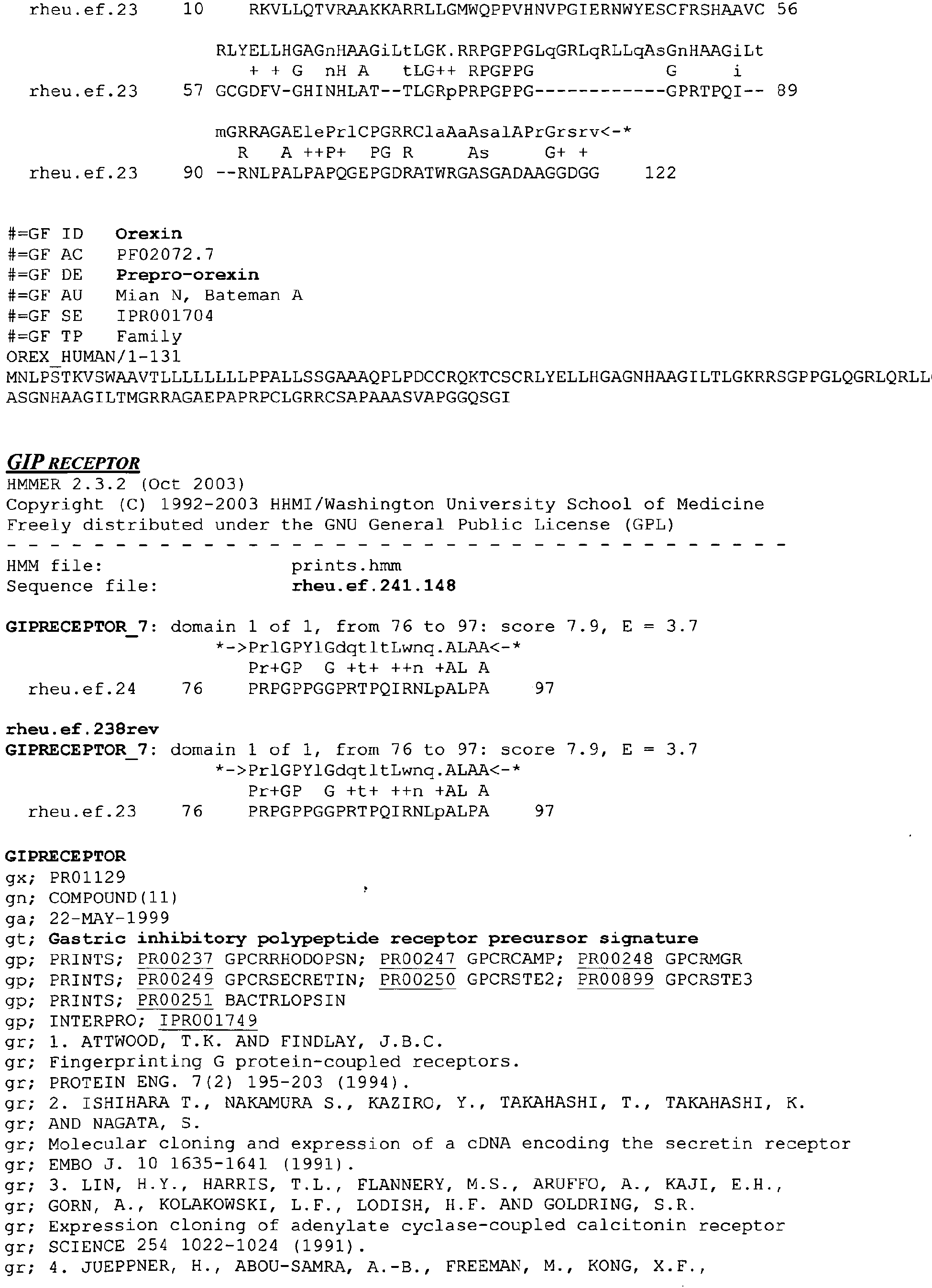

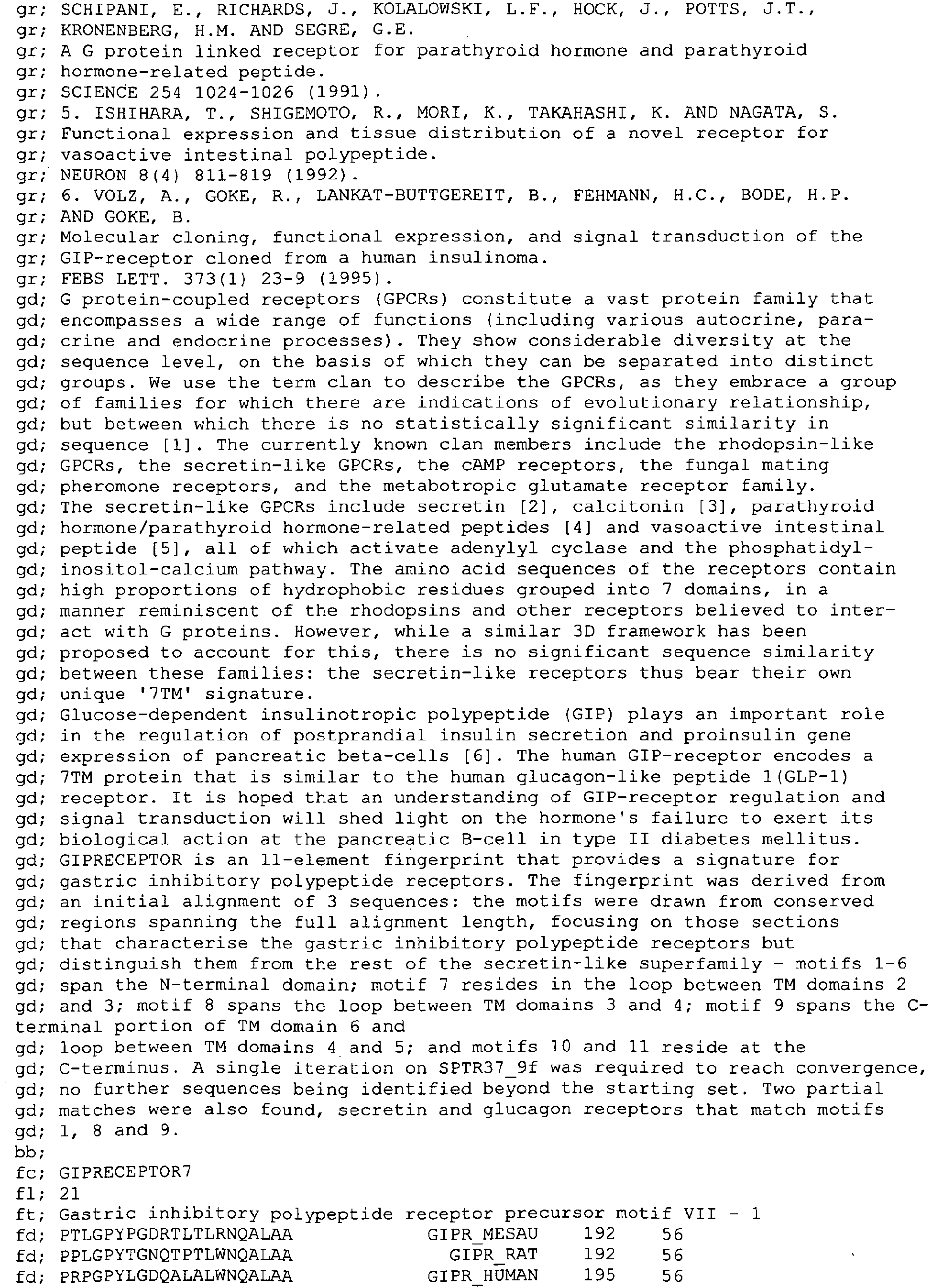

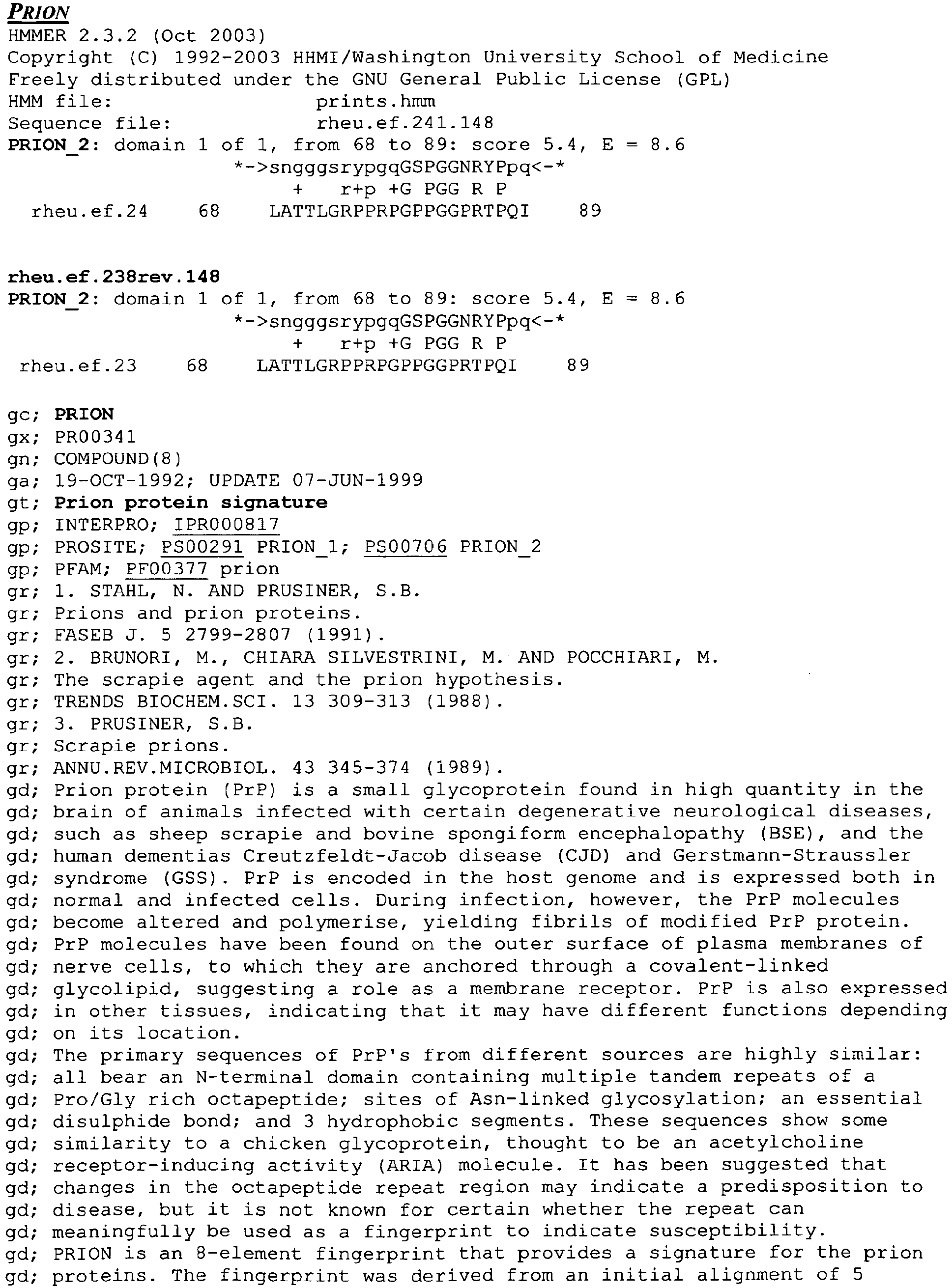

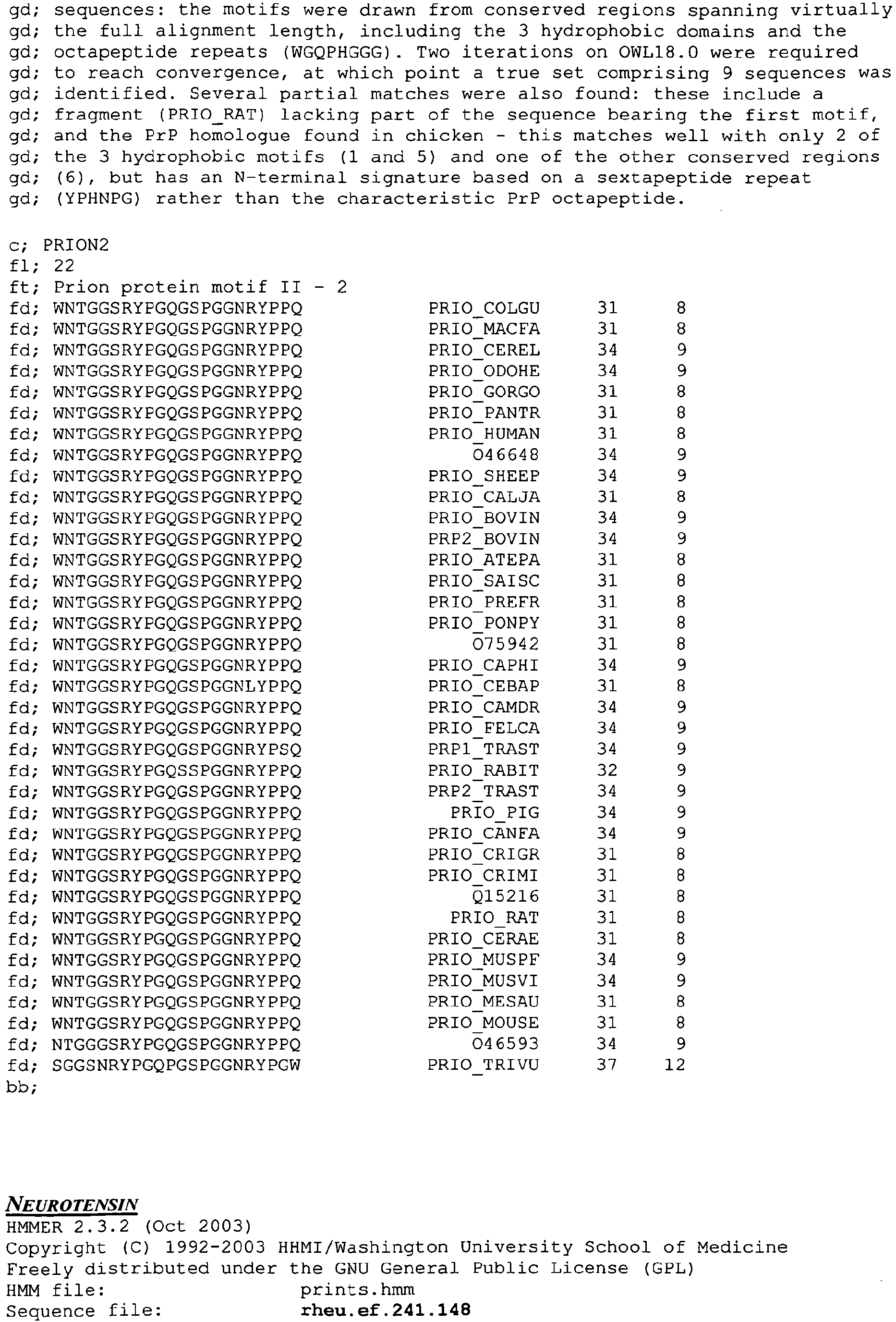

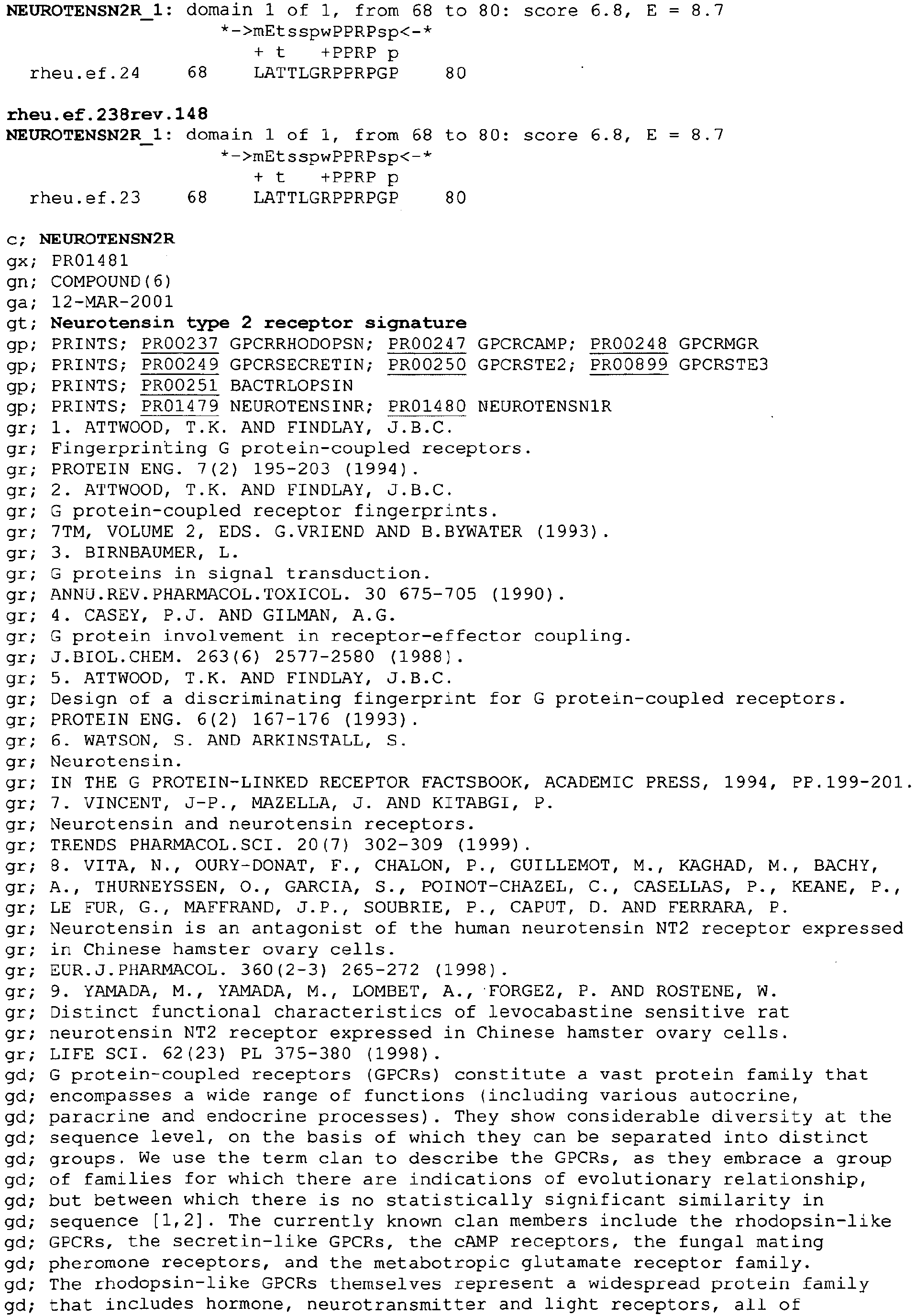

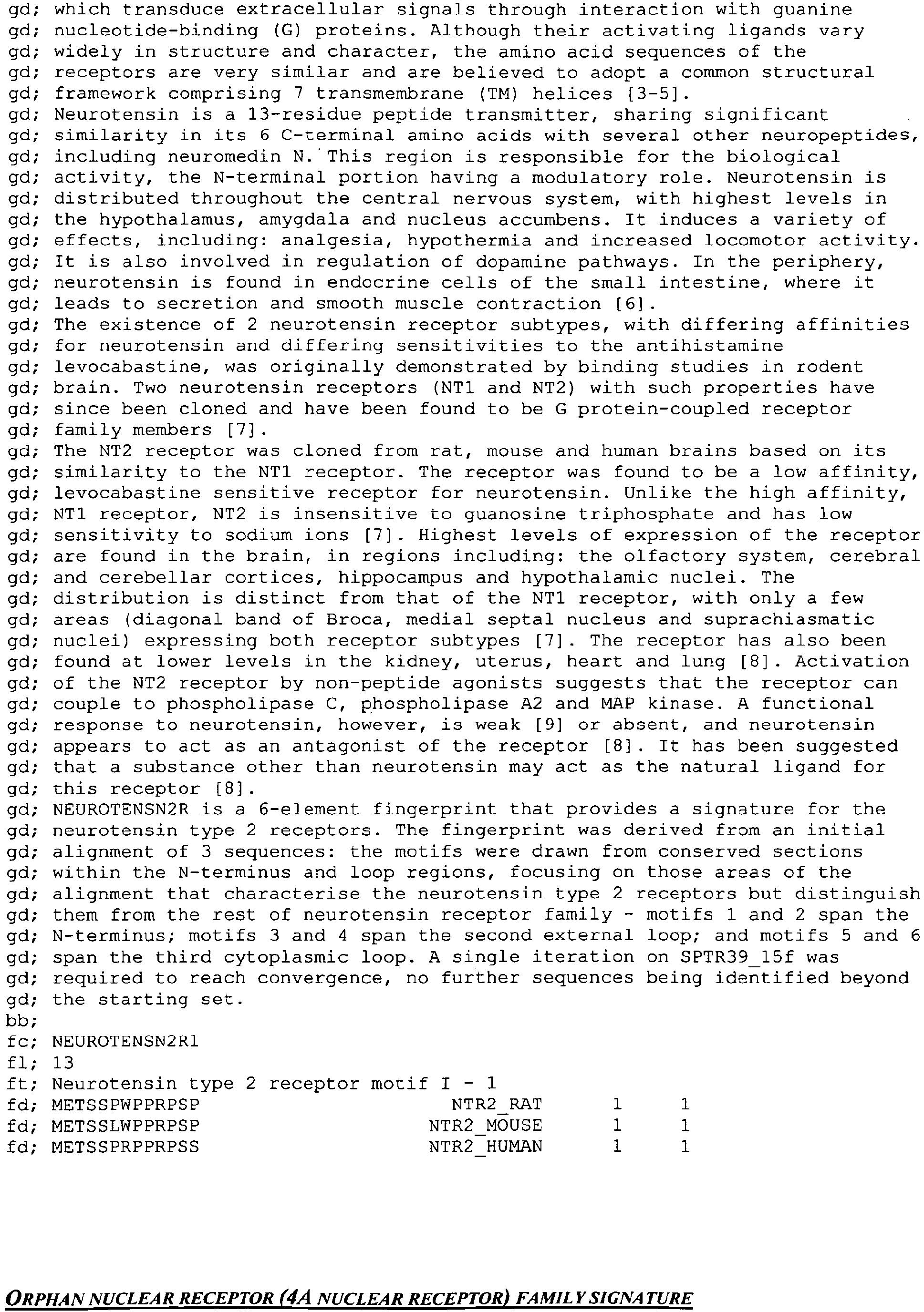

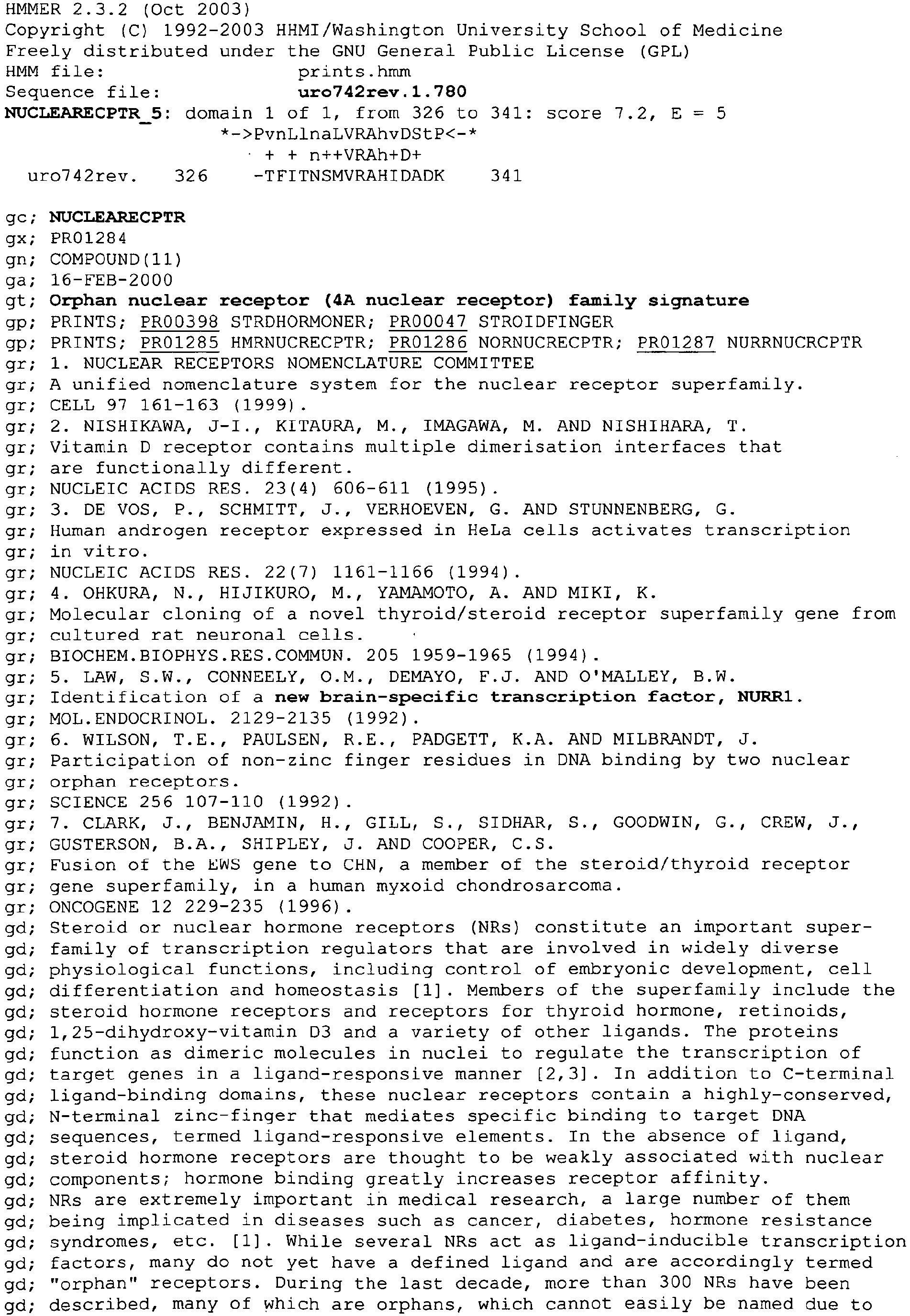

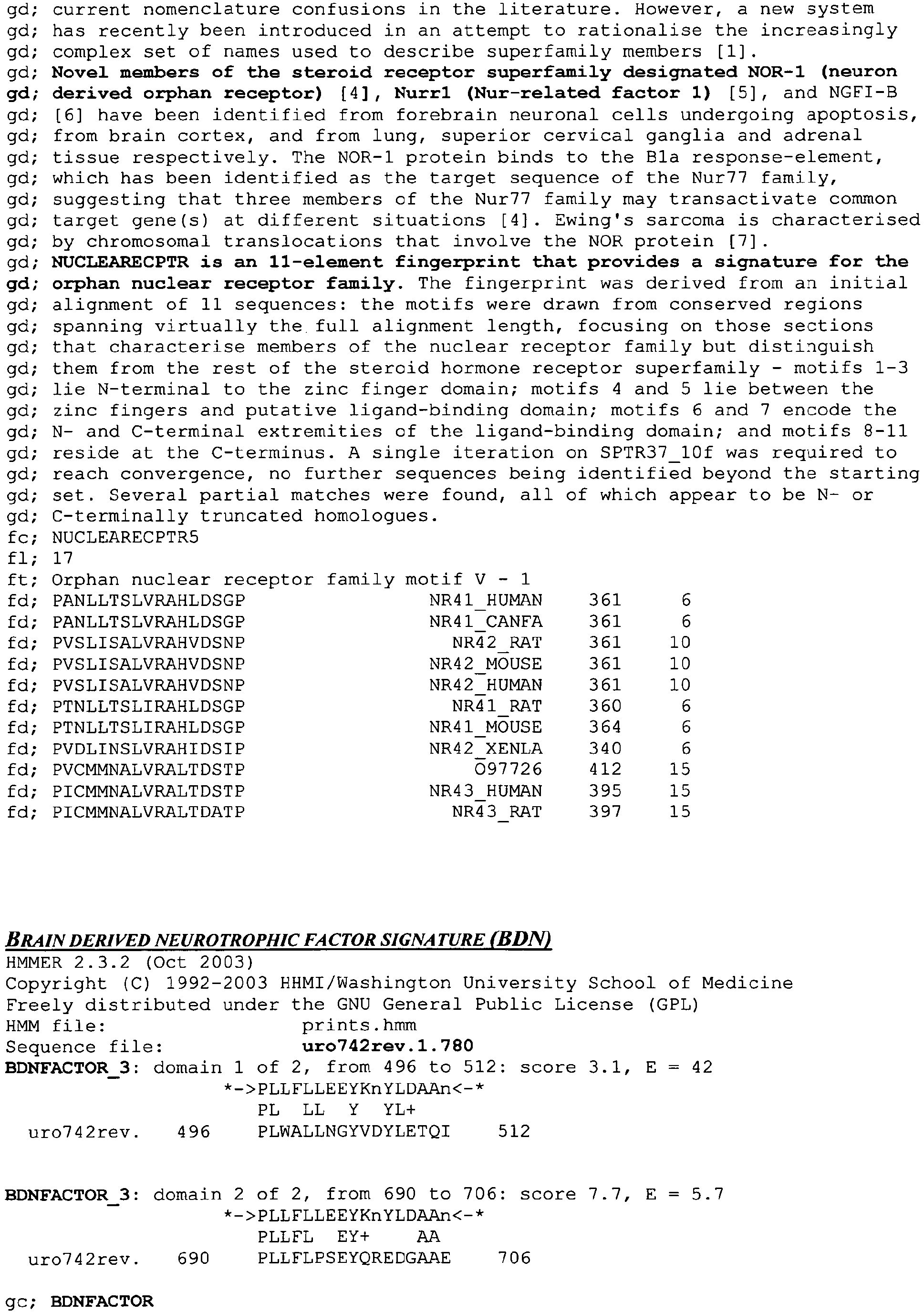

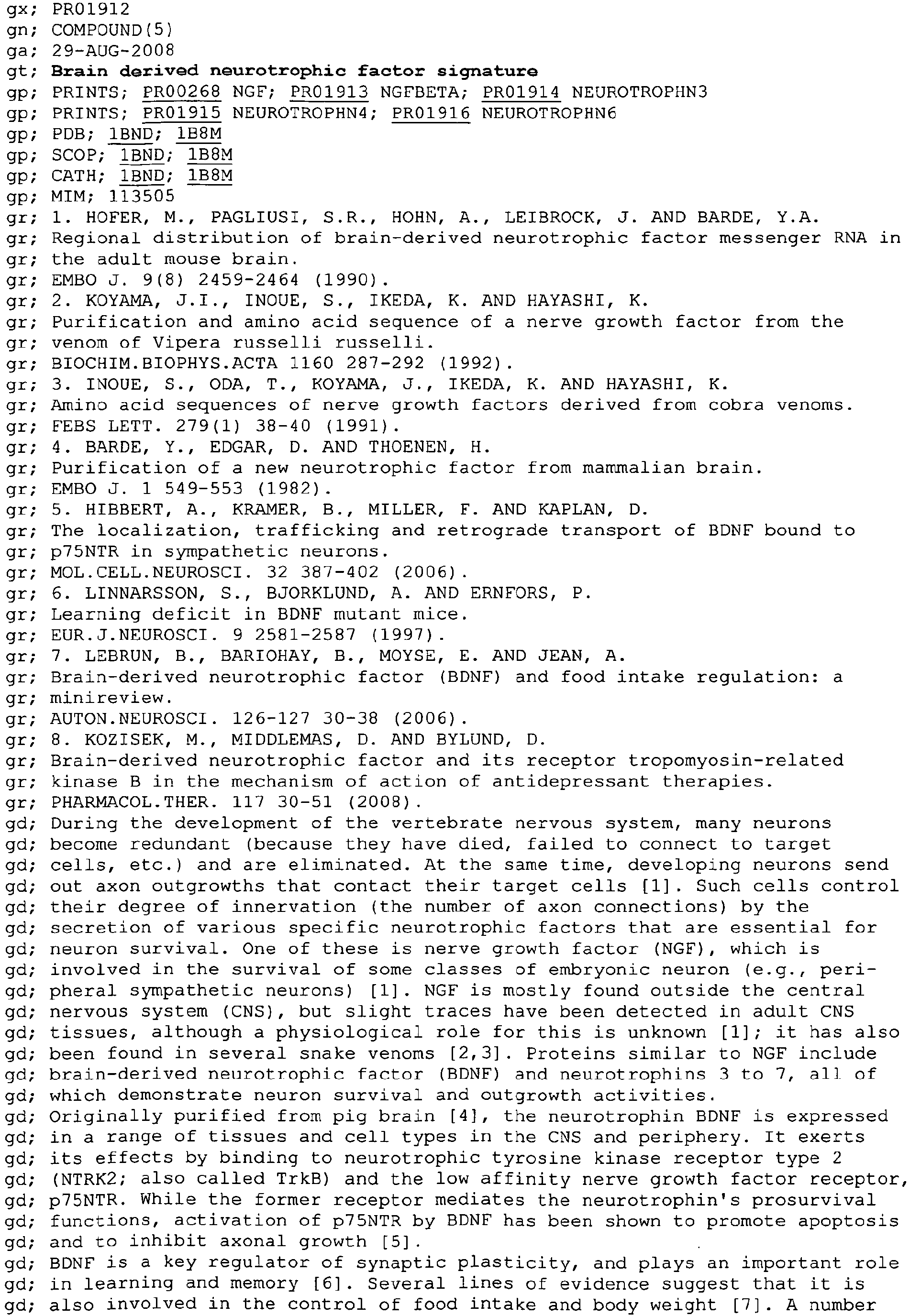

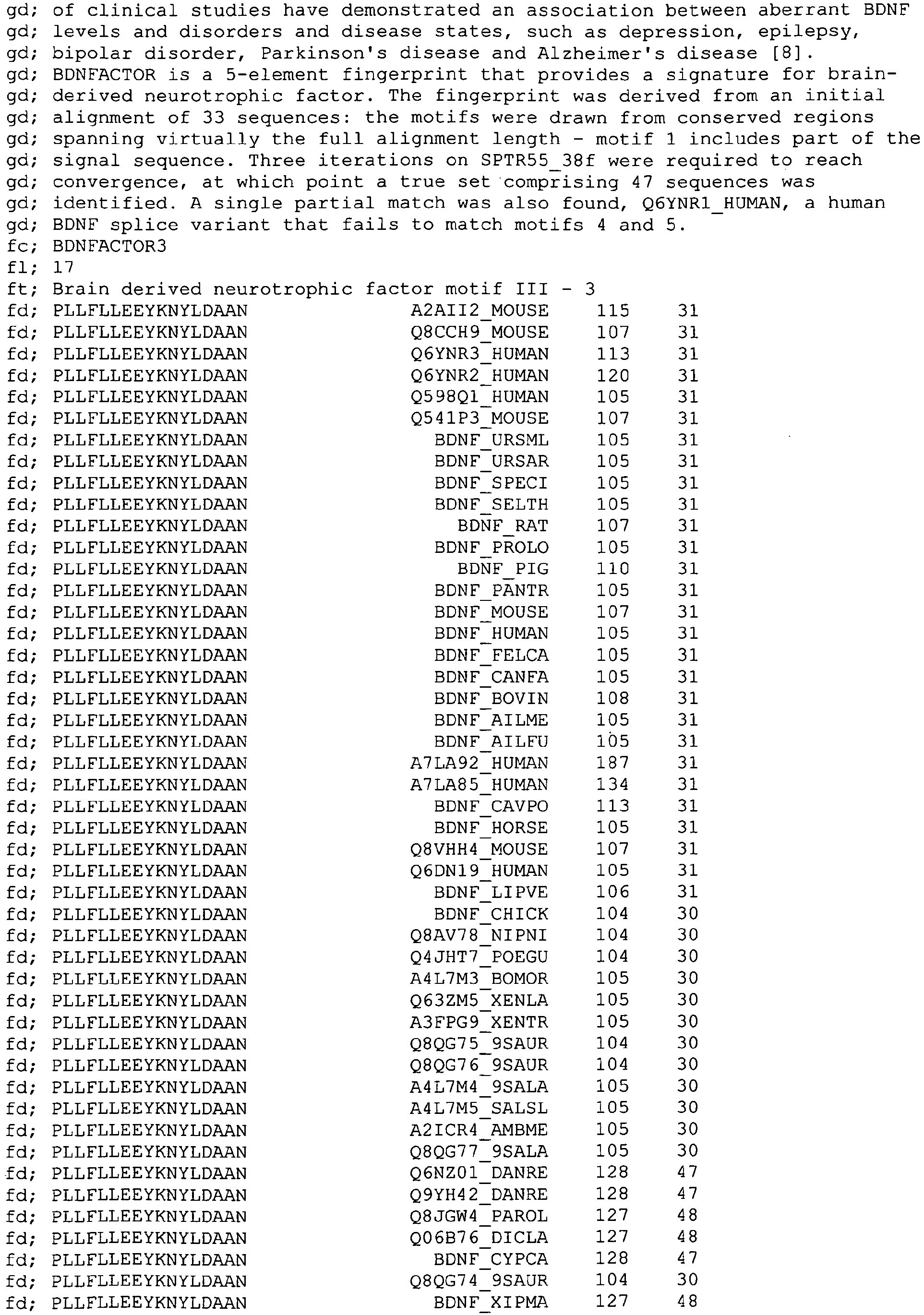

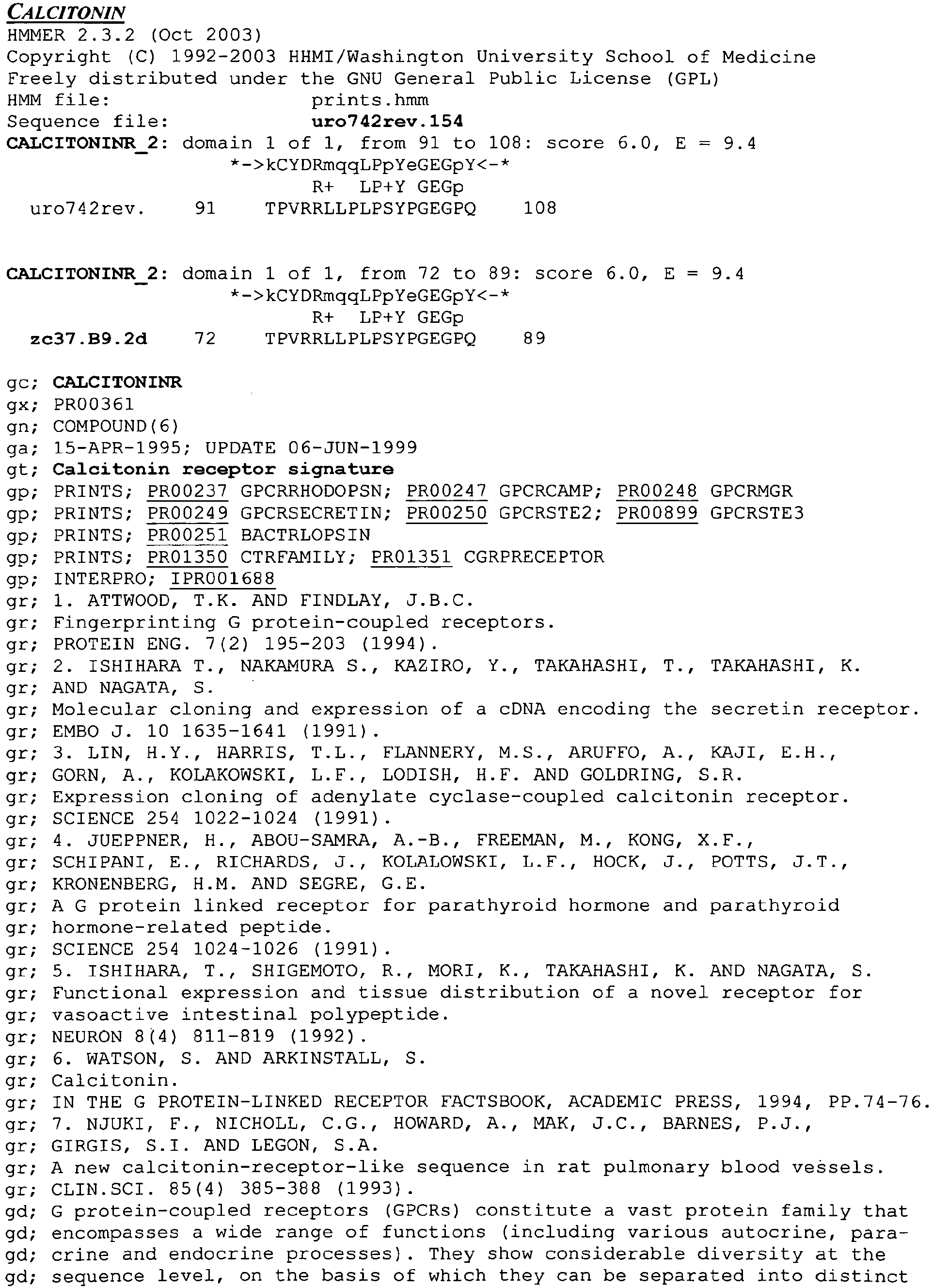

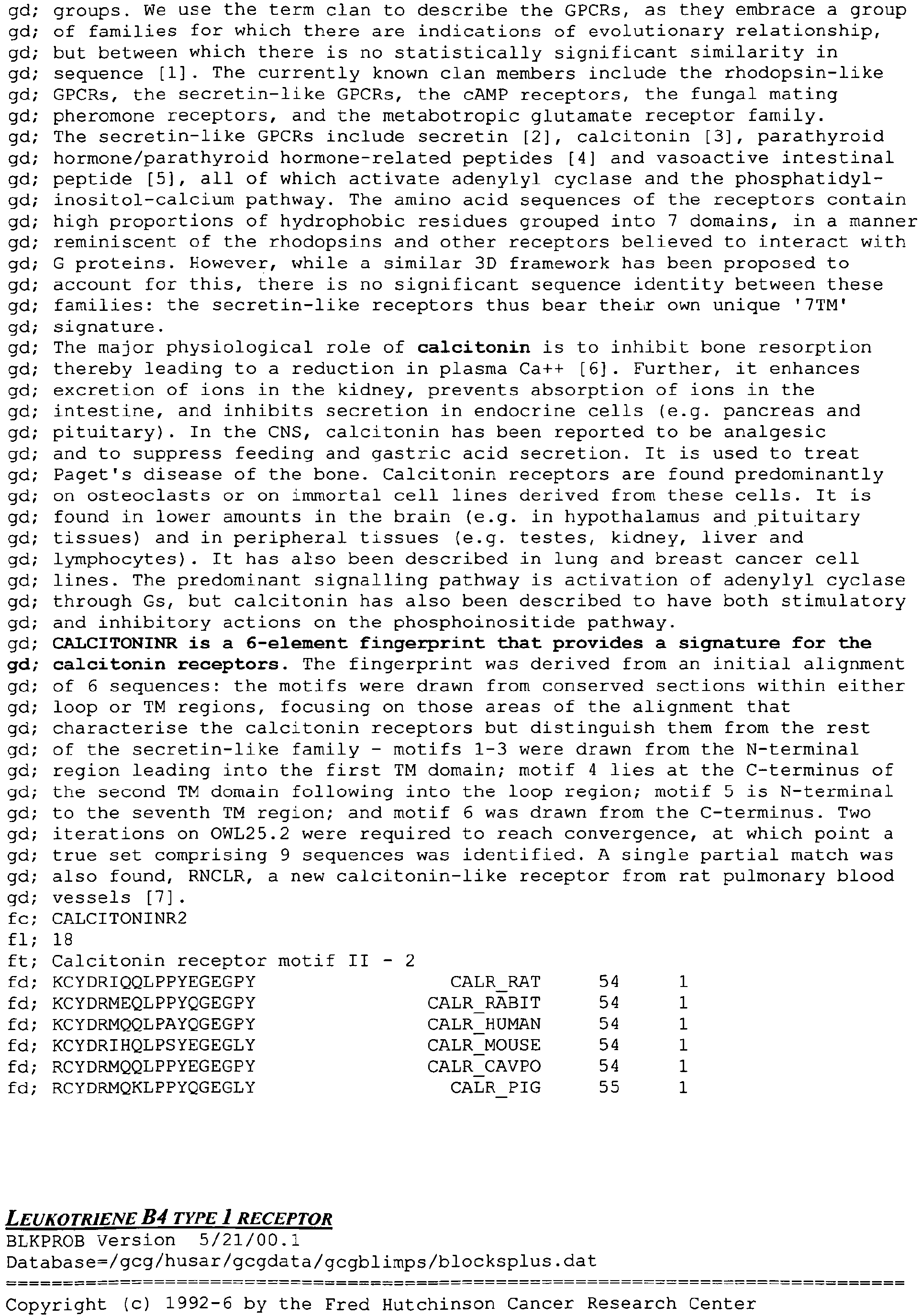

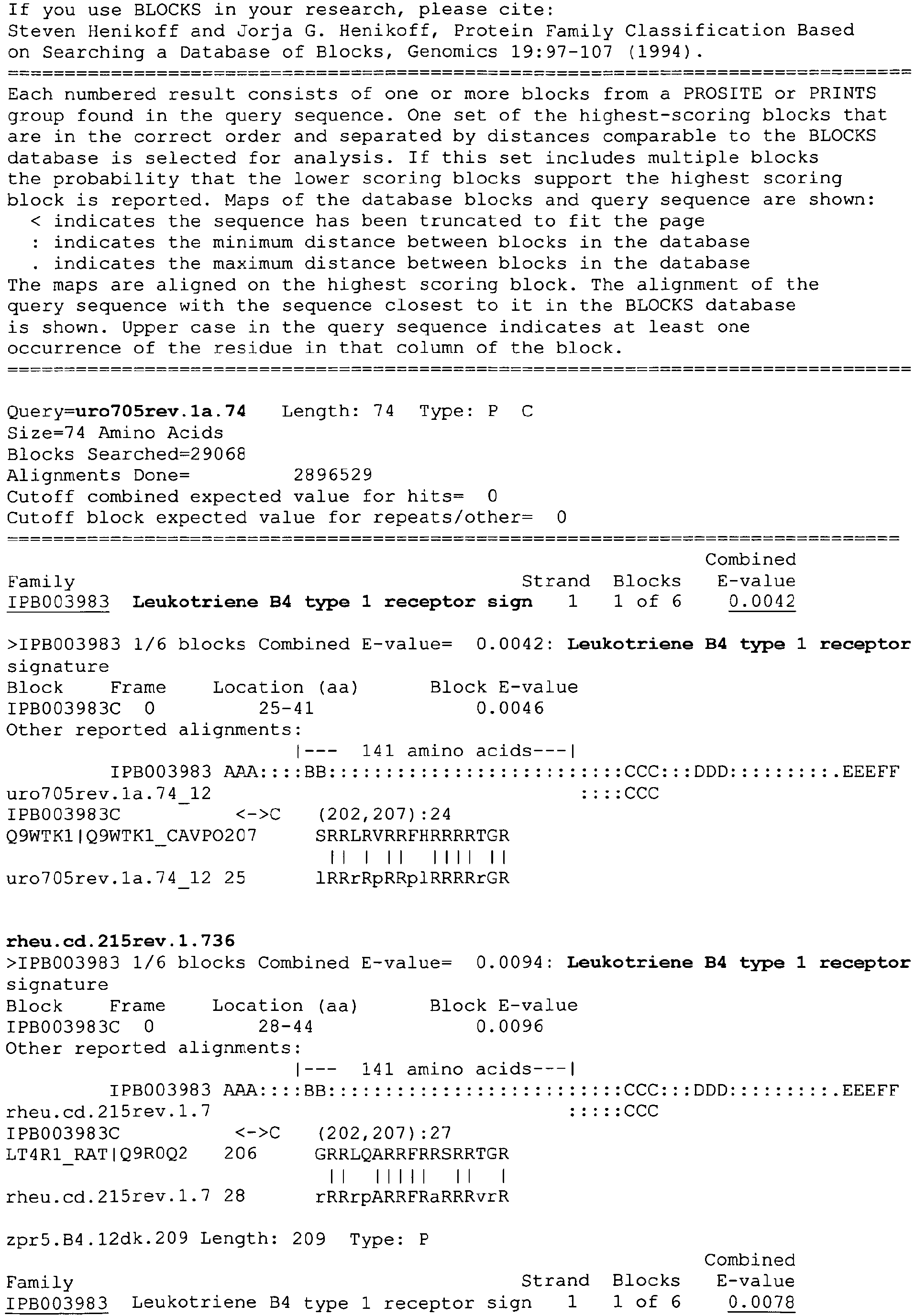

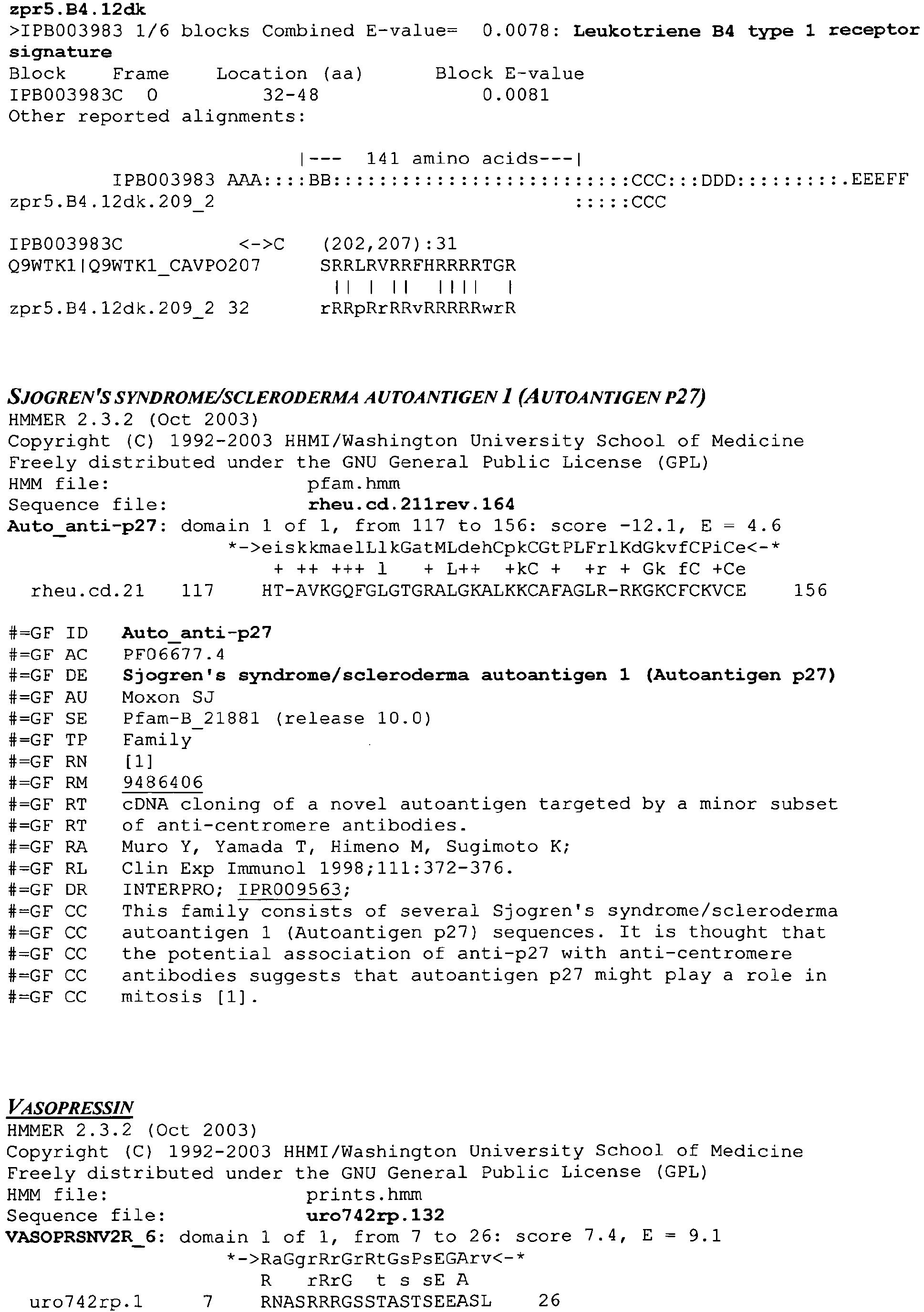

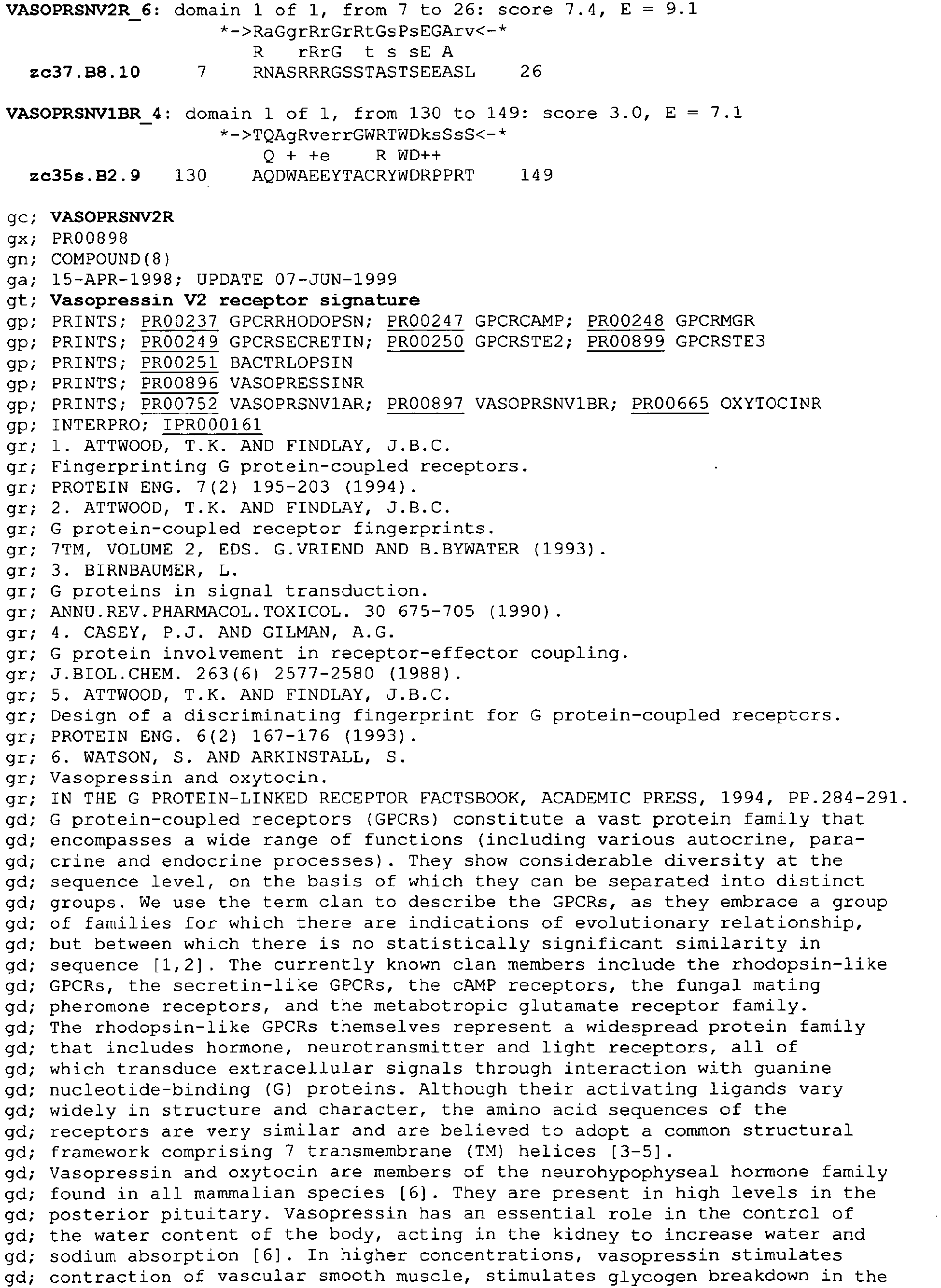

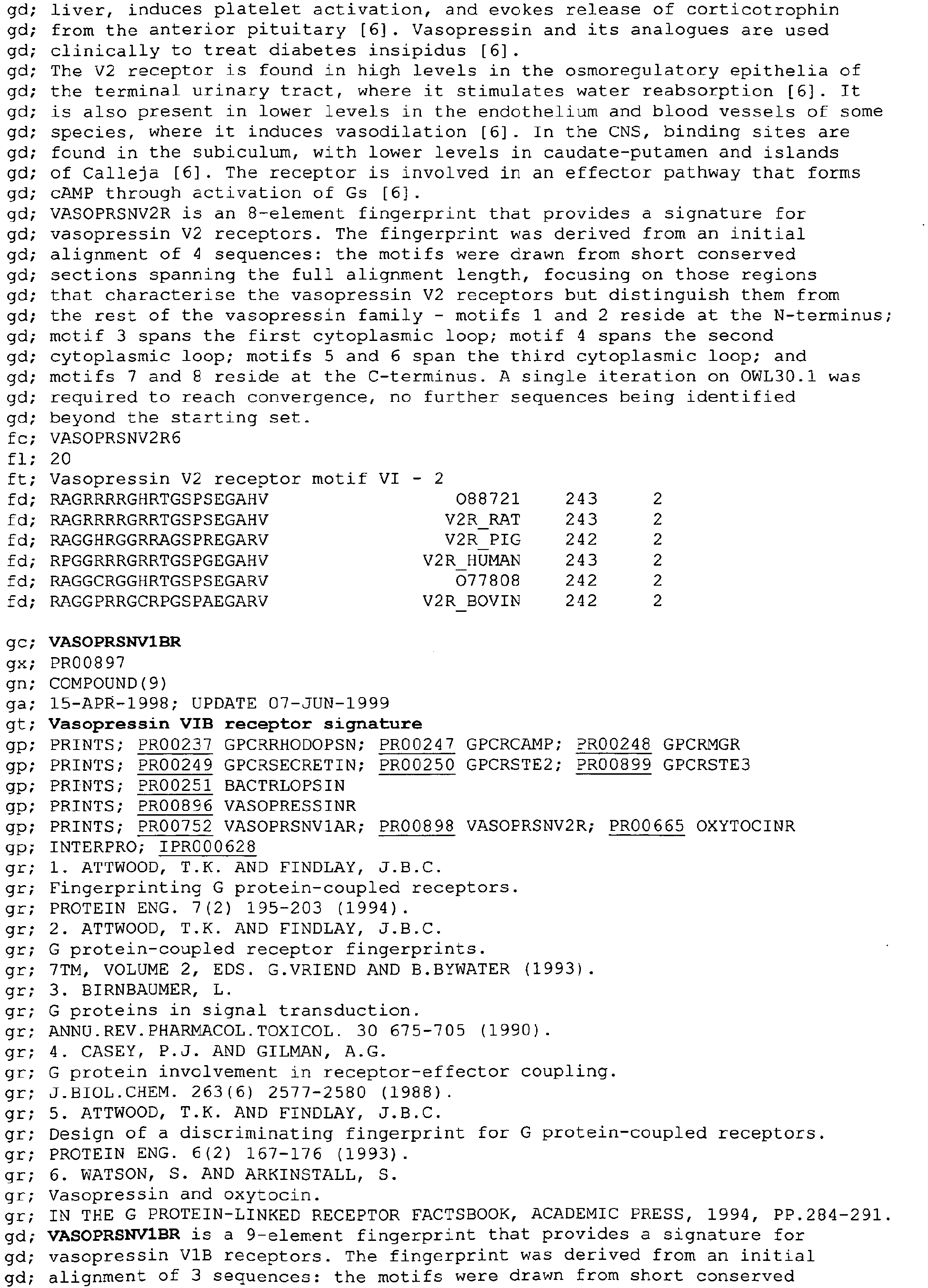

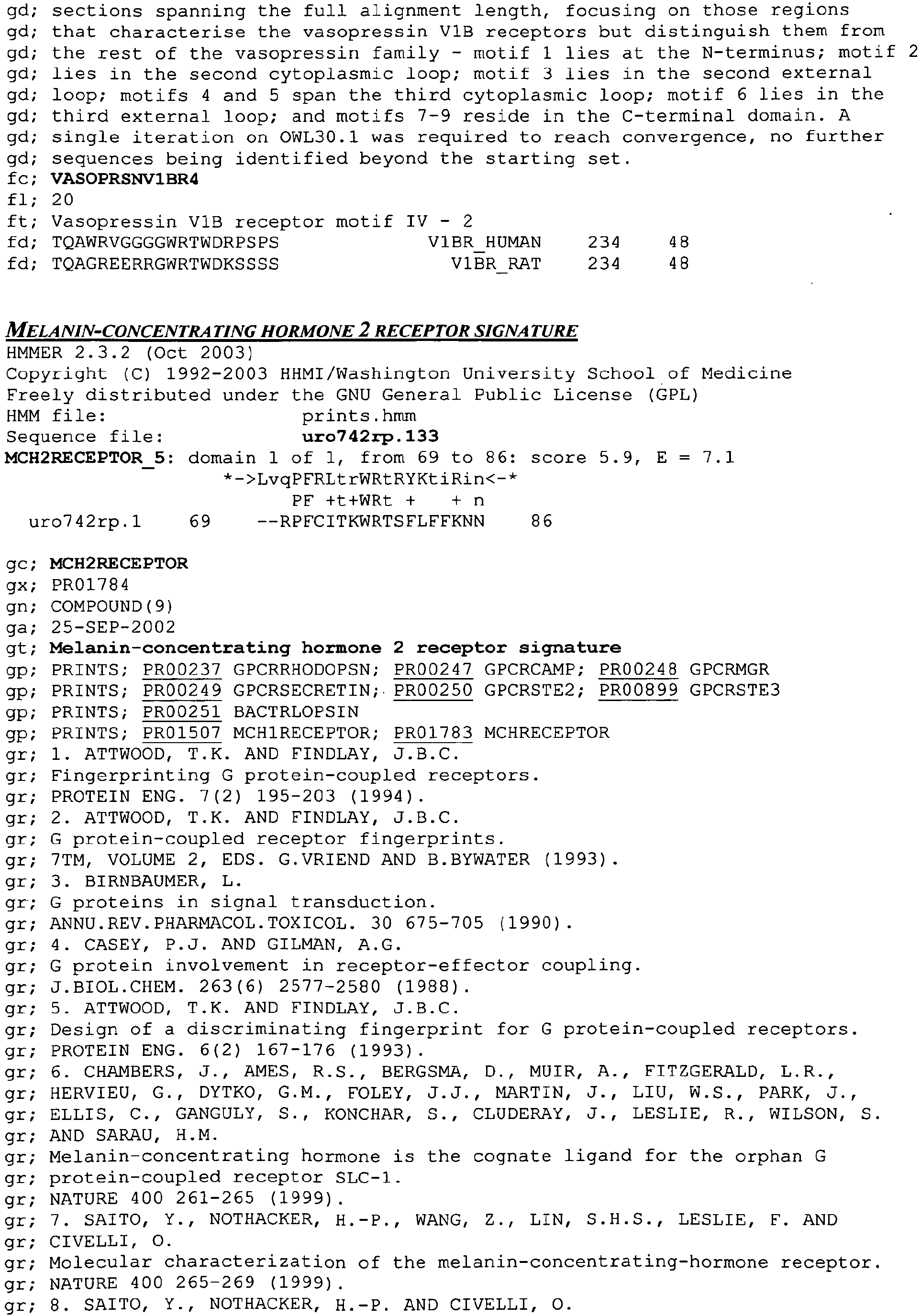

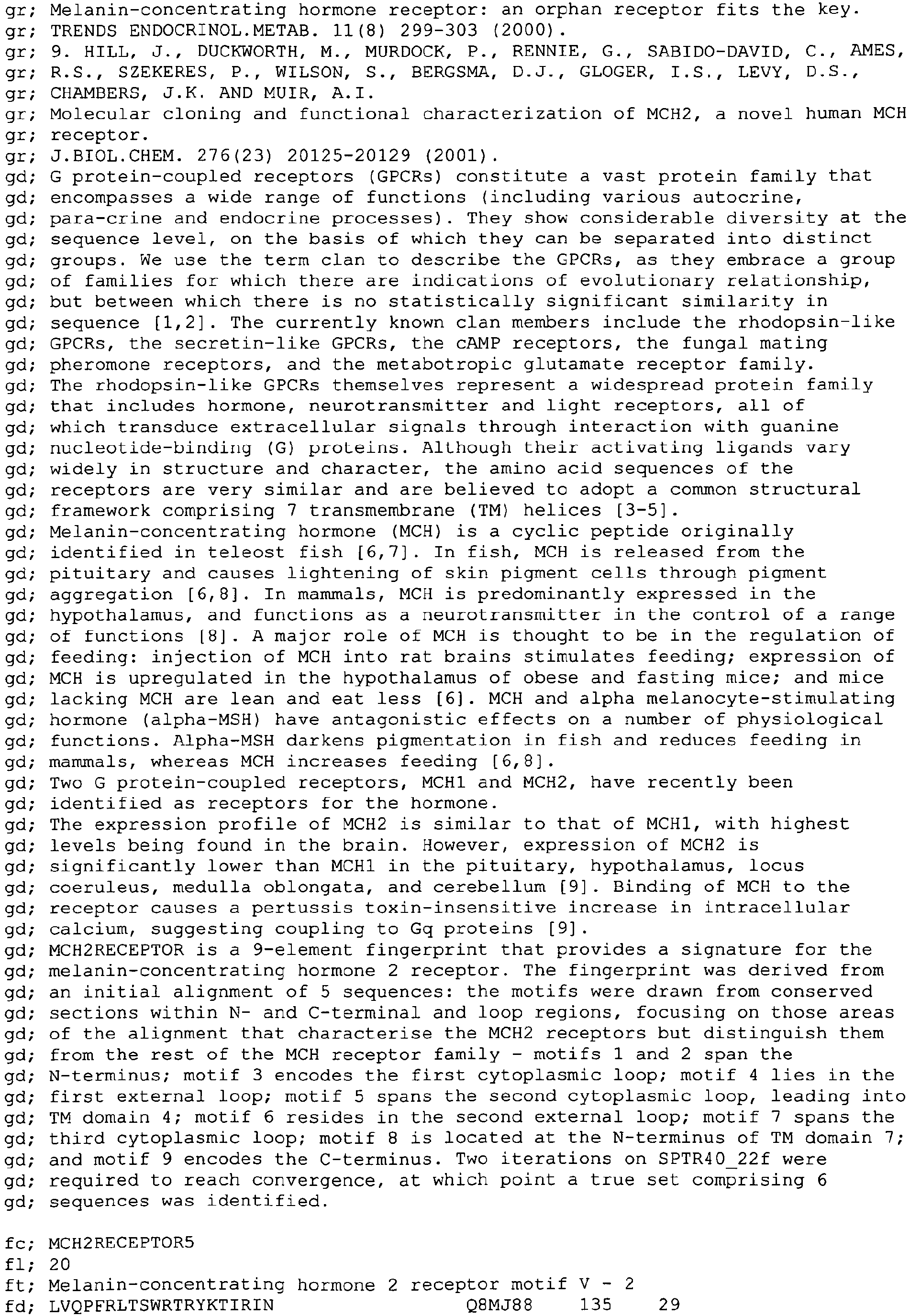

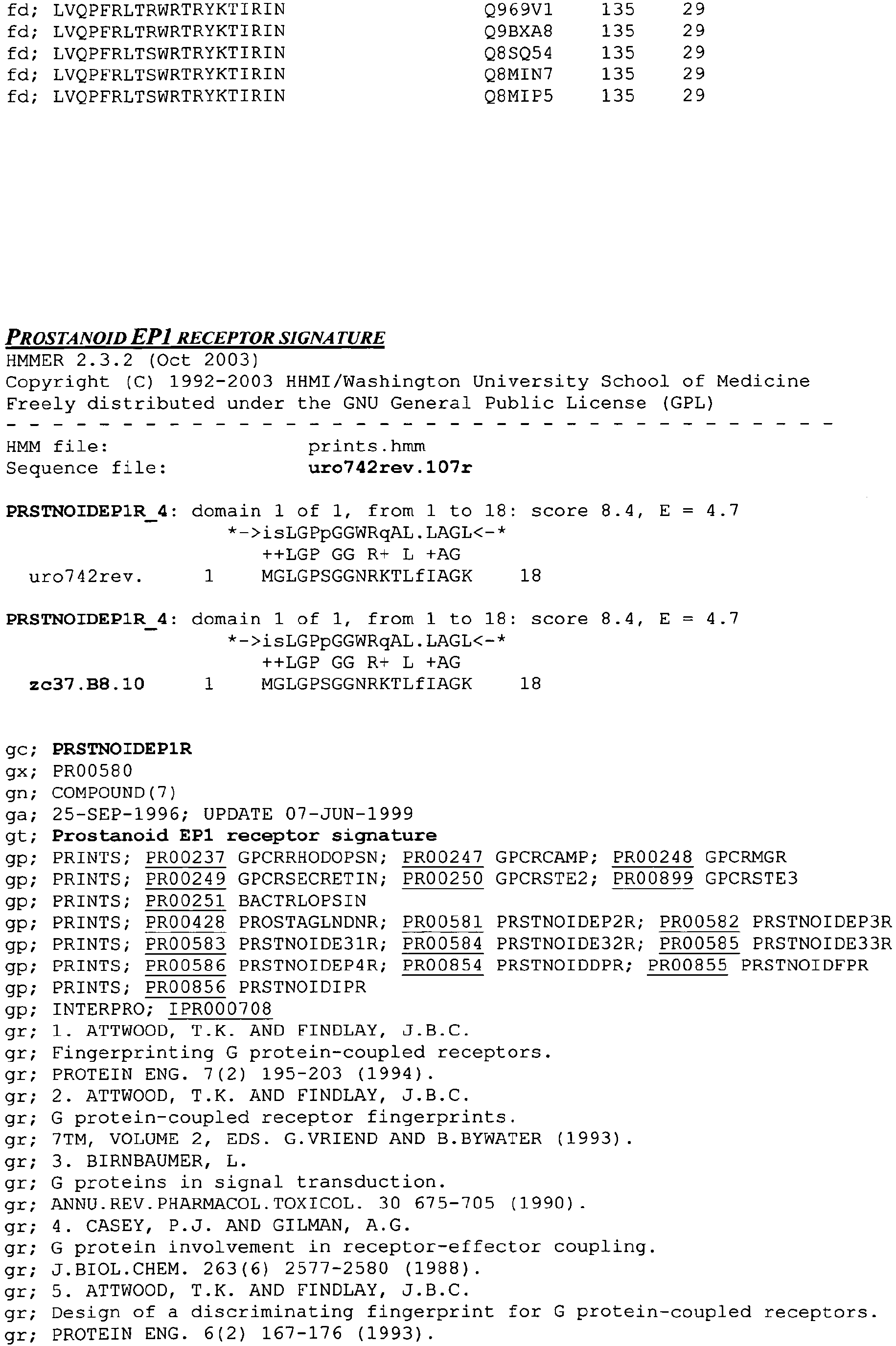

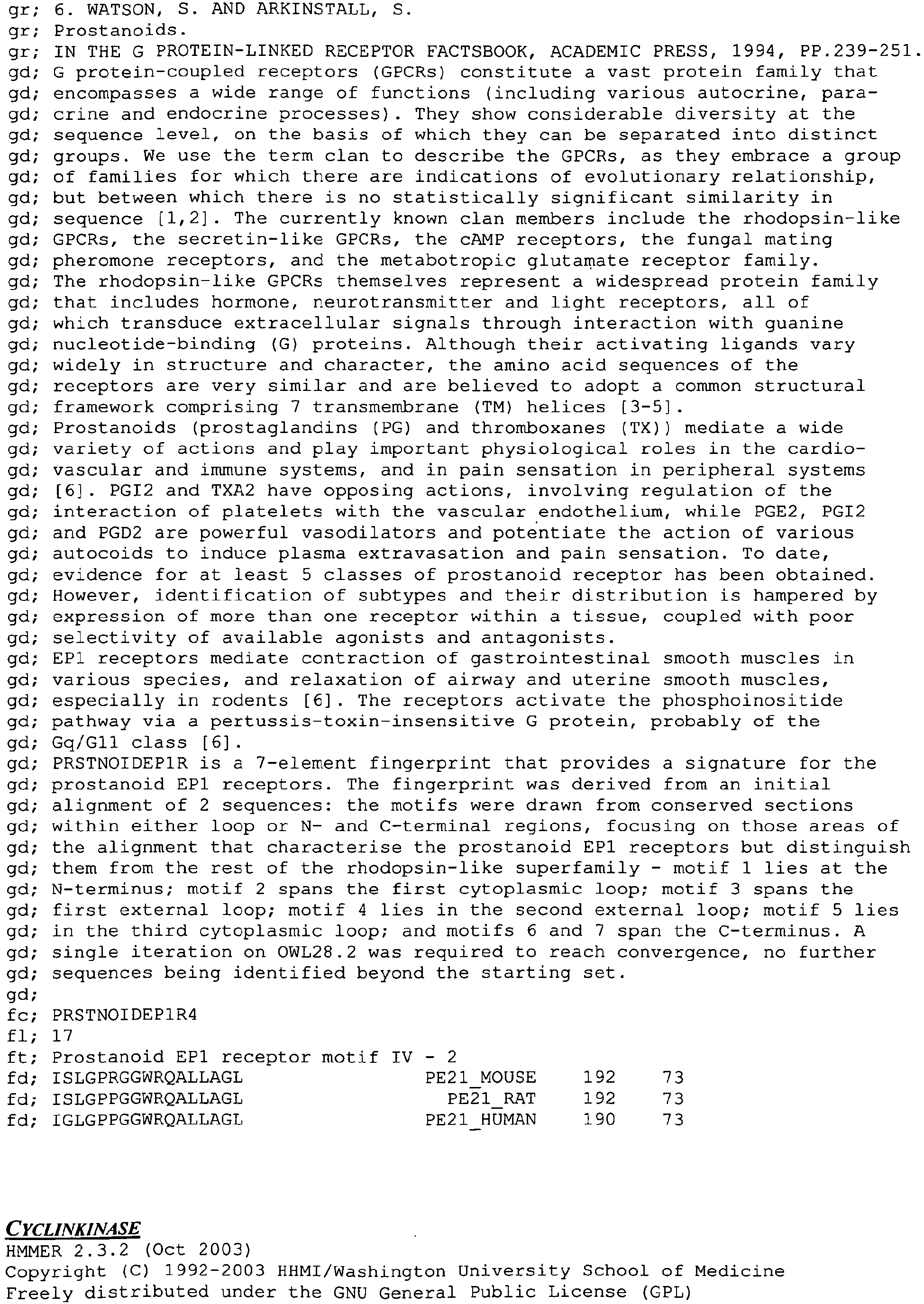

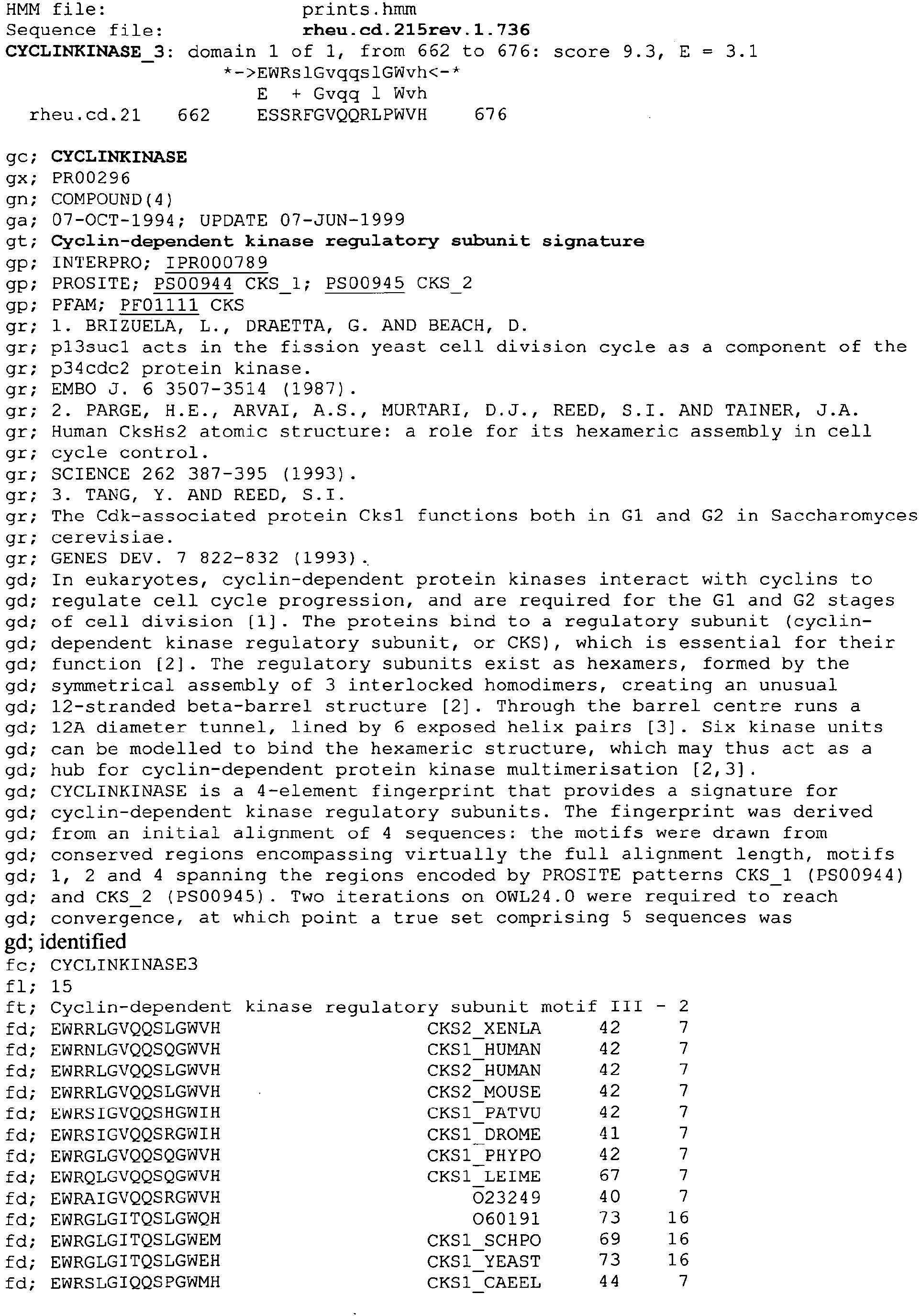

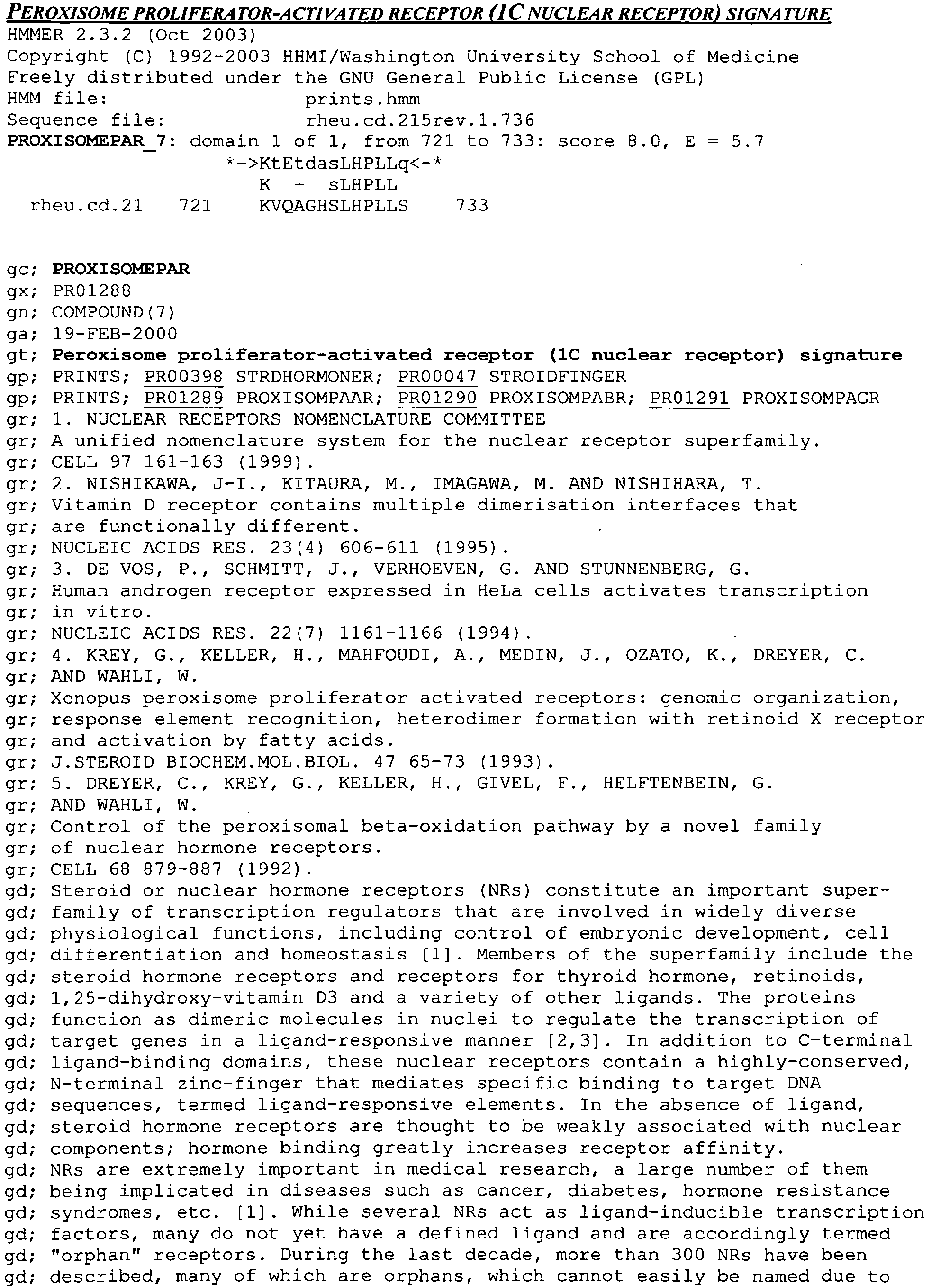

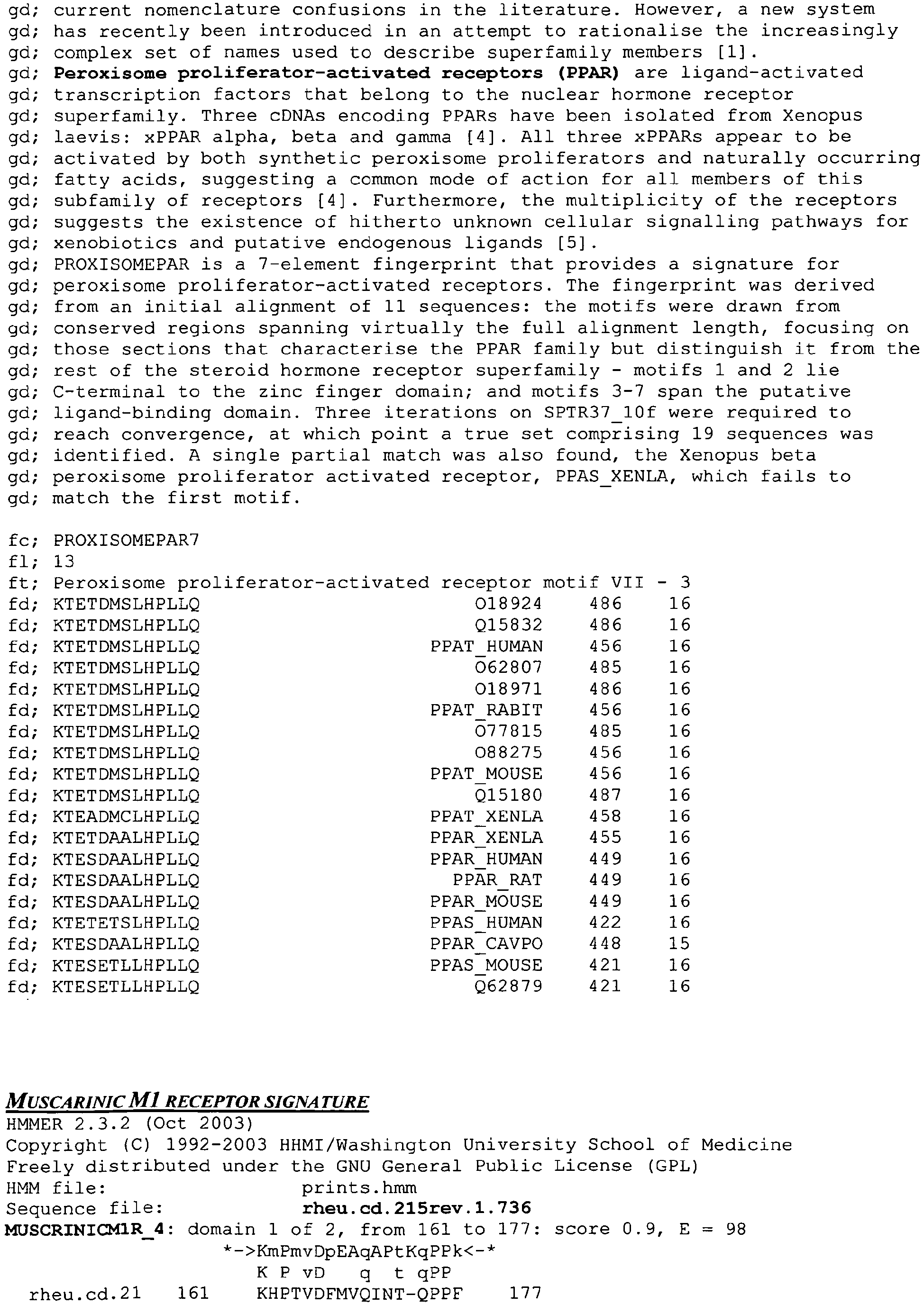

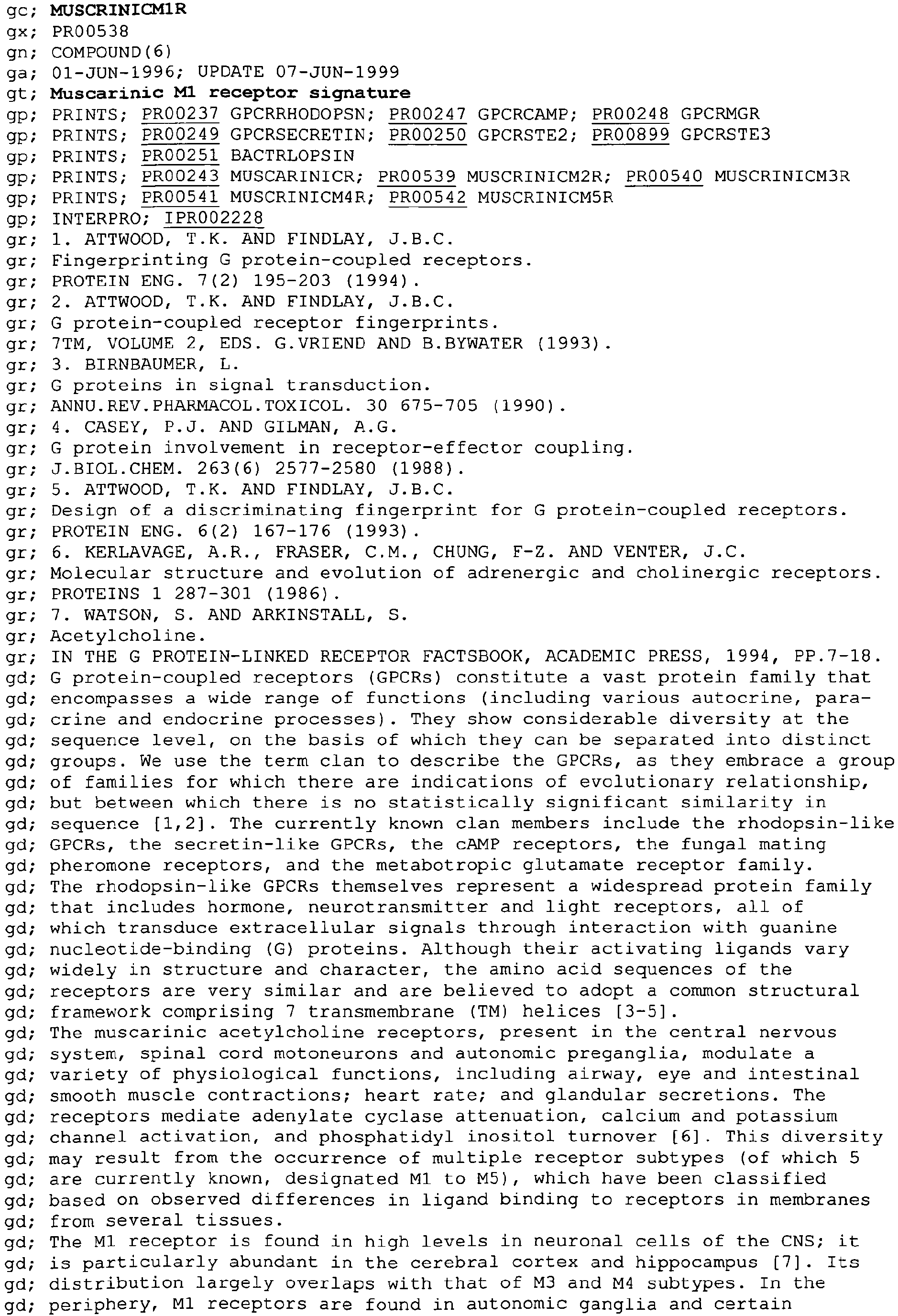

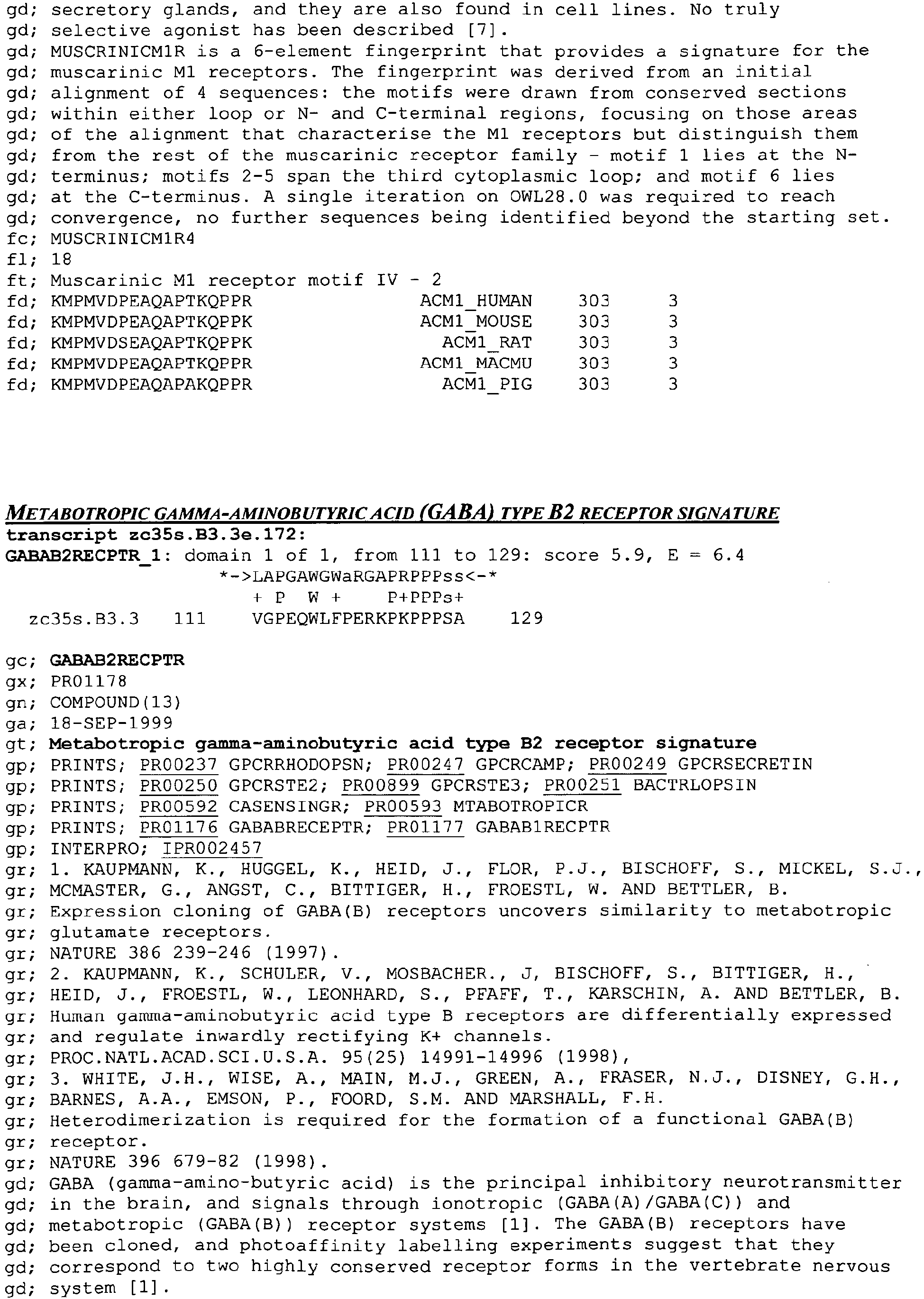

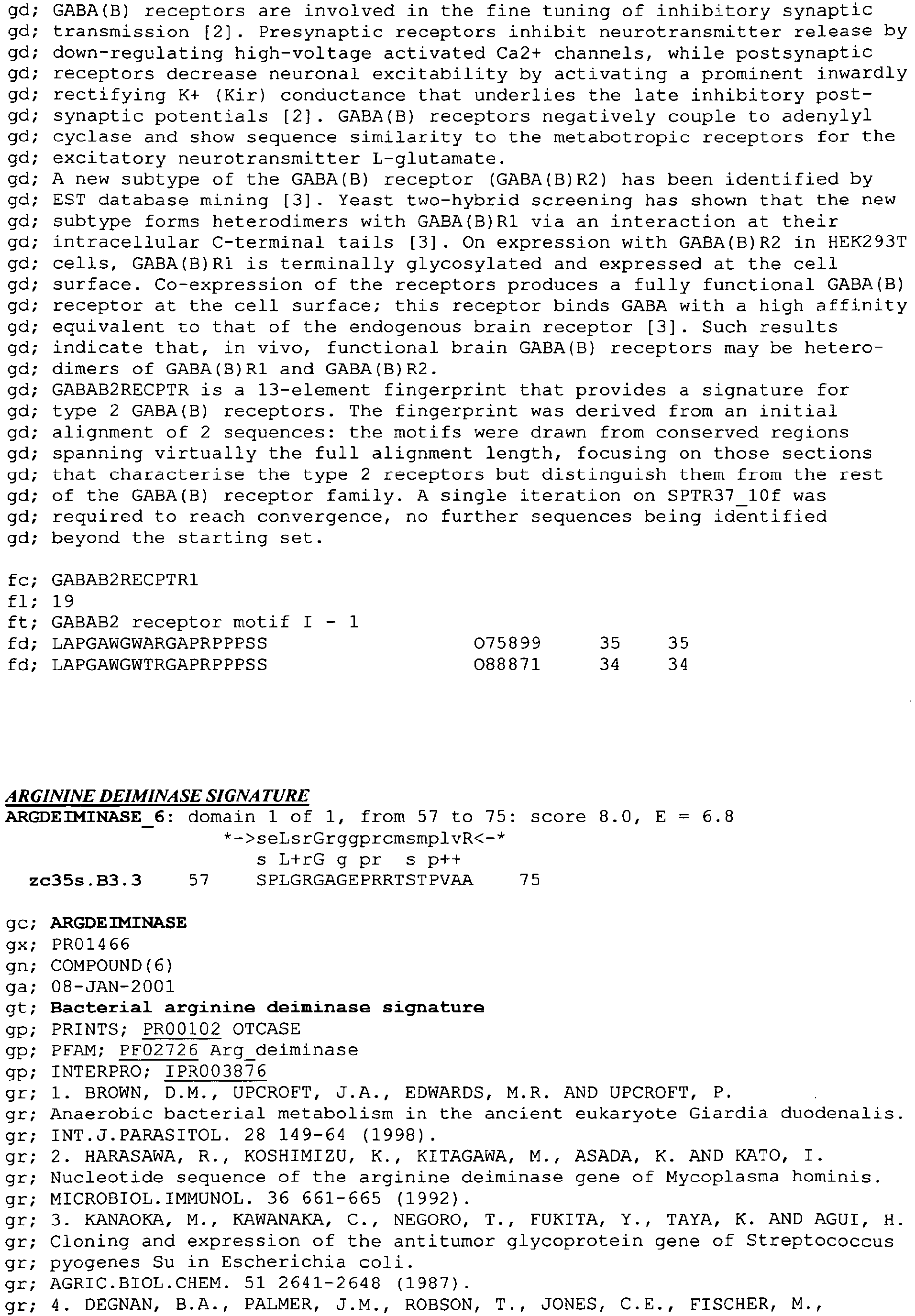

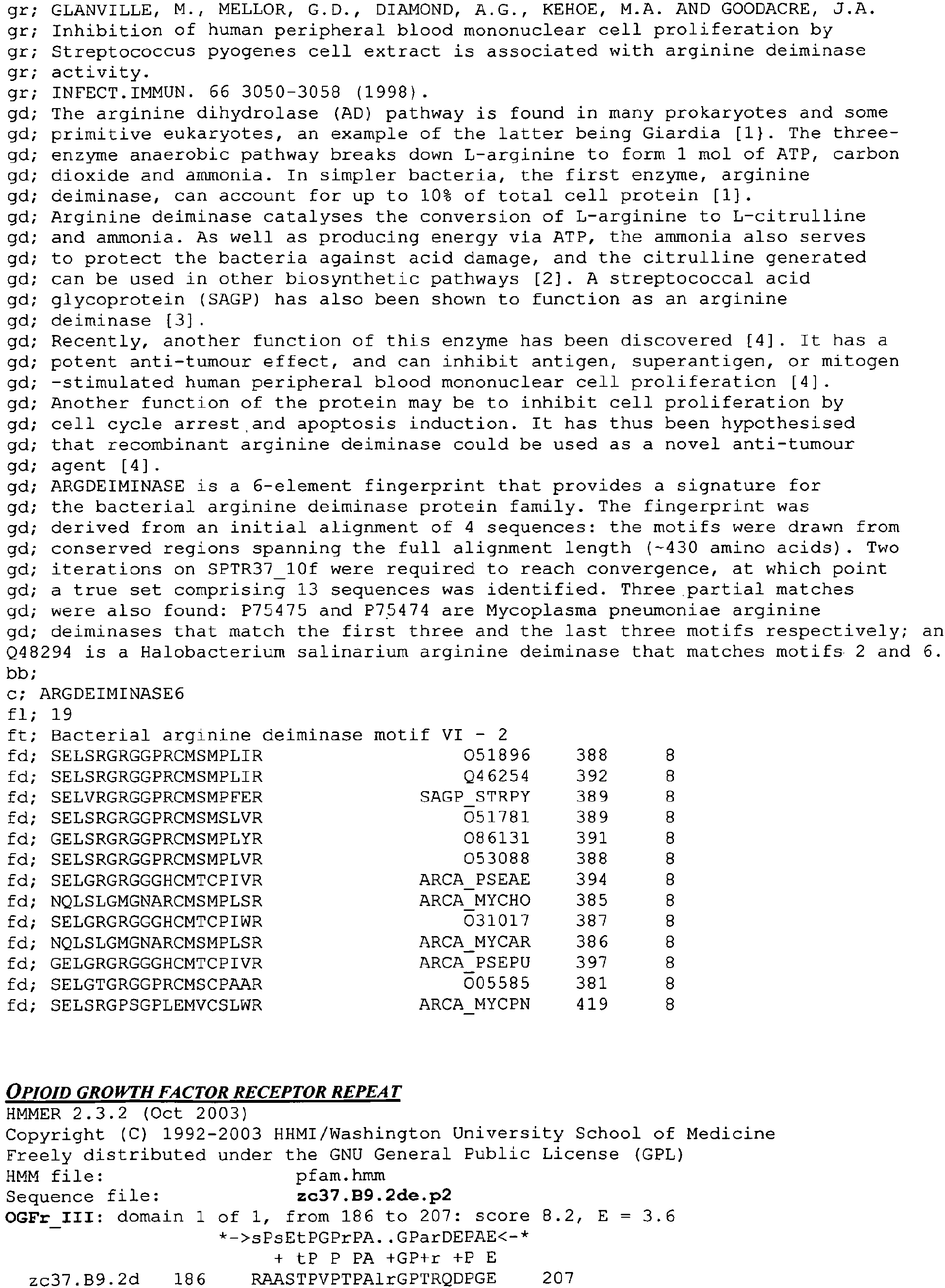

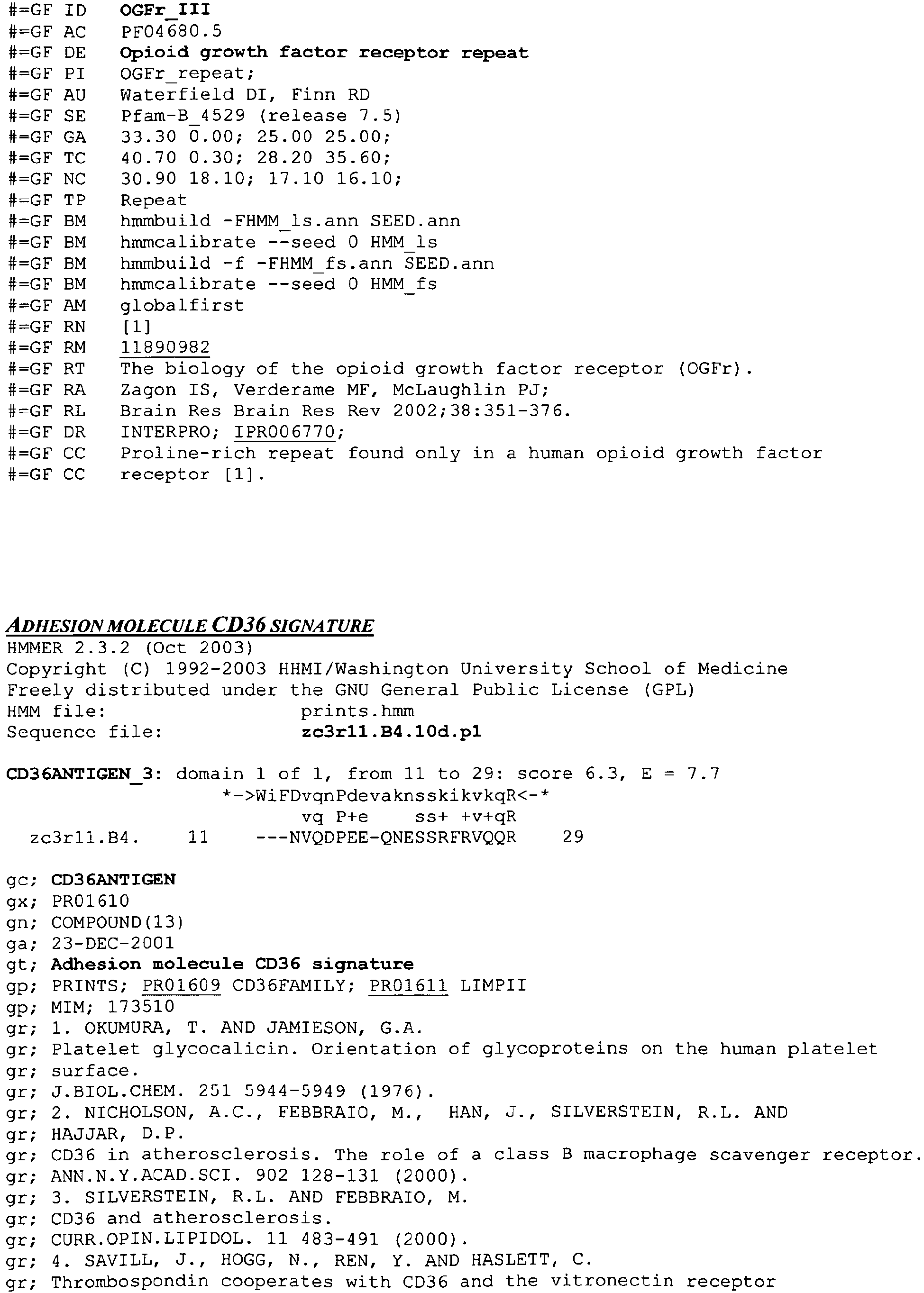

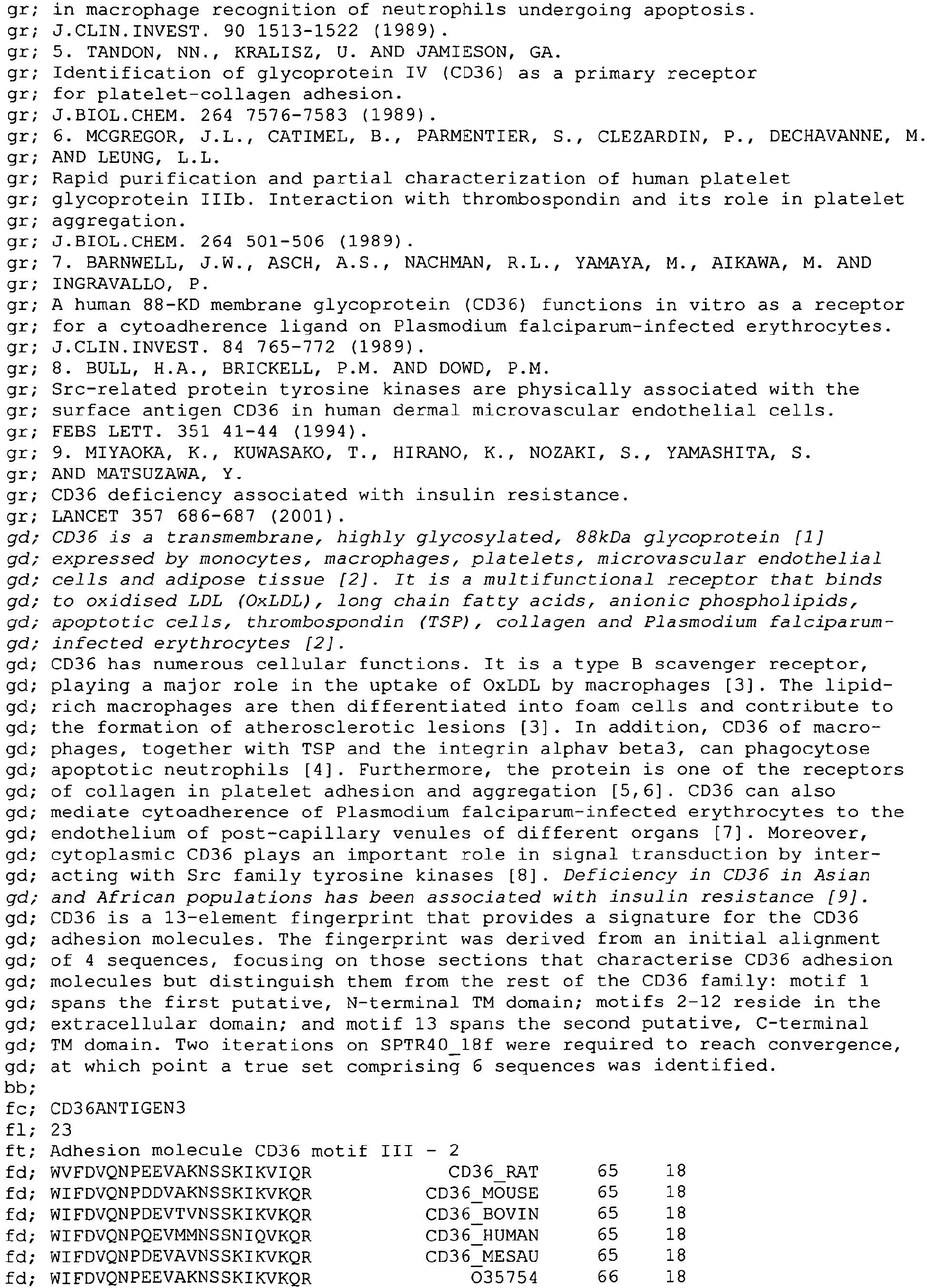

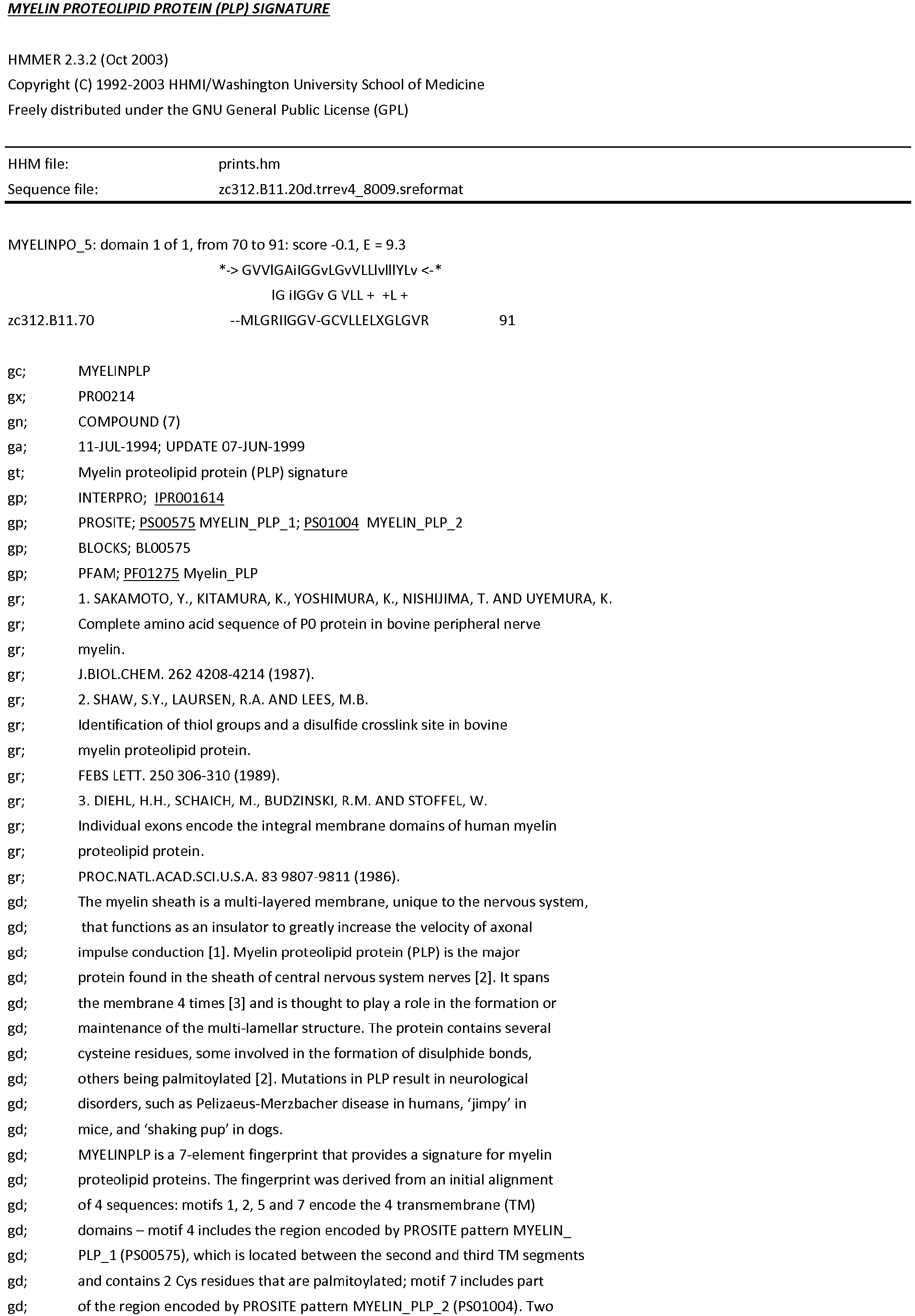

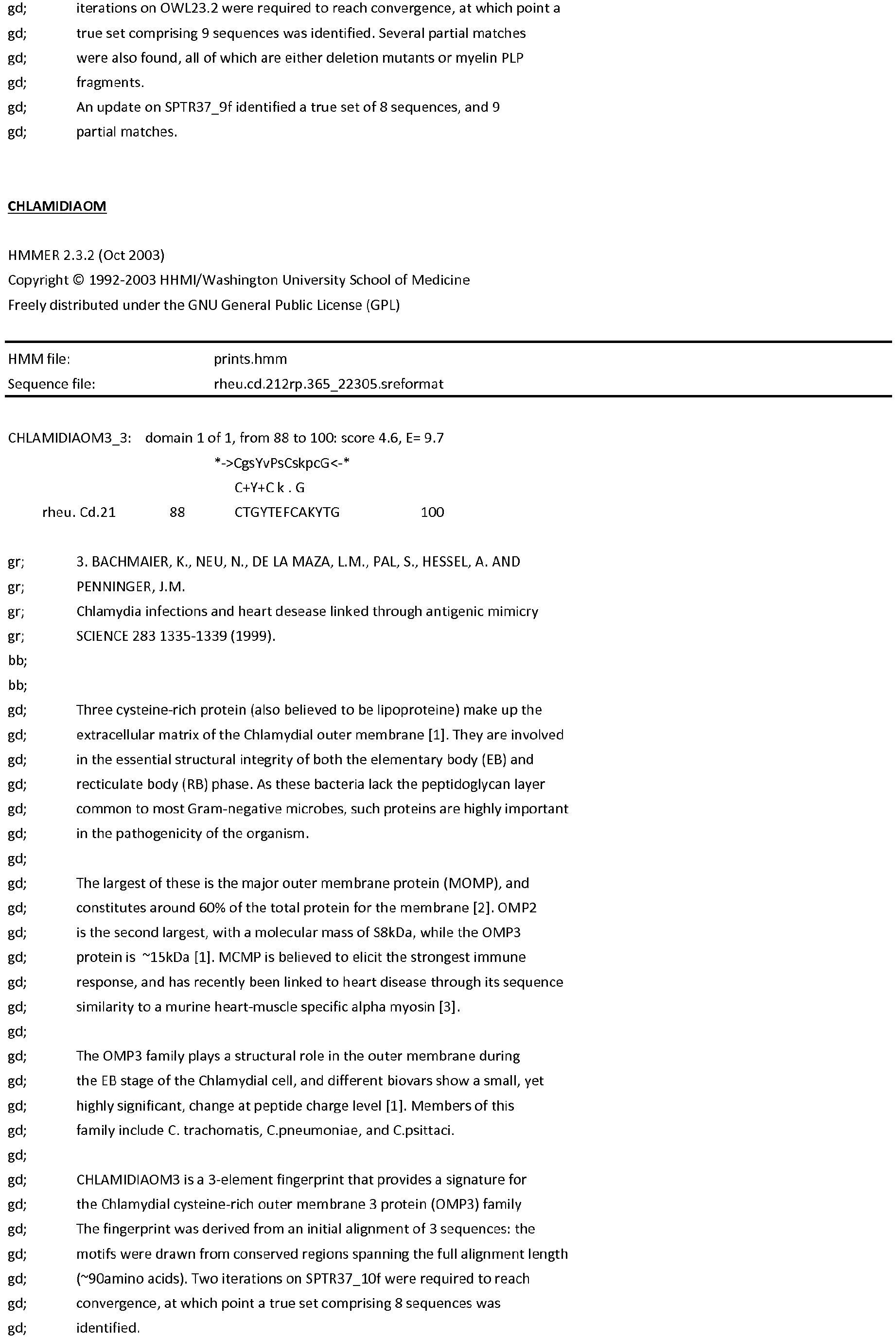

The present invention also relates to an oligonucleotide primer comprising or consisting of part of a polynucleic acid as defined above, with said primer being able to act as primer for specifically sequencing or specifically amplifying TT virus HCR polynucleic acid of the invention and attached cellular (host) DNA sequences.

The term "primer" refers to a single stranded DNA oligonucleotide sequence capable of acting as a point of initiation for synthesis of a primer extension product which is complementary to the nucleic acid strand to be copied. The length and the sequence of the primer must be such that they allow priming the synthesis of the extension products. Preferably the primer is about 5-50 nucleotides. Specific length and sequence will depend on the complexity of the required DNA or RNA targets, as well as on the conditions of primer use such as temperature and ionic strength.

The fact that amplification primers do not have to match exactly with corresponding template sequence to warrant proper amplification is amply documented in the literature. The amplification method used can be polymerase chain reaction (PCR), ligase chain reaction (LCR), nucleic acid sequence-based amplification (NASBA), transcription-based amplification system (TAS), strand displacement amplification (SDA) or amplification by means of Qβ replicase or any other suitable method to amplify nucleic acid molecules using primer extension. During amplification, the amplified products can be conveniently labelled either using labelled primers or by incorporating labelled nucleotides.

Labels may be isotopic (32P, 35S, etc.) or non-isotopic (biotin, digoxigenin, etc.). The amplification reaction is repeated between 20 and 70 times, advantageously between 25 and 45 times.

Any of a variety of sequencing reactions known in the art can be used to directly sequence the viral genetic information and determine the orf by translating the sequence of the sample into the corresponding amino acid sequence. Exemplary sequencing reactions include those based on techniques developed by Sanger or Maxam and Gilbert. It is also contemplated that a variety of automated sequencing procedures may be utilized when performing the subject assays including sequencing by mass spectrometry (see, for example: PCT publication WO 94/16101). It will be evident to one skilled in the art that, for example the occurrence of only two or three nucleic bases needs to be determined in the sequencing reaction.
Preferably, these primers are about 5 to 50 nucleotides long, more preferably from about 10 to 25 nucleotides. Most preferred are primers having a length of at least 13 bases. Preferably, a primer disclosed herein has a nucleotide sequence as shown in Table 2.

**Table 2**

| **Primers used to generate complete TTV-HD genomes and µTTV-HD subviral genomes by long distance PCR amplification** | | | |
|---|---|---|---|
| TTV | Primer | Nucleotide number | Sequence |
| TTV-jt34f (acc no AB064607) | jt34f-1s | 223-247 | 5'-GGCCGGGCCA TGGGCAAGGC TCTTA-3' |
| | jt34f-2as | 195-222 | 5'-AGTCAAGGGG CAATTCGGGC TCGGGACT-3' |
| | jt34f-5s | 205-222 | 5'-CAATTCGGGC TCGGGACT-3' |
| | jt34f-6as | 186-204 | 5'-ACACACCGCA GTCAAGGGG-3' |
| | jt34f-7s | 205-223 | 5'-CAATTCGGGC TCGGGACTG-3' |
| | jt34f-8as | 181-204 | 5'-AGTTTACACA CCGCAGTCAA GGGG-3' |
| TTV-HD1 (acc no AJ620222) | th25-1s | 126-156 | 5´-CCGCAGCGAG AACGCCACGG AGGGAGATCC T-3´ |
| | tth25-2as | 95-125 | 5′-ACTTCCGAAT GGCTGAGTTT TCCACGCCCG T-3′ |
| TTV-HD3 (acc no AJ620231) | tth8-1s | 133-164 | 5′-AGAGGAGCCA CGGCAGGGGA TCCGAACGTC CT-3′ |
| | tth8-2as | 102-132 | 5′-CTTACCGACT CAAAAACGAC GGGCAGGCGC C |
| TTV-HD4 (acc no AJ620226) | tth4-1s | 129-156 | 5′-CAGCGAGAAC GCCACGGAGG GAGATCCT- 3′ |
| | tth4-2as | 101-128 | 5'-GAATGGCTGA GTTTTCCACG CCCGTCCG-3' |
| TTV-t3pb (acc. no AF247138) | t3pb-1s | 209-226 | 5'-CAATTCGGGC *A*CGGGACT-3' * |
| | t3pb-2as | 185-208 | 5'-AGTTTACACA CCGAAGTCAA GGGG-3' |

| | | | |
|---|---|---|---|
| * A - TTV-t3pb sequence has a T at this position | | | |

The present invention also relates to an oligonucleotide probe comprising or consisting of part of a rearranged TT virus polynucleic acid as defined above, with said probe being able to act as a hybridization probe for specific detection of a TTV nucleic acid according to the invention.

The term "probe" refers to single stranded sequence-specific oligonucleotides which have a sequence which is complementary to the target sequence of the rearranged TTV polynucleic acid to be detected.

Preferably, these probes are about 5 to 50 nucleotides long, more preferably from about 10 to 25 nucleotides. Most preferred are probes having a length of at least 13 bases.

The probe can be labelled or attached to a solid support.

The term "solid support" can refer to any substrate to which an oligonucleotide probe can be coupled, provided that it retains its hybridization characteristics and provided that the background level of hybridization remains low. Usually the solid substrate will be a microtiter plate, a membrane (e.g. nylon or nitrocellulose) or a microsphere (bead). Prior to application to the membrane or fixation it may be convenient to modify the nucleic acid probe in order to facilitate fixation or improve the hybridization efficiency. Such modifications may encompass homopolymer tailing, coupling with different reactive groups such as aliphatic groups, NH₂ groups, SH groups, carboxylic groups, or coupling with biotin or haptens.

The oligonucleotides according to the present invention, used as primers or probes may also contain or consist of nucleotide analoges such as phosphorothioates, alkylphosphoriates or peptide nucleic acids or may contain intercalating agents. These modifications will necessitate adaptions with respect to the conditions under which the oligonucleotide should be used to obtain the required specificity and sensitivity. However, the eventual results will be essentially the same as those obtained with the unmodified oligonucleotides.

The introduction of these modifications may be advantageous in order to positively influence characteristics such as hybridization kinetics, reversibility of the hybrid-formation, biological stability of the oligonucleotide molecules, etc.
The polynucleic acids of the invention may be comprised in a composition of any kind. Said composition may be for diagnostic, therapeutic or prophylactic use.

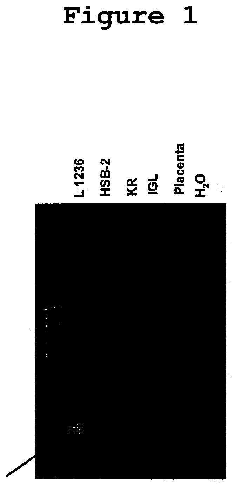

Also disclosed herein are sequence variants of the polynucleic acids as selected from any of the nucleotide sequences with said sequence variants containing either deletions and/or insertions of one or more nucleotides, especially insertions or deletions of 1 or more codons, mainly at the extremities of oligonucleotides (either 3' or 5'), or substitutions of some non-essential nucleotides by others (including modified nucleotides an/or inosine).
Rearranged TTV polynucleic acid sequences according to the present invention which are similar to the sequences as shown in Figure 1 can be characterized and isolated according to any of the techniques known in the art, such as amplification by means of sequence-specific primers, hybridization with sequence-specific probes under more or less stringent conditions, sequence determination of the genetic information of TTV, etc.
The present invention also relates to a recombinant expression vector comprising a rearranged TTV polynucleic acid of the invention as defined above operably linked to prokaryotic, eukaryotic or viral transcription and translation control elements.
The term "vector" may comprise a plasmid, a cosmid, an artificial chromosome, a phage, or a virus or a transgenic non-human animal. Particularly useful for vaccine development may be TT virus recombinant molecules, BCG or adenoviral vectors, as well as avipox recombinant viruses.

The term "recombinantly expressed" used within the context of the present invention refers to the fact that the polypeptides of the present invention are produced by recombinant expression methods be it in prokaryotes, or lower or higher eukaryotes as discussed in detail below.

The term "lower eukaryote" refers to host cells such as yeast, fungi and the like. Lower eukaryotes are generally (but not necessarily) unicellular. Preferred lower eukaryotes are yeasts, particularly species within Saccharomyces, Schizosaccharomyces, Kluiveromyces, Pichia (e. g. Pichia pastoris), Hansenula (e. g. Hansenula polymorph), Schwaniomyces, Schizosaccharomyces, Yarowia, Zygosaccharomyces and the like. Saccharomyces cerevisiae, S. carlsbergensis and K. lactis are the most commonly used yeast hosts, and are convenient fungal hosts.

The term "higher eukaryote" refers to host cells derived from higher animals, such as mammals, reptiles, insects, and the like. Presently preferred higher eukaryote host cells are derived from Chinese hamster (e. g. CHO), monkey (e. g. COS and Vero cells), baby hamster kidney (BHK), pig kidney (PK15), rabbit kidney 13 cells (RK13), the human osteosarcoma cell line 143 B, the human cell line HeLa and human hepatoma cell lines like Hep G2, and insect cell lines (e.g. Spodoptera frugiperda). The host cells may be provided in suspension or flask cultures, tissue cultures, organ cultures and the like. Alternatively the host cells may also be transgenic non-human animals.

The term "prokaryotes" refers to hosts such as E. coli, Lactobacillus, Lactococcus, Salmonella, Streptococcus, Bacillus subtilis or Streptomyces. Also these hosts are contemplated within the present invention.

The term "host cell" refers to cells which can be or have been, used as recipients for a recombinant vector or other transfer polynucleotide, and include the progeny of the original cell which has been transfected.

It is understood that the progeny of a single parental cell may not necessarily be completely identical in morphology or in genomic or total DNA complement as the original parent, due to natural, accidental, or deliberate mutation or recombination.

The term "replicon" is any genetic element, e. g., a plasmid, a chromosome, a virus, a cosmid, etc., that behaves as an autonomous unit of polynucleotide replication within a cell, i. e., capable of replication under its own control.

The term "vector" is a replicon further comprising sequences providing replication and/or expression of a desired open reading frame.

The term "control element" refers to polynucleotide sequences which are necessary to effect the expression of coding sequences to which they are ligated. The nature of such control sequences differs depending upon the host organism; in prokaryotes, such control sequences generally include promoter, ribosomal binding site, splicing sites and terminators; in eukaryotes, generally, such control sequences include promoters, splicing sites, terminators and, in some instances, enhancers. The term "control elements" is intended to include, at a minimum, all components whose presence is necessary for expression, and may also include additional components whose presence is advantageous, for example, leader sequences which govern secretion.

The term "promoter" is a nucleotide sequence which is comprised of consensus sequences which allow the binding of RNA polymerase to the DNA template in a manner such that mRNA production initiates at the normal transcription initiation site for the adjacent structural gene.

The expression "operably linked" refers to a juxtaposition wherein the components so described are in a relationship permitting them to function in their intended manner. A control sequence "operably linked" to a coding sequence is ligated in such a way that expression of the coding sequence is achieved under conditions compatible with the control sequences.

The segment of the rearranged TTV DNA encoding the desired sequence inserted into the vector sequence may be attached to a signal sequence. Said signal sequence may be that from a non-TTV source, but particularly preferred constructs according to the present invention contain signal sequences appearing in the TTV genome before the respective start points of the proteins.

Higher eukaryotes may be transformed with vectors, or may be infected with a recombinant virus, for example a recombinant vaccinia virus. Techniques and vectors for the insertion of foreign DNA into vaccinia virus are well known in the art, and utilize, for example homologous recombination. A wide variety of viral promoter sequences, possibly terminator sequences and poly(A)-addition sequences, possibly enhancer sequences and possibly amplification sequences, all required for the mammalian expression, are available in the art. Vaccinia is particularly preferred since vaccinia halts the expression of host cell proteins. For vaccination of humans the avipox and Ankara Modified Virus (MVA) are particularly useful vectors.

Also known are insect expression transfer vectors derived from baculovirus Autographa californica nuclear polyhedrosis virus (AcNPV), which is a helper-independent viral expression vector. Expression vectors derived from this system usually use the strong viral polyhedrin gene promoter to drive the expression of heterologous genes. Different vectors as well as methods for the introduction of heterologous DNA into the desired site of baculovirus are available to the man skilled in the art for baculovirus expression. Also different signals for posttranslational modification recognized by insect cells are known in the art.

The present invention also relates to a host cell as defined above transformed with a recombinant vector as defined above.

The present invention also relates to a diagnostic kit for use in determining the presence of a TT virus polynucleic acid of the invention, said kit comprising a primer and/or a probe of the invention.
Alternatively, the present invention also relates to a method for the detection of a rearranged TTV polynucleic acid according to the invention present in a biological sample, comprising: (a) optionally extracting sample polynucleic acid, (b) amplifying the polynucleic acid as described above with at least one primer as defined above, optionally a labelled primer, and (c) detecting the amplified polynucleic acids.
The term "polynucleic acid" can also be referred to as analyte strand and corresponds to a single- or double-stranded polynucleic acid molecule.
The term "labelled" refers to the use of labelled nucleic acids. This may include the use of labelled nucleotides incorporated during the polymerase step of the amplification or labelled primers, or by any other method known to the person skilled in the art.
The present invention also relates to a method for the detection of a rearranged TTV polynucleic acid according to the invention present in a biological sample, comprising: (a) optionally extracting sample polynucleic acid, (b) hybridizing the polynucleic acid as described above with at least one probe as defined above, and (c) detecting the hybridized polynucleic acids.
The hybridization and washing conditions are to be understood as stringent and are generally known in the art (e. g. Maniatis et al., Molecular Cloning: A Laboratory Manual, New York, Cold Spring Harbor Laboratory, 1982). However, according to the hybridization solution (SSC, SSPE, etc.), these probes should be hybridized at their appropriate temperature in order to attain sufficient specificity.

According to the hybridization solution (SSC, SSPE, etc.), these probes should be stringently hybridized at their appropriate temperature in order to attain sufficient specificity. However, by slightly modifying the DNA probes, either by adding or deleting one or a few nucleotides at their extremities (either 3' or 5'), or substituting some non-essential nucleotides (i. e. nucleotides not essential to discriminate between types) by others (including modified nucleotides or inosine) these probes or variants thereof can be caused to hybridize specifically at the same hybridization conditions (i. e. the same temperature and the same hybridization solution). Also changing the amount (concentration) of probe used may be beneficial to obtain more specific hybridization results. It should be noted in this context, that probes of the same length, regardless of their GC content, will hybridize specifically at approximately the same temperature in TMACI solutions.
Suitable assay methods for purposes of the present invention to detect hybrids formed between the oligonucleotide probes and the polynucleic acid sequences in a sample may comprise any of the assay formats known in the art, such as the conventional dot-blot format, sandwich hybridization or reverse hybridization. For example, the detection can be accomplished using a dot blot format, the unlabelled amplified sample being bound to a membrane, the membrane being incorporated with at least one labelled probe under suitable hybridization and wash conditions, and the presence of bound probe being monitored.
An alternative and preferred method is a "reverse" dot-blot format, in which the amplified sequence contains a label. In this format, the unlabelled oligonucleotide probes are bound to a solid support and exposed to the labelled sample under appropriate stringent hybridization and subsequent washing conditions. It is to be understood that also any other assay method which relies on the formation of a hybrid between the polynucleic acids of the sample and the oligonucleotide probes according to the present invention may be used.

Finally, disclosed herein is a process for the *in vitro* replication and propagation of Torque teno viruses (TTV), preferably a rearranged TTV according to the present invention, comprising the following steps:
(a) transfecting linearized TTV DNA into 293TT cells expressing high levels of SV40 large T antigen, preferably at least levels as reported in Buck et al.(2004);
(b) harvesting the cells and isolating cells showing the presence of TTV DNA;
(c) culturing the cells obtained in step (b) for at least three days, preferably at least one week or longer, depending on experimental conditions and TTV type concerned; and
(d) harvesting the cells of step (c).

The following examples illustrate the invention.

### Example 1

### Materials and Methods

### (A) TT virus isolation and characterization

The isolation of TT virus isolates TTV-HD3a (tth8, accession no AJ620231) and TTV-HD1a (tth25, acc. no AJ620222) was previously described (Jelcic et al., 2004). Full-length genomic sequences of both TTV-HD3a and TTV-HD1a were cloned into the vector pUC18 using restriction enzymes Sail (Leppik et al., 2007) and EcoR1, respectively. Additional TTV sequences were identified in human samples by DNA nested amplification using primers NG472/NG352 and NG473/NG351 as previously described (Peng et al., 2002; Leppik et al., 2007). The limited availability of DNA for a number of biopsy and serum samples required prior amplification using rolling circle amplification with a TempliPhi Kit (GE Healthcare). All amplified products were cloned and sequenced (Leppik et al., 2007). Samples harbouring TT virus DNA were subsequently subjected to long distance-PCR amplification using TaKaRa LA *Taq* enzyme (TAKARA BIO INC., Japan) and respective primers which had been designed based on the initially identified TTV DNA sequences. These back-to-back primers included the following combinations: tth25-1s and tth25-2as, jt34f-1s and jt34f-2as, jt34f-7s and jt34f-8as, jt34f-5s and jt34f-6as, tth4-1s and tth4-2as, t3pb-1s and t3pb-2as, as well as tth8-1s and tth-2as (Table 2). Long-PCR amplification was performed using a touchdown stepwise reaction as described previously (Leppik et al., 2007) with the exception of primer combinations t3pb-1/2, jt34f-5/6 and tth4. PCR conditions for PCR amplification with t3pb-1/2 and jt34f-5/6-primers were an initial denaturation at 94°C for 1 min, followed by 30 cycles of 94°C for 30 sec, annealing at 65°C for 1 min and elongation at 72°C for 4 min with a final elongation at 72°C for 10 min. PCR conditions for amplification with tth4 primers were similar except that annealing was performed at 68°C. All obtained amplicons in the range of 3,8 kb were eluted and purified after gel electrophoresis, cloned into vector pCR2.1 (TA-Cloning-Kit, Invitrogen) and propagated in NovaBlue Singles Competent Cells (Merck Chemicals, UK). All full-length genomes were sequenced through both strands. A total of 53 full-length genomes was obtained.

### (B) Sequence analyses and phylogeny

DNA sequences were compared to TTV sequences available in all databanks using the HUSAR software package (Jelcic et al., 2004). The ICTV recently classified TT viruses into the family *Anelloviridae* based on the DNA sequence of large open reading frame 1 (ORF1) (Biagini and de Micco, 2010). Characterizing the genomes of the isolates obtained revealed rearrangement of sequences in the ORF1 region. The full-length genomes of the genus Alphatorquevirus and the isolates were therefore subjected to phylogenetic analyses as previously described (Jelcic et al., 2004). The phylogenetic tree (Figure 4) was displayed using the Treeview program of the University of Glasgow. Translated ORFs were analyzed for homologous proteins and functional domains by using ProtSweep (del Val et al., 2004).

### (C) Cell culture and transfection

The human embryonic kidney cell line 293TT (Buck et al., 2004) was maintained in DMEM supplemented with 10% fetal calf serum, 1% Glutamax, 1% non-essential amino acids (both Invitrogen, Karlsruhe, Germany) and 400 µg/ml Hygromycin B (Roche Diagnostics, Mannheim). Linearized virus DNA (2 µg per well on 6-well plates) was transfected into cells grown without Hygromycin B using Lipofectamine reagent (Invitrogen) according to the manufacturer's instructions (Fei et al., 2005). Culture medium (2 ml) was supplemented with 800 µl Opti-MEM prior to incubation for 4 hours at 37°C. Transfected cultures were subsequently incubated with fresh medium containing Hygromycin B and propagated when confluency was reached. Full-length genomes of 12 TTV isolates were transfected, maintained and harvested in parallel at all times. TT virus genomes included TTV-HD14a, TTV-HD14b, TTV-HD14c, TTV-HD14e, TTV-HD15a, TTV-HD16a, TTV-HD20a, TTV-HD3a, TTV-HD1a, TTV-HD23a, TTV-HD23b and TTV-HD23d (Table 3).

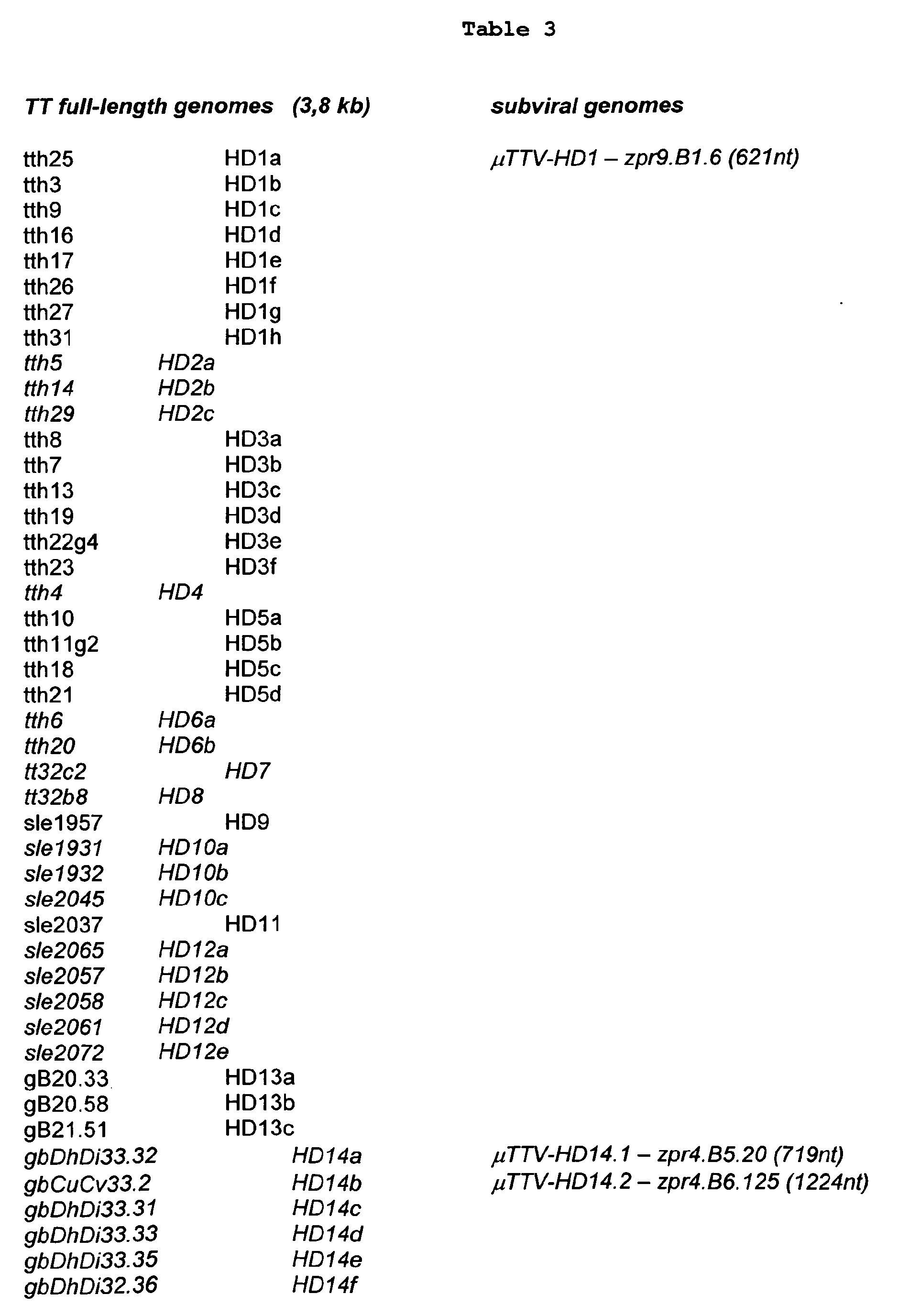

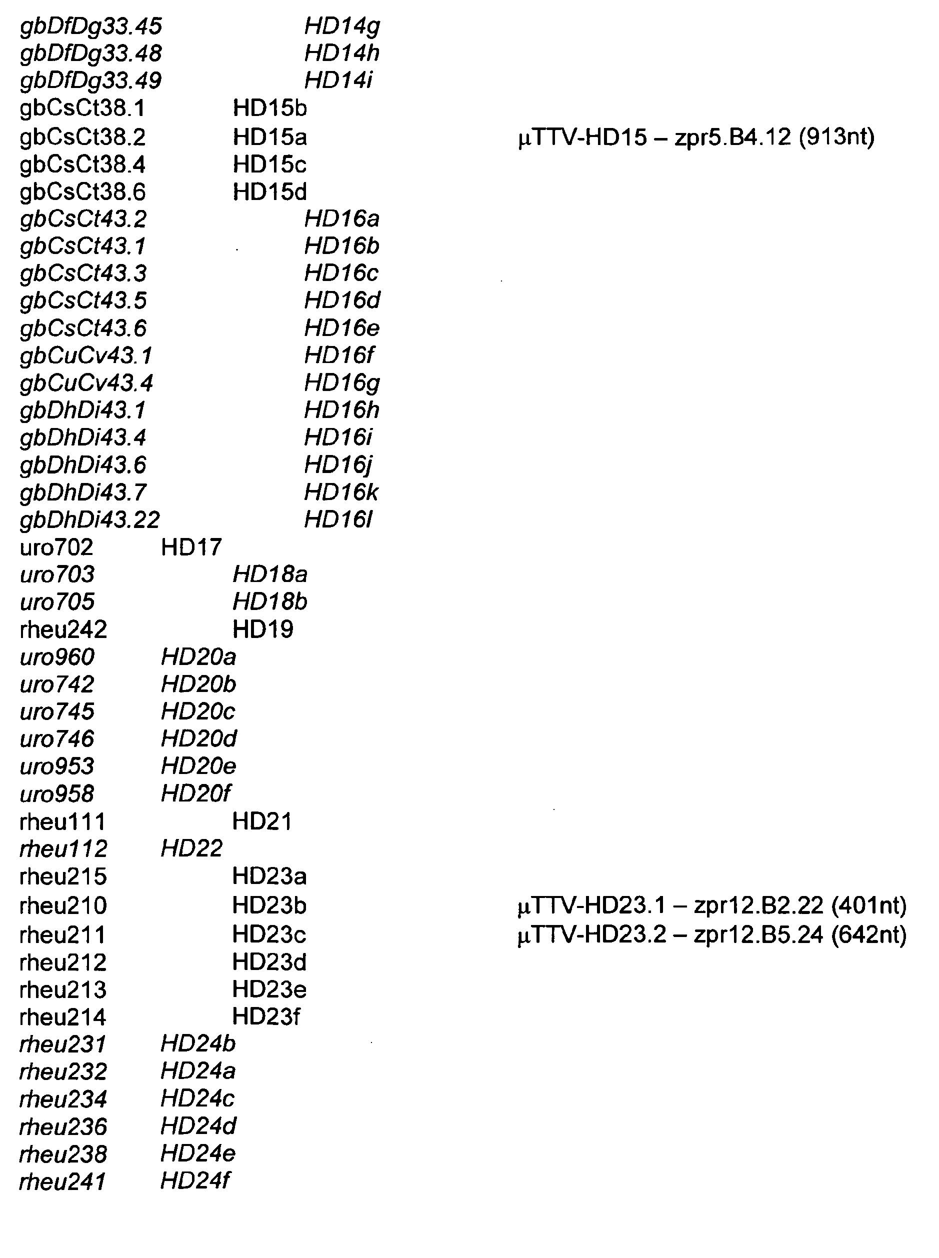

Virus DNA was released from the vector prior to transfection. Controls included transfection with vector alone and cells transfected with 1 x TE. Transfected cells and culture medium were frozen at -80°C and samples for DNA and RNA extraction taken at each time point during propagation. DNA was extracted with phenol-chloroform-isoamylalcohol and RNA using the RNeasy Mini Kit (Qiagen, Hilden, Germany). Replication of virus DNA was monitored and demonstrated by long-PCR amplification as described above. All transfection experiments were performed 3 times with 6 week intervals between primary transfections. Frozen cells or purified virus preparations were passaged between 4 to 6 times.

### (D) Virus propagation, purification and electronmicroscopy

Transfected cells were harvested from flasks by shaking followed by centrifugation for 10 min at 200 g. Cell pellets were resuspended in DPBS-Mg (Invitrogen) and separated on a 27-33-39% Optiprep (Sigma, St. Louis, MO) step gradients for 3.5 hr at 234,000 g (Buck et al., 2005). Gradients were fractionated and screened for the presence of virus DNA by gel electrophoresis of lysed aliquots. Aliquots were lysed with proteinase K, 0.25 mM EDTA and 0.5% SDS for 10 min at 56°C immediately prior to loading onto the gel. The supernatant of the re-suspended cells were alternatively filtered through a 0.22 µm filter. Aliquots of gradient fractions, as well as filtered supernatants were frozen at - 80°C for use as inoculum. Filtered aliquots were pelleted. Pellets were subjected to negative staining and visualized by electronmicroscopy. Cloned subviral µTTV genomes were transfected into 293TT in the same way as the full-length genomes. The cultures were propagated over several weeks. Cells were partially removed by scraping off part of the monolayer cells while allowing outgrowth of the remaining cells. Removed cells were pelleted and supernatant was filtered through a 0,22 µm filter before visualization in the electron microscopy. Cell pellets were treated as described above prior to centrifugation and separation through Optiprep gradients. Aliquots were lysed and the DNA visualized after gel electrophoresis.

### (E) Transcription analyses

Transcripts of TTV-HD full-length genomes were analysed using two different approaches. 5'- and 3'-RACE products were generated from single- as well as double-stranded cDNA. Single-stranded 5'-RACE-Ready and 3'- RACE-Ready cDNAs were respectively synthesized from 1 µg purified total RNA in a 10 µl reaction mix using the SMARTer™RACE cDNA Amplification Kit (Clontech cat#634923) in which RNA is reverse transcribed by SMARTScribe™ Reverse Transcriptase at 42°C for 90 min. 3'RACE-CDS primer A was used for the synthesis of 3'RACE-Ready cDNA, whereas the 5'RACE-CDS primer A and SMARTer IIA oligonucleotide were used for the synthesis of 5'-RACE-Ready cDNA. Double-stranded cDNA was concomitantly synthesized. Here full-length single stranded cDNA was initially synthesized using the SMARTer™PCR cDNA Synthesis Kit (Clontech cat#634925) according to the manufacturer's protocol. Purified total RNA (1 µg) was transcribed using SMARTScribe™ Reverse Transcriptase and primers 3'SMART CDS PrimerIIA and SMARTer IIA Oligonucleotide. These primers both contain a non-template nucleotide stretch thereby creating an extended template. Second-strand cDNA amplification was obtained by long distance PCR amplification (LD PCR) with 5'PCR Primer IIA and the Advantage 2 polymerase mix (Clontech cat#639201). PCR amplification was performed at follows: 15 sec at 95°C, 30 sec at 65°C and 3 min at 68°C per cycle and ranging number of cycles in order to determine optimal conditions.

5'- and 3'-RACE PCR amplification was performed using 5'-RACE-Ready or 3'-RACE-Ready cDNA, respectively, or double-stranded cDNA template in both cases. RACE-PCR was performed using Advantage 2 polymerase mix, a universal primer A mix (UPM) from the SMARTer™RACE cDNA Amplification Kit and forward and reverse primers fitting to the respective TTV types (Table 4).

**Table 4**

| **Nucleotide positions of primers used for PCR amplification in RACE** | | | |
|---|---|---|---|
| TTV | primer | Nucleotide number | transcript |
| TTV-HD14b | 1-f1 | 716-743 | + |
| | 1-f3 | 2886-2912 | + |
| | 1-r1 | 757-730 | + |
| | 1-r2 | 3521-3492 | + |
| TTV-HD14c | 2-f1 | 716-743 | + |
| | 2-f2 | 3054-3082 | + |
| | 2-r3 | 2912-2885 | + |
| TTV-HD14a | 3-f1 | 717-744 | + |
| | 3-f2 | 2890-2917 | + |
| | 3-f3 | 3496-3521 | + |
| | 3-r1 | 745-720 | - |
| | 3-r2 | 2914-2887 | + |
| TTV-HD14e | 4-f1 | 2887-2914 | + |
| | 4-f2 | 3494-3519 | + |
| | 4-f3 | 3053-3080 | + |
| | 4-r1 | 757-730 | + |
| | 4-r2 | 2911-2884 | + |
| TTV-HD15a | 5-f1 | 125-149 | + |
| | 5-f2 | 2807-2834 | + |
| | 5-f3 | 3388-3415 | + |
| | 5-r1 | 224-197 | + |
| | 5-r2 | 3014-2987 | + |
| | 5-r3 | 3425-3398 | - |
| TTV-HD16a | 6-f1 | 100-127 | + |
| | 6-f2 | 3145-3172 | - |
| | 6-f3 | 3564-3591 | - |
| | 6-r1 | 3204-3182 | + |
| | 6-r2 | 3443-3418 | - |
| TTV-HD20a | 7-f1 | 314-341 | + |
| | 7-f2 | 3025-3052 | + |
| | 7-r1 | 227-200 | + |
| | 7-r2 | 743-716 | + |
| | 7-r3 | 3332-3305 | - |
| TTV-HD23b | 10-f1 | 113-139 | - |
| | 10-f3 | 3121-3148 | + |
| TTV-HD23d | 11-f1 | 126-148 | + |
| | 11-f2 | 354-381 | + |
| | 11-f3 | 3397-3422 | - |
| | 11-r1 | 226-199 | + |
| | 11-r2 | 3653-3626 | + |
| | 11-r3 | 3327-3302 | + |
| TTV-HD23a | 12-f1 | 126-148 | + |
| | 12-f2 | 354-381 | + |
| | 12-r2 | 3177-3150 | + |
| | 12-r3 | 3326-3301 | + |

Conditions for amplification were: 29 cycles of 30 sec at 94°C, annealing for 30 sec at 68°C and elongation for 3 min at 72°C, with a final extension for 15 min at 72°C. All products were analysed by gel electrophoresis, purified after gel elution, cloned into vector pCR2.1 (Invitrogen cat#K2020-40) and sequenced. Two additional controls were performed in order to control for non-specific amplification. In one control amplification was performed using only one TTV-specific primer and in the second using the UPM primer alone. No products were detected in either of these.

### Example 2

### Demonstration of the persistence of TTV DNA in cells from tissue culture lines derived from malignant tumors

Cell lines derived from malignant tumors possess one advantage over primary tumor biopsy material. They commonly represent pure preparations of cancer cells, whereas primary materials are commonly contaminated by normal mesenchymal cells, by cells of the hematopoietic system and normal epithelial cells. On the other hand, one disadvantage of tissue culture lines may arise from the selection of specific clones growing under tissue culture conditions and the acquisition of secondary genetic modifications in the course of long-term cultivation. In addition, fetal calf sera may pose a risk due to the introduction of cattle viruses which survive serum inactivation procedures (e.g. bovine polyomavirus); see Table 5 summarizing these advantages/disadvantages.

**Table 5**

| **Analysis of primary tumor biopsies vs established cell lines for TTV-related sequences** | | | |
|---|---|---|---|
| | | | |
| **Biopsies** | | **Cell lines** | |
| **Advantage** | **Disadvantage** | **Advantage** | **Disadvantage** |
| **Authentic materials** | **Contaminated by admixture of normal cells** | **Pure preparations of cancer cells** | **Selection of specific Clones adapted to tissue culture conditions** |
| | | | |
| | **Search for TTV sequences clouded by the uniform presence of TTV in the peripheral blood** | **Available in unlimited amounts** | **Secondary genetic changes during long-term cultivation** |
| | | | **Use of fetal calf serum poses the risk of contaminations with cattle viruses** |
| | **Availability limited** | | |

Attempts to find TTV DNA in human primary tumor materials suffers from one disadvantage: the plurality of TTV genotypes in human material. This renders it virtually impossible to identify a specific genotype as an etiologic agent for a human cancer type. For these reasons studies on the persistence of TTV DNA sequences in cells derived from cancer tissue culture lines were initiated. Thus far the results have been extremely surprising: PCR primers used to discover regions of the TTV large open reading frame have been entirely unsuccessful. However, other primer combinations, discovering exclusively a short GC-rich regulatory region of the TTV genome of about 71 bases, detected this sequence in a larger number of cell lines (Figure 1). This regulatory region is highly conserved among different TTV genotypes and is not present in the human genome data bank.

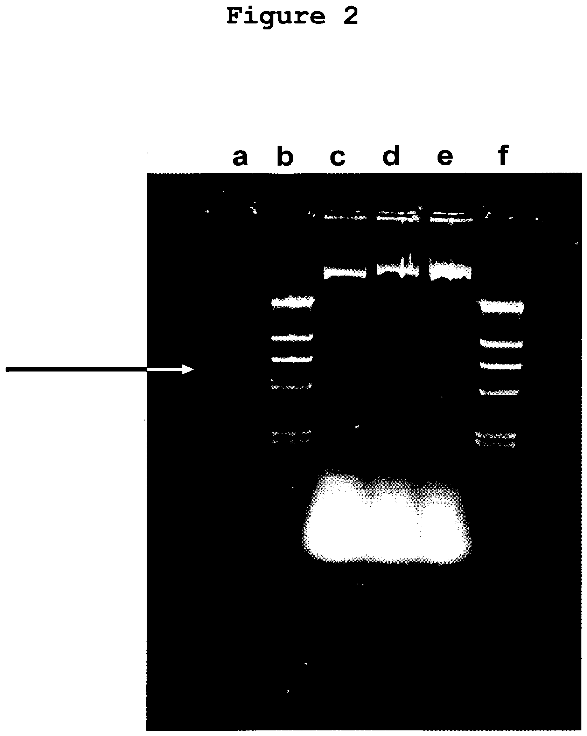

In a first series of experiments the same sequence was discovered in a number of additional cell lines. These included the following lines:
- *MCF7* (breast cancer line);
- *HAK-1, KMH-2, L1236* (all Epstein-Barr virus negative Hodgkin's lymphoma lines);
- *Y69* (Epstein-Barr virus negative B-lymphoma)
- *HSB-2* (acute lymphocytic leukemia);
- *P3HR-1* (Epstein-Barr virus-positive Burkitt's lymphoma);
- *BJAB* (Epstein-Barr virus negative Burkitt's lymphoma);
- *Ng* (EBV-immortalized B lymphoblasts from a patient with multiple sclerosis)-
-
Besides these 9 positive lines, two melanoma cell lines (IGL and KR, Fig. 1) and human placenta DNA were negative in initial experiments. Interestingly, after removal of spooled DNA from L1236 cells and RNase treatment of the remaining solution, besides mitochondrial DNA two faint bands of similar size became visible banding between positions 4.3-6.6 kb (double-stranded DNA size marker) in the agarose gels (Fig. 2). Analysis of these sequences revealed again the presence of the TTV regulatory region. Mung-bean nuclease, digesting selectively single-stranded DNA, completely abolished the cellular DNA-containing bands from four multiple sclerosis biopsies in contrast to double-stranded control DNA, underlining the single-stranded nature of the former. Similar studies are presently conducted for isolates from tumor DNA.

### Example 3

### Analyses of chimeric TTV/truncated host cell DNA sequences

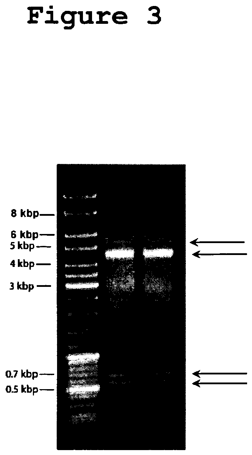

Initially, all attempts failed to use primers in outwards orientation starting within the regulatory region in order to find flanking TT viral DNA, surrounding this region. Invariably, however, human cellular DNA was demonstrated in the respective clones (Fig. 3).

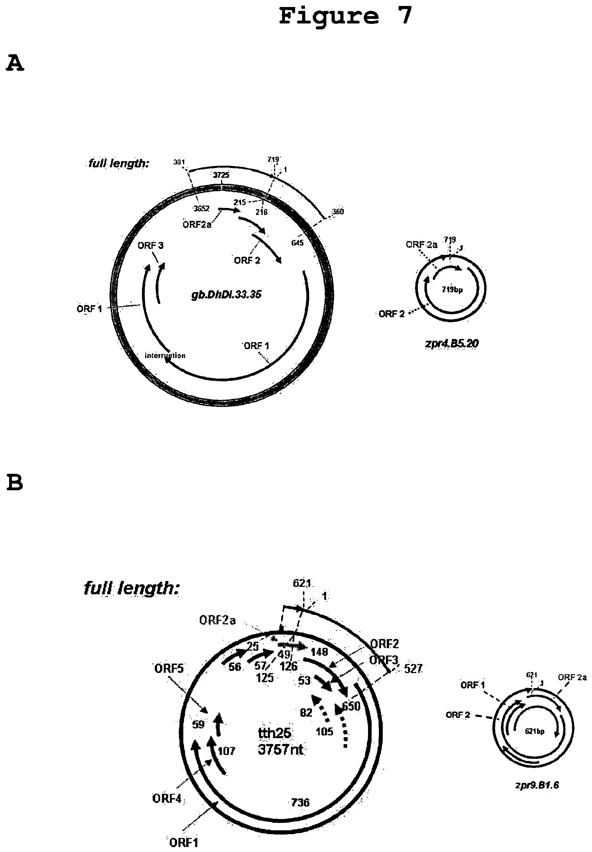

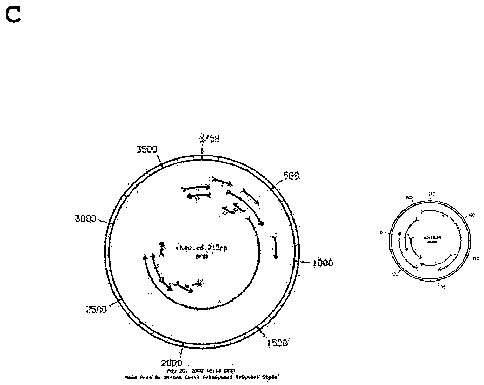

The human genes in these clones and their arrangements within the single-stranded episomal DNA, obviously controlled by the TTV 71 base region, are presently being analyzed. The available data indicate a substantial variation in the uptake of commonly truncated host cell genes. Their possible conversion into growth-stimulating oncogenes or into functions interfering with tumorsuppressor genes requires functional tests which are presently under investigation. The same accounts for rearranged TTV virus sequences. Some of the available data are presented in Figures 7, 8, 9, and 11 to 13.

### Example 4

### Identification and characterization of TTV genomes

Initial amplification of the short conserved GC-rich region of TT viruses in serum and biopsy samples led to the identification of TTV DNA in the majority of cases. Subsequent amplification of the complete genome is necessary to identify specific TTV types as many share exact DNA homology in the amplified 71 bp lying in the control region, but differ as much as 60-80% in sequence identity in the rest of their genomes. A number of back-to-back primer combinations was designed on sequences obtained during the course of the investigations (Table 2). Long distance PCR amplification was performed on TTV DNA positive samples. Amplicons ranging between 3 to 4 kb were cloned and sequenced. TTV DNA positive samples originated from healthy subjects as well as patients with leukaemia, multiple sclerosis, rheumatoid arthritis and kidney disease. Part of these data has previously been described (Leppik et al., 2007; Sospedra et al., 2005; de Villiers et al., 2009).

A total of 53 full-length DNA genomes were characterized. As many as 12 distinct full-length isolates were identified after sequencing 19 genomes from a single biopsy. The genome organization of different isolates of one TTV type varied despite low diversity of nucleotides (ranging from 1 - 4%). Although the large open reading frame ORF1 was mainly involved, differences within the noncoding region and other genes were also noted. These data confirmed earlier observations (Jelcic et al., 2004; Leppik et al., 2007; de Villiers et al., 2009). Modifications in the ORF1 included premature stop codons leading to separate smaller ORFs in this region, considerable sequence diversity in the hypervariable region (Nishizawa et al., 1999; Jelcic et al., 2004) or absence of a stop codon resulting in a larger ORF1 than present in the prototype (Figure 16). The official classification of the family *Anelloviridae* is based on comparisons of the ORF1 DNA sequences (Biagini and de Micco, 2010). Due to the ORF1 modifications in the isolates obtained, the full-length genomic sequences was included in the phylogenetic analyses presented here. The aim of this analysis was to gain an overview of the isolates TTV-HD in relation to established TTV species (Figure 18). All previous isolates are included in this tree as well (Jelcic et al., 2004; Leppik et al., 2007; de Villiers et al., 2009).

### Example 5

### In vitro replication of TTV-HD

Attempts to associate torque teno virus infection with the pathogenesis of a specific disease have repeatedly been reported in the past. Samples from a large range of diseases have been analysed. In vitro investigations were hampered by negative attempts to identify a cell culture system in which these viruses can readily be propagated over longer time periods. Virus particles were initially characterized with the help of density gradients and immunoglobulin aggregates (reviewed in Okamoto, 2009) and later visualized from sera and feces (Itoh et al., 2000). Torque teno viruses occur predominantly in cells of the hematopoietic system (Okamoto, 2009). The first isolates were obtained from the spleen of a patient with Hodgkin's lymphoma (Jelcic et al., 2004). Therefore, the L428 cell line was used in initial attempts to demonstrate *in vitro* replication and transcription of TTV-HD3a. Replication of the full-length genome for up to 7 days after transfection of the linearized virus DNA was achieved (Leppik et al., 2007). In order to extend this period of replication, full-length TTV genomes were transfected into the human embryonic kidney cell line 293TT which was engineered to express high levels of SV40 large T antigen (Buck et al., 2004). Secondly, it was decided to include 12 full-length isolates in this study in order to determine whether 1) variations in the ORF1 would influence replication and formation of virus particles, 2) divergent TTV types vary in their mode of replication. Great care was taken in propagating all 12 isolates in parallel in order to exclude variation as far as possible which may occur during handling.

The following isolates were chosen for transfection and propagation: TTV-HD3a (Leppik et al., 2007) and TTV-HD1a (Jelcic et al., 2004). TTV-HD1a is closest related to species TTV3 (hel32) and TTV-HD3a to species TTV12 (ct44f) (Figure 4). TTV-HD16a (species TTV22-related), TTV-HD15a (species TTV12-related), TTV-HD14a, TTV-HD14b, TTV-HD14c and TTV-HD14e (species TTV29-related) were all isolated from brain biopsies from patients with multiple sclerosis. TTV-HD20a (species TTV13-related) originated from kidney tissue and TTV-HD23a, TTV-HD23b and TTV-HD23d (species TTV3-related) were amplified from serum taken from patients with rheumatoid arthritis. The sequences of TTV-HD14a, TTV-HD14b, TTV-HD14c and TTV-HD14e vary between 1-2% in their full-length genomes. The prototype is TTV-HD14a with an intact ORF1 of 648 amino acids (aa) in size. The ORF1 of TTV-HD14b is 660aa in size with only 554aa sharing identity to TTV-HD14a ORF1, whereas the rest of the ORF indicates fusion to ORF4 (after de Schmidt and Noteborn, 2009). Similarly, TTV-HD14c ORF1 is 712aa and constitutes an ORF1 (first 645aa) fused to ORF5. TTV-HD14e ORF1 is interrupted resulting in 2 ORFs of 467aa and 179aa in size. The TTV-HD23b, TTV-HD23d and TTV-HD23a genomes vary only between 1-3% in sequence identity, but their ORF1 genes differ as follows: TTV-HD23a ORF1 as prototype is 736aa in size, TTV-HD23b ORF1 DNA sequence varies from that of TTV-HD23a in the hypervariable region by 18,4% (34,2% in amino acids). TTV-HD23b and TTV-HD23d DNA sequences differ only 1% in overall identity, but the TTV-HD23d ORF1 is interrupted resulting in 2 ORFs 307aa and 365aa in size (Figure 16).

Transfections were performed on semi-confluent 293TT cells. The nature of this cell line with its many rounded cells attached to the monolayer does not permit a clear-cut identification of cytopathic effects. Cells were passaged when confluent or when cells started to detach from the surface. Flasks were shaken to loosen all cells. Cells were centrifuged and aliquots frozen, as well as used for DNA and RNA extraction and electron microscopic analyses. Frozen infected cells were initially used to re-infect new 293TT cultures as re-infection failed if cells had previously been trypsinized at the time of harvest. Virus replication was monitored by performing long-distance PCR on DNA extracted from infected cells. Periods between re-infection and cell harvest varied between 3 to 7 days, depending on culture density. No obvious morphological differences were noted between cultures of different TTV isolates. Re-infection during the course of one experiment was performed several times using frozen cell aliquots frozen. In vitro propagation of TT viruses has not been described before. Restriction enzyme digestion was performed on cellular DNA obtained from the initially transfected samples to remove any residual bacteria-generated virus DNA. Long PCR amplification results indicated de novo replication of virus DNA. Examples of these TTV DNA amplicons using infected cellular DNA as template are presented in Figure 19.

Long distance PCR amplification of the full-length DNA molecules indicated considerable differences between cultures. Second round amplifications (using the same primers as in the first round) were necessary on all cultures infected with isolates from brain biopsies, i.e. TTV-HD16a, HD15a and the 4 individual TTV-HD14 isolates (Figure 19A), despite their divergence (45-50% nucleotide homology) according to the phylogenetic analyses (Figure 18). Modifications in ORF1 did not seem to influence amplification or propagation as visualized in the amplification of the full-length DNA (Fig 21A a-c). Additional DNA amplicons varying in size were observed in HD15a-infected cultures. The occurrence of these molecules increased during subsequent propagation with a concomitant reduction in the full-length genome (Figure 21A a-c lane 5). We previously reported subviral molecules of a similar nature in human serum samples (Leppik et al., 2007). Similar off-sized amplicons were also occasionally noted in TTV-HD16a-infected cultures (lane 6) and rarely in TTV-HD14 cultures (lanes 1-4).

Large differences were noted in the behaviour of the other 6 isolates. This variation was also evident between experiments and passages (Figure 19B b1, b2, b3) reflecting an apparent high sensitivity to very minor modifications in culturing conditions. The initially replicating full-length genome (3,8kb) was lost during propagation (Figure 19B a-c) in concurrence with prominent subgenomic amplicons ranging in size in TTV-HD20a-, TTV-HD3a- and TTV-HD1a-infected cells (lanes 7-9, Figure 19B). Amounts of input DNA used for long-distance PCR amplification, as well as of amplicons loaded onto gels were the same for all cultures. The high level of DNA amplicons of isolates TTV-HD23b, TTV-HD23d and TTV-HD23a after a single round of long-distance PCR may therefore indicate a stronger replication potential during early passages.

Due to the differences observed between the two groups of isolates, it was investigated whether variations could be observed during serial sampling. Equivalent passages of TTV-HD14e and TTV-HD23b were propagated in parallel and samples were taken daily. Long-distance amplification indicated a constant replication of TTV-HD14e (visible after two rounds of DNA amplification) in contrast to the decreasing replication of TTV-HD23b (visible already after a single round of DNA amplification) which was lost after 10 days in culture (Figure 19C). These cultures were not passaged and morphological differences between cultures were not noticeable.

### Example 6

### In vitro formation, replication and characterization of µTTV subviral molecules

The appearance of smaller DNA amplicons of a constant size in cultures from isolates TTV-HD14b, TTV-HD14c, TTV-HD14d and TTV-HD14e, as well as TTV-HD1a and the 3 TTV-HD23 isolates, was already noted early after transfection and was maintained during passages (Figure 19A and B). They were cloned and characterized. These subviral DNA molecules (µTTV-HD14, 719 bases in size) from TTV-HD14b and the 3 TTV-HD14 isolates were all identical in DNA sequence and represented circular subgenomic rearranged molecules originating from the parental TTV-HD14 genome (Figure 20A). Similarly, a rearranged subviral DNA molecule (µTTV-HD1, 621 bases) originated from the parental TTV-HD1a genome (Figure 20B). Interestingly, replication of µTTV-HD1 was maintained during passages, despite the disappearance of the full-length TTV-HD1a genome. This presence or absence of the subviral molecules in TTV-HD23 cultures indicates a possible influence of culturing conditions. Here these molecules ranged from 400 to 900 bases in size with an increased level of 642 and 401 base molecules. Characterization of the cloned molecules indicated an apparent evolutionary preferred maturation process as a segment of the 401 base subviral molecule (µTTV-HD23.1) was duplicated in the 642 base subviral DNA (µTTV-HD23.2; Figure 20C). Multiple versions of this segment were present in larger molecules. Subviral genomes originating from TTV-HD23b, TTV-HD23d as well as TTV-HD23a cultures, were all identical in DNA sequence. Transfection of these subviral rearranged molecules in 293TT cells resulted in replication of their genomes (Figure 21) as visualized after PCR amplification. Interestingly, the respective µTTV reacted exactly in the same way as the parental genomes, i.e. genomic µTTV-HD15 DNA initial replication was strong, but was subsequently only visualized after nested PCR amplification (Figure 21). Small protein-like structures 10 nm in size were visible by electron microscopy after filtration (0.22 µm) of the culture medium from these cell cultures (Figure 22).

### Example 7

### Purification of virus-like particles (complete genomes and µTTV)

Attempts to purify virus particles were initiated after second round re-infections. Crude cell extracts were centrifuged on 27-33-39% Optiprep step gradients (Buck et al., 2005). Aliquots of gradient fractions were lysed prior to separation by gel electrophoresis. Gradient fractions indicating virus DNA were frozen at -80°C and used for further re-infections. Two DNA bands at the 2 kb and 1.0 kb level of the double-stranded DNA size marker were clearly visible (Figure 8A). The exact sizes of these DNA molecules could not be determined as suitable single-stranded DNA markers are not available. Cell suspensions were, in addition, filtered through a 0.22 µm filter prior to gradient centrifugation. Negative staining of these samples indicated virus-like particles of approximately 30 nm in size (Figure 8). Similarly protein structures (ca. 10 nm in size) were seen after filtration of the culture medium after propagation of the µTTV-HD genomes (Figure 22). These filtrates were lysed and the DNA separated on agarose gels (Figure 22).

### Example 8

### In vitro transcription

Detailed transcription patterns of TTV have been reported for the isolates TTV-P1C1 (Müller et al., 2008), TTV-HEL32 (Qiu et al., 2005; Kakkola et al., 2009) and TTV-HD3a (Leppik et al., 2007). Three main mRNA species (1.0, 1.2 and 3.0kb) had earlier been reported in bone marrow cells (Okamoto et al., 2000a) and in COS1 cells (Kamahora et al., 2000). Predictions for use of initiation codons according to Kozak rules (Jelcic et al., 2004) in combination with use of alternative splice acceptor and donor sites (Leppik et al., 2007) indicated the involvement of non-conserved mechanisms during transcription of torque teno viruses. The transcription of the isolates was investigated by using single-, as well as double-stranded cDNA as templates for 3'-and 5'RACE mapping. Double-stranded cDNA reduces the possibility for the formation of non-specific hybrids. In addition, primers (forward and reverse) were selected which were located within the intergenic regions, instead of commonly used gene-specific primers. This was done in aim of covering the expression of any unpredicted genes in the TTV genome. RNA from all cultures was extracted on day 7 after transfection. RNA from control transfections with vector alone was included to control for false positive amplification. The transcription analyses were repeated to control for a suitable time point for harvesting mRNA by extracting RNA 48 hours after transfection in the case of isolate TTV-HD14e. Transcription patterns observed did not differ between day 2 and day 7. All results obtained in the transcription analyses are presented in Figure 17.

Abundant transcripts were isolated from TTV-HD23 infected cultures. Their transcription patterns, as well as those for TTV-HD20a, TTV-HD15a, TTV-HD16a were in general similar to previously described transcription patterns (reviewed in Kakkola et al., 2009). An exception is the absence of a full-length ORF1 transcript from all of the isolates. This is surprising in view of the fact that virus-like particles are concomitantly being produced. Transcripts covering sections of the ORF1 gene (either the 5'- or the 3'-ends) and which could code for smaller proteins, were present (examples in Figure 17). In silico analyses for putative proteins revealed additional information from what have to date been reported. Examples are splicing (fusions) between either ORF2 or ORF2a with ORF1 or with ORF5 in TTV-HD16a (6.3s.2, 6.3s.3, 6.3s.9), Splicing between ORF1 and ORF5 is another possibility (6.3.7). Short transcripts covering the region of ORF2 in TTV-HD20a may also be expressed as a smaller ORF1 protein (7.3.5, 7.3.4, 7.5.13) (Figure 17). Transcripts were in addition obtained using primers (forward or reverse) located in the control region. Two observations were made. Reverse primers resulted in spliced or non-spliced transcripts covering extended regions of the genome (12.5.19, 12.5.20, 12.5.21, 5.5s.16, 5.5s.17, 5.5s.18, 5.5s.19) or transcripts varying in length which did not have any coding capacity (5.5s.12, 5.5s.13, 5.5s.14, 5.5s.15, 11.5.7, 11.5.8, 11.5.9). Amplification with forward primers in this region resulted in other short non-coding transcripts or spliced transcripts with coding capacity even as distant as ORF5 (4.3.4, 3.3.1, 3.3.2) (Figure 17).

### List of References

1. Belotserkovskii, B.P., Liu, R., Tornaletti, S., Krasilnikova, M.M., Mirkin, S.M. and Hanawalt, P.C. 2010. Mechanisms and implications of transcription blockage by guanine-rich DNA sequences. Proc. Natl. Acad. Sci USA. 107:12816-12821.
2. Biagini, P., and P. de Micco. 2010. La famille des Anelloviridae: virus TTV et genres apparentés. Virologie 14:3-16.
3. Biagini, P., Charrel, R.N., de Micco, P., and X. de Lamballerie. 2003. Association of TT virus primary infection with rhinitis in a newborn. Clin. Infect. Dis. 36:128-129.
4. Buck, C.B., Pastrana, D.V., Lowy, D.R., and J.T. Schiller. 2004. Efficient intracellular assembly of papillomaviral vectors. J. Virol.78:751-757.
5. Buck, C.B., Pastrana, D.V., Lowy, D.R., and J.T. Schiller. 2005. Generation of HPV pseudovirions using transfection and their use in neutralization assays. Methods Mol. Med. 119:445-462.
6. Del Val, C., Mehrle, A., Falkenhahn, M., Seiler, M., Glatting, K-H., Poustka, A., Suhai, S., and S. Wiemann. 2004. High-throughput protein analysis integrating bioinformatics and experimental assays. Nucleic Acid Res. 32:742-748.
7. de Schmidt, M.H., and M.H.M. Noteborn. 2009. Apoptosis-inducing proteins in chicken anemia virus and TT virus. Curr. Topics Microbiol. Immunol. 331:131-149.
8. de Villiers, E-M., Kimmel, R., Leppik, L., and K. Gunst. 2009. Intragenomic rearrangement in TT viruses: a possible role in the pathogenesis of disease. Curr. Topics Microbiol. Immunol.331:91-107.
9. de Villiers, E-M., Schmidt, R., Delius, H., and H. zur Hausen. 2002. Heterogeneity of TT virus related sequences isolated from human tumor biopsy specimens. J. Mol. Med. 80:44-50.
10. Fei, J-W., Wei, Q-X., Angel, P., and E-M. de Villiers. 2005. Differential enhancement of a cutaneous HPV promoter by p63, Jun and mutant p53. Cell Cycle 4:689-696.
11. Garbuglia, A.R., Iezzi, T., Capobianchi, M.R., Pignoloni, P., Pulsoni, A., Sourdis, J., Pescarmona, E., Vitolo, D., and F. Mandelli. 2003. Detection of TT virus in lymph node biopsies of B-cell lymphoma and Hodgkin's disease, and its association with EBV infection. Int. J. Immunopathol. Pharmacol. 16:109-118.
12. Itoh, Y., Takahashi, M., Fukuda, M., Shibayama, T., Ishikawa, T., Tsuda, F., Tanaka, T., Nishizawa, T., and H. Okamoto. 2000. Visualization of TT virus particles recovered from the sera and feces of infected humans. Biochem. Biophys. Res. Commun 279:718-724.
13. Jelcic, I., Hotz-Wagenblatt, A., Hunziker, A., zur Hausen, H., and E-M. de Villiers. 2004. Isolation of multiple TT virus genotypes from spleen biopsiey tissue from a Hodgkin's disease patient: Genome reorganization and diversity in the hypervariable region. J. Virol. 78:7498-7507.
14. Jeske, H. 2009. Geminiviruses. Curr Top Microbiol Immunol. 331:185-226
15. Kakkola, L., Bondén, H., Hedman, L., Kivi, N., Moisala, S. Julin, J., Ylä-Liedenpohja, Miettinen, S., Kantola, K., Hedman, K., and M. Söderlund-Venermo. 2008. Expression of all six human Torque teno virus (TTV) proteins in bacteria and in insect cells, and analysis of their IgG responses. Virology 382:182-189.
16. **Ka**kkola, L., Hedman, K., Qiu, J., Pintel, D., and M. Söderlund-Venermo. 2009. Replication of and protein synthesis by TT viruses. Curr. Topics Microbiol. Immunol.331: 53-64.
17. Kakkola, L., Tommiska, J., Boele, L.C.L., Miettinen, S., Blom, T., Kekarainen, T., Qiu, J., Pintel, D., Hoeben, RC., Hedman, K., and M. Söderlund-Venermo. 2007. Construction and biological activity of a full-length molecular clone of human Torque teno virus (TTV) genotype 6. FEBS. J. 274:4719-4730.
18. Kamada, K., Kamahora, T., Kabat, P., and S. Hino. 2004. Transcriptional regulation of TT virus: promoter and enhancer regions in the 1.2-kb noncoding region. Virology 321:341-348.
19. Kamahora, T., Hino, S., and H. Miyata. 2000. Three spliced mRNAs of TT virus transcribed from a plasmid containing the entire genome in COS1 cells. J. Virol 74:9980 - 9986.
20. Kanda, Y., Tanaka, Y., Kami, M., Saito, T., Asai, T., Izutsu, K., Yuji, S., Ogawa, S., Honda, H., Mitani, K., Ciba, S., Yasaki, Y., and H. Hirai. 1999. TT virus in bone marrow transplant recipients. Blood 93: 2485-2490.
21. Kazi, A., Miyata, H., Kurokawa, K., Khan, M.A., Kamahora, T., Katamine, S., and S. Hino. 2000. High frequency of postnatal transmission of TT virus in infancy. Arch. Virol. 145:535-540.
22. Kovacs, E., Tompa, P., Liliom, K., and L. Kalmar. 2010. Dual coding in alternative reading frames correlates with intrinsic protein disorder. Proc. Natl. Acad. Sci. U. S. A. 107:5429-5434
23. Leppik, L., Gunst, K., Lehtinen, M., Dillner, J., Streker, K., and E-M. de Villiers. 2007. In vivo and in vitro intragenomic rearrangement of TT viruses. J Virol 81:9346-9356.
24. Maggi, F., Andreoli, E., Riente, L., Meschi, S., Rocchi, J., Delle Sedie, A., Vatteroni, ML., Ceccherini-Nelli, L., Specter, S., and M. Bendinelli. 2007. Torquetenovirus in patients with arthritis. Rheumatology 46:885-886.
25. Maggi, F., Focosi, D., Albani, M., Lanini, L., Vatteroni, ML, Petrini, M., Ceccherini-Nelli, L., Pistello, M., and M Bendinelli. 2010. Role of hematopoietic cells in the maintenance of chronic human torquetenovirus plasma viremia. J. Virol. 84:6891-6893.
26. Maggi, F., Fornai, C., Vatteroni, ML., Siciliano, G., Menichetti, F., Tascini, C., Specter, S., Pistello, M., and M. Bendinelli. 2001a. Low prevalence of TT virus in the cerebrospinal fluid of viremic patients with central nervous system disorders. J. Med. Virol. 65:418-422
27. Maggi, F., Fornai, C., Zaccaro, L., Morrica, A., Vatteroni, M.L., Isola, P., Marchi, S., Ricchiuti, A., Pistello, M., and M. Bendinelli. 2001b. TT virus (TTV) loads associated with different peripheral blood cell types and evidence for TT replication in activated mononuclear cells. J. Med. Virol. 64:190-194.
28. Maggi, F., Pifferi, M., Fornai, C., Andreoli, A., Tempestini, E., Vatteroni, M., Presciuttini, S., Marchi, S., Pietrobelli, A., Boner, A., Pistello, M., and M. Bendinelli. 2003a. TT virus in the nasal secretions of children with acute respiratory disease: relations to viremia and disease severity. J. Virol. 77:2418- 2425.
29. Maggi, F., Pifferi, M., Tempestini, E., Fornai, C., Lanini, L., Andreoli, E., Vatteroni, M., Presciuttini, S., Pietrobelli, A., Boner, A., Pistello, M., and M. Bendinelli. 2003b. TT virus loads and lymphocyte subpopulations in children with acute respiratory diseases. J. Virol 77:9081-9083.
30. Mariscal, L.F., Lopez-Alcorocho, J.M., Rodriguez-Inigo, E., Ortiz-Movilla, N., de Lucas, S., Bartolome, J., and V. Carreno. 2002. TT virus replicates in stimulated but not in nonstimulated peripheral blood mononuclear cells. Virology 301:121-129.
31. Müller, B., März, A., Doberstein, K., Finsterbusch, T., and A. Mankertz. 2008. Gene expression of the human Torque Teno Virus isolate P/1C1. Virology 381:36-45.
32. Nawaz-ul-Rehman, M.S., and C.M. Fauquet. 2009. Evolution of geminiviruses and their satellites. FEBS Letter 583:1825-1832.
33. Nishizawa, T., Okamoto, K., Konishi, H., Yoshikawa, H., Miyakawa, Y., and M. Mayumi. 1997. A novel DNA virus (TTV) associated with elevated transaminase levels in posttransfusion hepatitis of unknown etiology. Biochem. Biophys. Res. Commun. 241:92-97.
34. Ninomiya, M., Nishizawa, T., Takahashi, M., Lorenzo, F.R., Shimosegawa, T., and H. Okamoto. 2007. Identification and genomic characterization of a novel human torque teno virus of 3.2kb. J. Gen. Virology 88:1939-1944.
35. Ninomiya, M., Takahashi, M., Nishizawa, T., Shimosegawa, T., and H. Okamoto. 2008. Development of PCR assays with nested primers specific for differential detection of three human anelloviruses and early acquisition of dual or triple infection during infancy. J. Clin. Microbiol. 46:507-514.
36. Okamoto, H. 2009. History of discoveries and pathogenicity of TT viruses. Curr. Top. Microbiol. Immunol. 331:1-20.
37. Okamoto, H., Nishizawa, T., Tawara, A., Takahashi, M., Kishimoto, J., Sai, T., and Y. Sugai. 2000a. TT virus mRNAs detected in the bone marrow cells from an infected individual. Biochem. Biophys. Res. Commun. 279:700-707.
38. Okamoto, H., Takahashi, M., Kato, N., Fukuda, M., Tawara, A., Fukuda, S., Tanaka, T., Miyakawa, Y., and M. Mayumi. 2000b. Sequestration of TT virus of restricted genotypes in peripheral blood mononuclear cells. J. Virol. 74:10236-10239.
39. Okamoto, H., Takahashi, M., Nishizawa, T., Tawara, A., Sugai, Y., Sai, T., Tanaka, T., and F. Tsuda. 2000c. Replicative forms of TT virus DNA in bone marrow cells. Biochem. Biophys. Res. Commun. 270:657-662.
40. Okamoto, H., Ukita, M., Nishizawa, T., Kishimoto, J., Hoshi, Y., Mizuo, H., Tanka, T., Miyakawa, Y., and M. Mayumi. 2000d. Circular double-stranded forms of TT virus DNA in the liver. J. Virol. 74:5161-5167.
41. Paprotka, T., Metzler, V., and H. Jeske. 2010. The first DNA 1-like a satellite in association with New World begomovirus in natural infections. Virology 404:148-157.
42. Patil, B.L, and C.M. Fauquet. 2010. Differential interaction between cassava mosaic geminivirus and geminivirus satellites. J. Gen. Virol. 91:1871-1882.
43. Peng, Y.H., Nishizawa, T., Takahashi, T., Ishikawa, T., Yoshikawa, A., and H. Okamoto. 2002. Analysis of the entire genomes of thirteen TT virus variants classifiable into the fourth and fifth genetic groups, isolated from viremic infants. Arch. Virol. 147:21-41.
44. Pifferi, M., Maggi, F., Andreoli, E., Lanini, L., Marco, ED., Fornai, C., Vatteroni, ML., Pistello, M., Ragazzo, V., Macchia, P., Boner, A., and M. Bendinelli. 2005. Associations between nasal torquetenovirus load and spitometric indices in children with asthma. J. Infect. Dis. 192:1141-1148.
45. Qiu, J., Kakkola, L., Cheng, F., Ye, C., Söderlund-Venermo, M., Hedman, K., and D.J. Pintel. 2005. Circovirus TT virus genotype 6 expresses six proteins following transfection of a full-length clone. J. Virol. 79:6506-6510.
46. Ryabova, L.A., Pooggin, M., and T. Hohn. 2006. Translation reinitiation and leaky scanning in plant viruses. Virus Res. 119:52-62.
47. Saunders, K., Bedford, I.D., Briddon, R.W., Markham, P.G., Wong, S.M., and J. Stanley. 2000. A unique virus complex causes Ageratum yellow vein disease. Proc. Natl. Acad. Sci. USA 97:6890-6895.
48. Shiramizu, B., Yu, Q., Hu, N., Yanagihara, R., and V.R. Nerurkar. 2002. Investigation of TT virus in the etiology of pediatric acute lymphoblastic leukaemia. Pediatr. Hematol. Oncol. 19:543-551.
49. Sospedra, M., Zhao, Y., zur Hausen, H., Muraro, P.A., Hamashin, C., de Villiers, E.M., Pinilla, C., and R. Martin. 2005. Recognition of conserved amino acid motifs of common viruses and ist role in autoimmunity. PLoS Pathog. 1:e41.
50. Stanley, J. 2004. Subviral DNAs associated with geminivirus disease complexes. Vet. Microbiol 98:121-129.
51. Takahashi, M., Asabe, S., Gotanda, Y., Kishimoto, J., Tsuda, F., and H. Okamoto. 2002. TT virus is distributed in various leukocyte subpopulations at distinct levels, with the highest viral load in granulocytes. Biochem. Biophys. Res. Commun. 290:242-248.
52. Takahashi, K., Iwasa, Y., Hijikata, M., and S. Mishiro. 2000. Identification of a new human DNA virus (TTV-like mini virus, TLMV) intermediately related to TT virus and chicken anemia virus. Arch. Virol. 145:979-993.
53. Zhong, S., Yeo, W., Tang, M., Liu, C., Lin, X.R., Ho, W.M., Hui, P., and P.J. Johnson. 2002. Frequent detection of the replicative form of TT virus DNA in peripheral blood mononuclear cells and in bone marrow cells in cancer patients. J. Med. Virol. 66:428-434.
54. zur Hausen H., and E-M. de Villiers. 2005. Virus target cell conditioning model to explain some epidemiologic characteristics of childhood leukemias and lymphomas. Int. J. Cancer 115:1-5.

## Claims

1. A rearranged TT virus polynucleic acid comprising
A) a nucleotide sequence which shows at least 70% identity to a nucleotide sequence being selected from the nucleotide sequences shown in Figure 6 and a polynucleic acid encoding a polypeptide containing a signature motif of a mammalian protein being associated with cancer or an autoimmune disease, wherein the rearranged TT virus polynucleic acid is selected from the group of µTTV molecules consisting of zpr4.20 shown in Figure 11B, zpr9.6 shown in Figure 12B and zpr12.24 shown in Figure 13B;
or
B)
(a) a nucleotide sequence being selected from the nucleotide sequences shown in Figure 6;
(b) a nucleotide sequence which shows at least 70% identity to a nucleotide sequence of (a) and said rearranged TT virus polynucleic acid is capable of replicating autonomously upon transfection in 293TT cells;
(c) a nucleotide sequence which is the complement of the nucleotide sequence of (a), or (b); or
(d) a nucleotide sequence encoding an amino acid sequence selected from the amino acid sequences RVPKVSLHTA VKGQFGLGTG RAM, RVPKVSLHTA VKGQFGLGTG RAM, RVPEVSLHTA VKGQFGLGTG RAM, GAEGEFTHRS QGAIRARDWP GHG, GAEGEFTHRS QGAIRARDWP GYG, GAVGEFTHRS QGAIRARDWP GYG and GAGGEFTHRS QGAIRARDWP GYG shown in Figure 14
and said rearranged TT virus polynucleic acid is capable of replicating autonomously upon transfection in 293TT cells, wherein said nucleotide sequence of (a), (b), (c) or (d) is linked to a polynucleic acid encoding a polypeptide containing a signature motif of a mammalian protein being associated with cancer or an autoimmune disease via a phosphodiester bond, the signature motif is at least 10 aa and the degree of identity of this signature motif to a corresponding motif in a mammalian protein is at least 80%, but is not identical with said corresponding motif, wherein said corresponding motif is selected from the group consisting of
(1) opsin selected from the amino acid sequences IYNSFHRGFALG and RLELQKRLPW LELNEKAVE;
(2) protamine 1 selected from the amino acid sequences ARYRRRSRSRSRSRYGRRRRRSRSRRRRSRRRRR, ARYRCCRSKSRSRCRRRRRRCRRRRRRCCRRRRR,and ARYRCCRSPSRSRCRRRRRRFYRRRRRCHRRRRR;
(3) protamine 2 selected from the amino acid sequences HTRRRRSCRRRRRRACRHRRHRRGCRRIRRRRRCR, RRRSRSCRRRRRRSCRYRRRPRRGCRSRRRRRCRR, HRRRRSCRRRRRHSCRHRRRHRRGCRRSRRRRRCR, and RRRHRSCRRRRRRSCRHRRRHRRGCRTRRRRCRRY;
(4) galanin that has the amino acid sequence of ATLGLGSPVKEKRGWTLNSAGYLLGPHAIDNHRSFSDKHGLTGKRELEPEDEARPGSFDRPL SESNIVRTIIEFLSFLHLKEAGALDRLPGLPAAASSEDLERS;
(5) plexin/semaphrin/integrin type repeat signature that has the amino acid sequence of RCSQVGVTSCSECLLARDPVGCGWCSSEGRCTRGERCDERRGSRQNWSSGPSSQCQ
(6) gastrin that has the amino acid sequence of VAGEDSDGCYVQLPRSR;
(7) collagenase that has the amino acid sequence of MSRLAELYLLGDSIKGRHDNLWLAAAEMLSYYAPEGKSELGIDICQAKLELAAKV1PYLYEC SGPAAIRSQDLTDGQAASACDILRNKEKDFHQVKYTGKTPVADDGNTRVEVGVFVSEEDYKR YSAFASKEVKAQFGRVTDNGGMYLEGNPSDAGNQVRFIAYEEAKLNADLSIGNLEHEYTHYL DGRFDTYGTFSRNLEESHIVWWEEGFAEYVHYKQGGVPYQAAPELIGQGSKLYLSDVFTTTE EGYAELFAGSHDTDRIYRWGYLAVRFMLETNHNRDYESLLYHSRYGNSFAFYAYLVKLLGYM YNNEFGIWNNEFGIW;
(8) collagen helix repeat that has the amino acid sequence of GPPGPPGPPGPPGPPGPPGPPGPAGAPGPPGPPGEPGPPGPPGPPGPPGPPGAPGAPGP;
(9) male specific sperm protein that has the amino acid sequence of VGGPCGPCGPCGGPCCGSCCSPCGGPCGPCGPCGPCGPCCGGCGPC GPCGPCCGTTEKYCGL;
(10) microbial collagenase metalloprotease (M9) signature that has the amino acid sequence of GLETLVEFLRAGYYVRFYN;
(11) MIC1 microneme protein signature that has the amino acid sequence of TYISTKLDVAVGSCHK;
(12) autoimmune regulator (AIRE) signature that has the amino acid sequence of DFWRVLFKDYNLERY;
(13) gliadin that has the amino acid sequence of PQAQGSVQPQQLPQFEEIRNL;
(14) neuro peptide Y2 receptor signature having the sequence of AFLSAFRCEQRLDAIHS;
(15) aerolysin that has the amino acid sequence of WDKRYIPGEVKWWDWNWTIQ;
(16) orexin that has the amino acid sequence of MNLPSAKVSWAAYTLLLLLLLLPPALLSLGVDAQPLPDCCRQKTCSC RLYELLHGAGNHAAGILTLGKRRPGPPGLQGRLQRLLQASGNHAAGILT MGRRAGAELEPRLCPGRRCLAAAASALAPRGRSRV;
(17) GIP receptor that has the amino acid sequence of PRLGPYlGDQTLTLWNQALAA;
(18) prion that has the amino acid sequence of SNGGSRYPGQGSPGGNRYPPQ;
(19) neurotensin that has the amino acid sequence of METSSPWPPRPSP;
(20) orphan nuclear receptor (4A nuclear receptor) family signature that has the amino acid sequence of PVNLLNALVRAHVDSTP;
(21) brain derived neurotrophic factor signature (BDN) that has the amino acid sequence of PLLFLLEEYKNYLDAAN;
(22) calcitonin that has the amino acid sequence of KCYDRMQQLPPYEGEGPY;
(23) leukotriene B4 type I receptor selected from the amino acid sequences SRRLRVRRFHRRRRTGR and GRRLQARRFRRSRRTGR;
(24) Sjogren's syndrome/scleroderma autoantigen 1 (autoantigen p27) that has the amino acid sequence of EISKKMAELLLKGATMLDEHCPKCGTPLFRLKDGKVFCPICE;
(25) vasopressin that has the amino acid sequence of RAGGRRRGRRTGSPSEGARV;
(26) melanin-concentrating hormone 2 receptor signature that has the amino acid sequence of LVQPFRLTRWRTRYKTIRIN;
(27) prostanoid EP1 receptor signature that has the amino acid sequence of ISLGPPGGWRQALLAGL;
(28) cyklinkinase that has the amino acid sequence of EWRSLGVQQSLGWVH;
(29) peroxisime proliferator-activated receptor (1C nuclear receptor) signature that has the amino acid sequence of KTETDASLHPLLQ;
(30) muscarinic M1 receptor signature that has the amino acid sequence of KMPMVDPEAQAPTKQPPK;
(31) metabotropic gamma-aminobutyric acid (GABA) type B2 receptor signature that has the amino acid sequence of LAPGAWGWARGAPRPPPSS;
(32) arginine deaminase signature that has the amino acid sequence of SELSRGRGGPRCMSMPLVR;
(33) opioid growth factor receptor repeat that has the amino acid sequence of SPSETPGPRPAGPARDEPAE;
(34) adhesion molecule CD36 signature that has the amino acid sequence of WIFDVQNPDEVAKNSSKIKVKQR;
(35) myelin proteolipid protein (PLP) signature that has the amino acid sequence of GVVLGAIIGGVLGVVLLLVLLLYLV; and
(36) chlamidiaom that has the amino acid sequence of CGSYVPSCSKPCG.

2. The rearranged TT virus polynucleic acid of claim 1 which is present as a single- or double-stranded extrachromosomal episome.

3. The rearranged TT virus polynucleic acid of claim 1 or 2 which is a single-stranded DNA.

4. An oligonucleotide primer shown in Figure 10 and that has a nucleotide sequence selected from the group consisting of CAGCGAGAACGCCACGGAGG GAGATCCT;
CGGACGGGCGTGGAAAACTCAGCCATTC;
CGGACTGGCCGGGCTAT; and
AGCCCGAATTGCCCCTTGA.

5. An expression vector comprising a rearranged TT virus polynucleic acid of any one of claims 1 to 3 operably linked to prokaryotic, eukaryotic or viral transcription and translation control elements.

6. The expression vector of claim 5 which is an artificial chromosome.

7. A host cell comprising an expression vector according to claim 5 or 6.

8. A method for the detection of a rearranged TTV polynucleic acid of claim 1 in a biological sample, comprising: (a) optionally extracting sample polynucleic acid, (b) hybridizing the polynucleic acid comprised in said sample with at least one probe specifically hybridizing to the polynucleic acid of claim 1, wherein said probe comprises a part of the polynucleic acid according to claim 1, said probe is optionally labelled, and (c) detecting the hybridized polynucleic acid.

## Patentansprüche

1. Eine umgeordnete TT-Virus-Polynukleinsäure umfassend
A) eine Nukleotidsequenz, die mindestens 70% Identität zu einer Nukleotidsequenz aufweist, die aus den in Figur 6 gezeigten Nukleotidsequenzen ausgewählt ist, und eine Polynukleinsäure, die ein Polypeptid kodiert, das ein Signaturmotiv eines Säugetierproteins enthält, das mit Krebs oder einer Autoimmunkrankheit assoziiert ist, wobei die umgeordnete TT-Virus-Polynukleinsäure ausgewählt ist aus der Gruppe von µTTV Molekülen, bestehend aus zpr4.20, gezeigt in Fig. 11B, zpr9.6, gezeigt in Fig. 12B und zpr12.24, gezeigt in Fig.. 13B;
oder
B)
(a) eine Nukleotidsequenz, die aus den in Abb. 6 gezeigten Nukleotidsequenzen ausgewählt ist;
(b) eine Nukleotidsequenz, die mindestens 70% Identität mit einer Nukleotidsequenz von (a) aufweist und die umgeordnete TT Virus Polynukleinsäure nach Transfektion in 293TT Zellen autonom replizieren kann;
(c) eine Nukleotidsequenz, die das Komplement der Nukleotidsequenz von (a) oder (b) ist; oder
(d) eine Nukleotidsequenz, die eine Aminosäuresequenz kodiert, die aus den Aminosäuresequenzen RVPKVSLHTA VKGQFGLGTG RAM, RVPKVSLHTA VKGQFGLGTG RAM, RVPEVSLHTA VKGQFGLGTG RAM, GAEGEFTHRS QGAIRARDWPGHG, GAEGEFTHRS QGAIRARDWP GYG, GAVGEFTHRS QGAIRARDWP GYG und GAGGEFTHRS QGAIRARDWP GYG ausgewählt ist, die in Abb. 14 gezeigt sind
und die umgeordnete TT-Virus-Polynukleinsäure in der Lage ist, bei Transfektion in 293TT Zellen autonom zu replizieren, wobei die Nukleotidsequenz von (a), (b), (c) oder (d) über eine Phosphodiesterbindung mit einer Polynukleinsäure verbunden ist, die ein Polypeptid kodiert, umfassend ein Signaturmotiv eines Säugetierproteins, das mit Krebs oder einer Autoimmunkrankheit assoziiert ist, wobei das Signaturmotiv mindestens 10 aa beträgt und der Grad der Identität dieses Signaturmotivs zu einem entsprechenden Motiv in einem Säugetierprotein mindestens 80% beträgt, aber nicht identisch mit dem entsprechenden Motiv ist, wobei das entsprechende Motiv ausgewählt ist aus der Gruppe bestehend aus
(1) Opsin, ausgewählt aus den Aminosäuresequenzen IYNSFHRGFALG und RELELQKRLPW LELNEKAVE;
(2) Protamin 1 ausgewählt aus den Aminosäuresequenzen ARYRRRSRSRSRSRYGRRRRRSRSRRRRSRRRRR, ARYRCCRSKSRSRCRRRRRRCRRRRRRCCRRRRR, und ARYRCCRSPSRSRCRRRRRRFYRRRRRCHRRRRR;
(3) Protamin 2 ausgewählt aus den Aminosäuresequenzen HTRRRRSCRRRRRRACRHRRHRRGCRRIRRRRRCR, RRRSRSCRRRRRRSCRYRRRPRRGCRSRRRRRCRR, HRRRRSCRRRRRHSCRHRRRHRRGCRRSRRRRRCR, und RRRHRSCRRRRRRSCRHRRRHRRGCRTRRRRCRRY;
(4) Galanin, das die Aminosäuresequenz ATLGLGSPVKEKRGWTLNSAGYLLGPHAIDNHRSFSDKHGLTGKRELEPEDE ARPGSFDRPLSESNIVRTIIEFLSFLHLKEAGALDRLPGLPAAASSEDLERS aufweist;
(5) Wiederholungssignatur vom Plexin/Semaphrin/Integrin Typ, die die Aminosäuresequenz RCSQVGVTSCSECLLARDPVGCGWCSSEGRCTRGERCDERRGSRQNWSS GPSSQCQ aufweist;
(6) Gastrin, das die Aminosäuresequenz VAGEDSDGCYVQLPRSR aufweist;
(7) Kollagenase, das die Aminosäuresequenz MSRLAELYLLGDSIKGRHDNLWLAAAEMLSYYAPEGKSELGIDICQAKLELAA KVIPYLYECSGPAAIRSQDLTDGQAASACDILRNKEKDFHQVKYTGKTPVAD DGNTRVEVGVFVSEEDYKRYSAFASKEVKAQFGRVTDNGGMYLEGNPSDA GNQVRFIAYEEAKLNADLSIGNLEHEYTHYLDGRFDTYGTFSRNLEESHIVW WEEGFAEYVHYKQGGVPYQAAPELIGQGSKLYLSDVFTTTEEGYAELFAGS HDTDRIYRWGYLAVRFMLETNHNRDYESLLYHSRYGNSFAFYAYLVKLLGY MYNNEFGIWNNEFGIW aufweist;
(8) Kollagen-Helix-Wiederholung, die die Aminosäuresequenz GPPGPPGPPGPPGPPGPPGPPGPAGAPGPPGPPGEPGPPGPPGPPGPPG PPGAPGAPGP aufweist;
(9) Männliches spezifisches Spermaprotein, das die Aminosäuresequenz VGGPCGPCGPCGGPCCGSCCSPCGGPCGPCGPCGPCGPCCGGCGPC GPCGPCCGTTEKYCGL aufweist;
(10) Mikrobielle Kollagenase Metalloprotease (M9) Signatur, die die Aminosäuresequenz GLETLVEFLRAGYYVRFYN aufweist;
(11) MIC1 mikroneme Proteinsignatur, die die Aminosäuresequenz TYISTKLDVAVGSCHK aufweist;
(12) Autoimmunregulator (AIRE) Signatur, die die Aminosäuresequenz DFWRVLFKDYNLERY aufweist;
(13) Gliadin, das die Aminosäuresequenz PQAQGSVQPQQLPQFEEIRNL aufweist;
(14) Neuropeptid Y2 Rezeptor Signatur, die die Aminosäuresequenz AFLSAFRCEQRLDAIHS aufweist;
(15) Aerolysin, das die Aminosäuresequenz WDKRYIPGEVKWWWWNWTIQ aufweist;
(16) Orexin, das die Aminosäuresequenz MNLPSAKVSWAAYTLLLLLLLPPALLSLGVDAQPLPDCCRQKTCSC RLYELLHGAGNHAAGILTLGKRRPGPPGLQGRLQRLLQASGNHAAGILT MGRRAGAELEPRPLPGRRCLAAAASALAPRGRSRV aufweist;
(17) GIP-Rezeptor, der die Aminosäuresequenz PRLGPYIGDQTLTLWNQALAA aufweist;
(18) Prion, das die Aminosäuresequenz SNGGSRYPGQGSPGGNRYPPQ aufweist;
(19) Neurotensin, das die Aminosäuresequenz METSSPWPPRPSP aufweist;
(20) Orphan Kernrezeptor (4A Kernrezeptor) Familiensignatur, die die Aminosäuresequenz PVNLLNALVRAHVDSTP aufweist;
(21) vom Gehirn stammende neurotrophe Faktorsignatur (BDN), die die Aminosäuresequenz PLLFLLEEYKNYLDAAN aufweist;
(22) Calcitonin, das die Aminosäuresequenz KCYDRMQQLPPYEGEGPY aufweist;
(23) Leukotrien B4 Typ I Rezeptor, ausgewählt aus den Aminosäuresequenzen SRRLRVRRFHRRRRTGR und GRRLQARRFRRSRRTGR;
(24) Sjögren-Syndrom / Sklerodermie Autoantigen 1 (Autoantigen p27), das die Aminosäuresequenz EISKKMAELLLKGATMLDEHCPKCGTPLFRLKDGKVFCPICE aufweist;
(25) Vasopressin, das die Aminosäuresequenz RAGGRRRGRRTGSPSEGARV aufweist;
(26) Melanin-konzentrierendes Hormon 2 Rezeptor Signatur, die die Aminosäuresequenz LVQPFRLTRWRTRYKTIRIN aufweist;
(27) Prostanoid EP1 Rezeptor Signatur, die die Aminosäuresequenz ISLGPPGGWRQALLAGL aufweist;
(28) Cyklinkinase, das die Aminosäuresequenz EWRSLGVQQSLGWVH aufweist;
(29) Peroxisim Proliferator-aktivierter Rezeptor (1C nuklearer Rezeptor) Signatur, der die Aminosäuresequenz KTETDASLHPLLQ aufweist;
(30) Muskarin M1 Rezeptor Signatur, die die Aminosäuresequenz KMPMVDPEAQAPTKQPPK aufweist;
(31) metabotrope Gamma-Aminobuttersäure (GABA) Typ B2 Rezeptorsignatur, die die Aminosäuresequenz LAPGAWGWARGAPRPPPSS aufweist;
(32) Arginin Deaminase Signatur, die die Aminosäuresequenz SELSRGRGGPRCMSMPLVR aufweist;
(33) Opioid Wachstumsfaktor Rezeptor Wiederholung, das die Aminosäuresequenz SPSETPGPRPAGPARDEPAE aufweist;
(34) Adhäsionsmolekül CD36-Signatur, das die Aminosäuresequenz WIFDVQNPDEVAKNSSKIKVKQR aufweist;
(35) Myelinproteolipidprotein (PLP) Signatur, die die Aminosäuresequenz GVVLGAIIGGVLGVVLLVLLLYLV aufweist; und
(36) Chlamidiaom die die Aminosäuresequenz CGSYVPSCSKPCG aufweist.

2. Umgeordnete TT-Virus-Polynukleinsäure nach Anspruch 1, die als ein- oder doppelsträngiges extrachromosomales Episom vorliegt.

3. Umgeordnete TT-Virus-Polynucleinsäure nach Anspruch 1 oder 2, die eine einzelsträngige DNA ist.

4. Ein in Figur 10 gezeigter Oligonukleotidprimer mit einer Nukleotidsequenz ausgewählt aus der Gruppe bestehend aus CAGCGAGAACGCCACGGAGG GAGATCCT;
CGGACGGGCGTGGAAAACTCAGCCATTC;
CGGACTGGCCGGGCTAT; und
AGCCCGAATTGCCCCTTGA.

5. Expressionsvektor, umfassend eine umgeordnete TT-Virus-Polynukleinsäure nach einem der Ansprüche 1 bis 3, funktionsfähig verknüpft mit prokaryotischen, eukaryotischen oder viralen Transkriptions- und Translationskontrollelementen.

6. Expressionsvektor nach Anspruch 5, der ein künstliches Chromosom ist.

7. Wirtszelle, umfassend einen Expressionsvektor nach Anspruch 5 oder 6.

8. Verfahren zum Nachweis einer umgeordneten TTV-Polynukleinsäure nach Anspruch 1 in einer biologischen Probe, umfassend: (a) gegebenenfalls Extrahieren von Proben-Polynukleinsäure, (b) Hybridisieren der in der Probe enthaltenen Polynukleinsäure mit mindestens einer spezifisch hybridisierenden Sonde zur Polynukleinsäure nach Anspruch 1, wobei die Sonde einen Teil der Polynukleinsäure nach Anspruch 1 umfasst, wobei die Sonde gegebenenfalls markiert ist und (c) die hybridisierte Polynukleinsäure nachgewiesen wird.

## Revendications

1. Acide polynucléique de virus TT réarrangé comprenant
A) une séquence nucléotidique qui présente une identité d'au moins 70% avec une séquence nucléotidique choisie parmi les séquences nucléotidiques représentées à la figure 6 et un acide polynucléique codant pour un polypeptide contenant un motif de signature d'une protéine de mammifère associée à un cancer ou à une maladie autoimmune, où l'acide polynucléique de virus TT réarrangé est choisi parmi le groupe de molécules µTTV constituées de zpr4.20 représentée à la figure 11B, zpr9.6 représentée à la figure 12B et zpr12.24 représentée à la figure 13B;
ou
B)
(a) une séquence nucléotidique choisie parmi les séquences nucléotidiques représentées à la figure 6;
(b) une séquence nucléotidique qui présente une identité d'au moins 70% avec une séquence nucléotidique de (a) et ledit acide polynucléique de virus TT réarrangé est apte à se répliquer de manière autonome après transfection dans des cellules 293TT;
(c) une séquence nucléotidique qui est le complément de la séquence nucléotidique de (a) ou (b); ou
(d) une séquence nucléotidique codant pour une séquence d'acides aminés choisie parmi les séquences d'acides aminés RVPKVSLHTA VKGQFGLGTG RAM, RVPKVSLHTA VKGQFGLGTG RAM, RVPEVSLHTA VKGQFGLGTG RAM, GAEGEFTHRS QGAIRARDWP GHG, GAEGEFTHRS QGAIRARDWP GYG, GAVGEFTHRS QGAIRARDWP GYG et GAGGEFTHRS QGAIRARDWP GYG représentées à la figure 14
et ledit acide polynucléique de virus TT réarrangé est apte à se répliquer de manière autonome après transfection dans des cellules 293TT,
dans lequel ladite séquence nucléotidique de (a), (b), (c) ou (d) est liée à un acide polynucléique codant pour un polypeptide contenant un motif de signature d'une protéine de mammifère associée à un cancer ou une maladie auto-immune via une liaison phosphodiester, le motif de signature est d'au moins 10 aa et le degré d'identité de ce motif de signature avec un motif correspondant dans une protéine de mammifères est d'au moins 80%, mais n'est pas identique audit motif correspondant, où ledit motif correspondant est choisi dans le groupe constitué de
(1) opsine choisie parmi les séquences d'acides aminés IYNSFHRGFALG et RLELQKRLPW LELNEKAVE;
(2) protamine 1 choisie parmi les séquences d'acides aminés ARYRRRSRSRSRSRYGRRRRRSRSRRRRSRRRRR, ARYRCCRSKSRSRCRRRRRRCRRRRRRCCRRRRR, et ARYRCCRSPSRSRCRRRRRRFYRRRRRCHRRRRR;
(3) protamine 2 choisie parmi les séquences d'acides aminés HTRRRRSCRRRRRRACRHRRHRRGCRRIRRRRRCR, RRRSRSCRRRRRRSCRYRRRPRRGCRSRRRRRCRR, HRRRRSCRRRRRHSCRHRRRHRRGCRRSRRRRRCR, et RRRHRSCRRRRRRSCRHRRRHRRGCRTRRRRCRRY;
(4) galanine qui présente la séquence d'acides aminés de ATLGLGSPVKEKRGWTLNSAGYLLGPHAIDNHRSFSDKHGLTGKRELEPEDEARPGSFDRPL SESNIVRTIIEFLSFLHLKEAGALDRLPGLPAAASSEDLERS;
(5) signature de répétition de type plexine/sémaphorine/intégrine qui présente la séquence d'acides aminés de RCSQVGVTSCSECLLARDPVGCGWCSSEGRCTRGERCDERRGSRQNWSSGPSSQCQ
(6) gastrine qui présente la séquence d'acides aminés de VAGEDSDGCYVQLPRSR;
(7) collagénase qui présente la séquence d'acides aminés de MSRLAELYLLGDSIKGRHDNLWLAAAEMLSYYAPEGKSELGIDICQAKLELAAKV1PYLYEC SGPAAIRSQDLTDGQAASACDILRNKEKDFHQVKYTGKTPVADDGNTRVEVGVFVSEEDYKR YSAFASKEVKAQFGRVTDNGGMYLEGNPSDAGNQVRFIAYEEAKLNADLSIGNLEHEYTHYL DGRFDTYGTFSRNLEESHIVWWEEGFAEYVHYKQGGVPYQAAPELIGQGSKLYLSDVFTTTE EGYAELFAGSHDTDRIYRWGYLAVRFMLETNHNRDYESLLYHSRYGNSFAFYAYLVKLLGYM YNNEFGIWNNEFGIW;
(8) répétition d'hélice de collagène qui présente la séquence d'acides aminés de GPPGPPGPPGPPGPPGPPGPPGPAGAPGPPGPPGEPGPPGPPGPPGPPGPPGAPGAPGP;
(9) protéine de sperme spécifique mâle qui présente la séquence d'acides aminés de VGGPCGPCGPCGGPCCGSCCSPCGGPCGPCGPCGPCGPCCGGCGPC GPCGPCCGTTEKYCGL;
(10) signature de métalloprotéase de collagénase microbienne (M9) qui présente la séquence d'acides aminés de GLETLVEFLRAGYYVRFYN;
(11) signature de protéine micronème MIC1 qui présente la séquence d'acides aminés de TYISTKLDVAVGSCHK;
(12) signature de régulateur auto-immun (AIRE) qui présente la séquence d'acides aminés de DFWRVLFKDYNLERY;
(13) gliadine qui présente la séquence d'acides aminés de PQAQGSVQPQQLPQFEEIRNL;
(14) signature de récepteur neuropeptide Y2 qui présente la séquence de AFLSAFRCEQRLDAIHS;
(15) aérolysine qui présente la séquence d'acides aminés de WDKRYIPGEVKWWDWNWTIQ;
(16) orexine qui présente la séquence d'acides aminés de MNLPSAKVSWAAYTLLLLLLLLPPALLSLGVDAQPLPDCCRQKTCSC RLYELLHGAGNHAAGILTLGKRRPGPPGLQGRLQRLLQASGNHAAGILT MGRRAGAELEPRLCPGRRCLAAAASALAPRGRSRV;
(17) récepteur GIP qui présente la séquence d'acides aminés de PRLGPY1GDQTLTLWNQALAA;
(18) prion qui présente la séquence d'acides aminés de SNGGSRYPGQGSPGGNRYPPQ;
(19) neurotensine qui présente la séquence d'acides aminés de METSSPWPPRPSP;
(20) signature de la famille des récepteurs nucléaires orphelins (récepteur nucléaire 4A) qui présente la séquence d'acides aminés de PVNLLNALVRAHVDSTP;
(21) signature de facteur neurotrophique dérivé du cerveau (BDN) qui présente la séquence d'acides aminés de PLLFLLEEYKNYLDAAN;
(22) calcitonine qui présente la séquence d'acides aminés de KCYDRMQQLPPYEGEGPY;
(23) récepteur de type I de leucotriène B4 choisi parmi les séquences d'acides aminés de SRRLRVRRFHRRRRTGR et GRRLQARRFRRSRRTGR;
(24) autoantigène 1 de syndrome de Sjögren/scleroderma (autoantigène p27) qui présente la séquence d'acides aminés de EISKKMAELLLKGATMLDEHC PKCGT PLFRLKDGKVFC PICE;
(25) vasopressine qui présente la séquence d'acides aminés de RAGGRRRGRRTGSPSEGARV;
(26) signature de récepteur de l'hormone de concentration de mélanine 2 qui présente la séquence d'acides aminés de LVQPFRLTRWRTRYKTIRIN;
(27) signature de récepteur de prostanoïde EP1 qui présente la séquence d'acides aminés de ISLGPPGGWRQALLAGL;
(28) cyklinkinase qui présente la séquence d'acides aminés de EWRSLGVQQSLGWVH;
(29) signature de récepteur activé par proliférateur de peroxisime (récepteur nucléaire 1C) qui présente la séquence d'acides aminés de KTETDASLHPLLQ;
(30) signature de récepteur M1 muscarinique qui présente la séquence d'acides aminés de KMPMVDPEAQAPTKQPPK;
(31) signature de récepteur de type B2 de l'acide métabotropique gamma-aminobutyrique (GABA) qui présente la séquence d'acides aminés de LAPGAWGWARGAPRPPPSS;
(32) signature d'arginine désaminase qui présente la séquence d'acides aminés de SELSRGRGGPRCMSMPLVR;
(33) répétition du récepteur de facteur de croissance opioïde qui présente la séquence d'acides aminés de SPSETPGPRPAGPARDEPAE;
(34) signature de la molécule d'adhésion CD36 qui présente la séquence d'acides aminés de WIFDVQNPDEVAKNSSKIKVKQR;
(35) signature de la protéine protéolipidique de myéline (PLP) qui présente la séquence d'acides aminés de GWLGAIIGGVLGWLLLVLLLYLV; et
(36) chlamidiaom qui présente la séquence d'acides aminés de CGSYVPSCSKPCG.

2. Acide polynucléique de virus TT réarrangé selon la revendication 1, qui est présent sous forme d'un épisome extrachromosomique monobrin ou double brin.

3. Acide polynucléique de virus TT réarrangé selon la revendication 1 ou 2, qui est un ADN monobrin.

4. Amorce oligonucléotidique représentée à la figure 10 et qui présente une séquence nucléotidique choisie dans le groupe constitué de CAGCGAGAACGCCACGGAGG GAGATCCT;
CGGACGGGCGTGGAAAACTCAGCCATTC;
CGGACTGGCCGGGCTAT; et
AGCCCGAATTGCCCCTTGA.

5. Vecteur d'expression comprenant un acide polynucléique de virus TT réarrangé selon l'une quelconque des revendications 1 à 3, lié de manière opérationnelle à des éléments de contrôle de transcription et de translation procaryotes, eucaryotes ou viraux.

6. Vecteur d'expression selon la revendication 5, qui est un chromosome artificiel.

7. Cellule hôte comprenant un vecteur d'expression selon la revendication 5 ou 6.

8. Procédé de détection d'un acide polynucléique de TTV réarrangé selon la revendication 1 dans un échantillon biologique, comprenant le fait de: (a) extraire optionnellement un échantillon d'acide polynucléique, (b) hybrider l'acide polynucléique compris dans ledit échantillon avec au moins une sonde qui s'hybride spécifiquement avec l'acide polynucléique selon la revendication 1, où ladite sonde comprend une partie de l'acide polynucléique selon la revendication 1, ladite sonde est optionnellement étiquetée et (c) détecter l'acide polynucléique hybridé.
